# EUROPEAN PATENT APPLICATION

(11) **EP 1 832 591 A1**
(43) Date of publication of application: **12.09.2007**
(21) Application number: 05820293.8
(22) Date of filing: 21.12.2005
(51) Int. Cl.: C07D 487/08, A61K 31/407, A61P 31/04, C07C 49/487, C07C 49/517

(54) **12-POSITION SUBSTITUTED MUTILIN DERIVATIVE**

(30) Priority: 27.12.2004 JP 2004377424
(71) Applicant: KYORIN PHARMACEUTICAL CO., LTD., Tokyo 101-8311 (JP)
(72) Inventor: TAKADOI, Masanori, 3460003 (JP); FUKUDA, Yasumichi, Oyama-shi,Tochigi 3290207 (JP); SATO, Taro, Tochigi; 3290101 (JP); NAGAE, Osamu, Tochigi; 3290101 (JP); KISHII, Ryuta, 3290114 (JP); FUKUDA, Hideyuki, 3210123 (JP)
(74) Representative: Vossius & Partner
(86) International application number: PCT/JP2005/023515
(87) International publication number: WO 2006/070671

(57) **Abstract**

A position 12-substituted mutilin derivative, a novel mutilin analogue, is provided that exhibits strong antimicrobial activity against a broad spectrum of Gram-positive or Gram-negative bacteria, including various drug-resistant bacteria, as well as intermediates for the production of such mutilin derivatives.

Specifically, a mutilin derivative or an acid-addition salt thereof is provided that is represented by the following chemical formula (1): 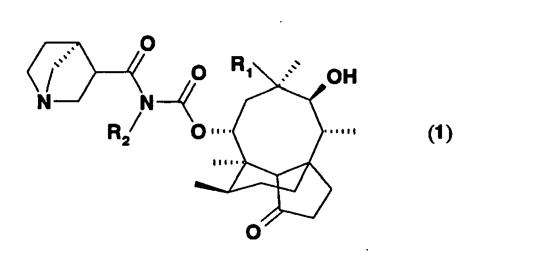 (wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; and R₂ is a hydrogen atom, a lower alkyl group or an aralkyl group whose aromatic ring may be optionally substituted) (R₁ is neither an ethyl group nor a vinyl group).

## Description

### TECHNICAL FIELD

The present invention relates to position 12-substituted mutilin derivatives, novel mutilin analogues that exhibit strong antimicrobial activity against Gram-positive or Gram-negative bacteria including various drug-resistant bacteria and are a potential treatment for infectious diseases.

### TECHNICAL BACKGROUND

Pleuromutilin is a diterpene compound isolated/identified from *Pleurotus mutilus Sacc.* in 1951 and from *Pleurotuspasseckerianus Pil.* in 1976 (Non-Patent Documents 1 and 2). The aglycone part of the compound is referred to as mutilin, which has a characteristic three-ring structure consisting of a highly functionalized eight-membered ring fused with a hydroindanone structure. Mutilin is structurally unique in that it contains 9 asymmetric carbon atoms.

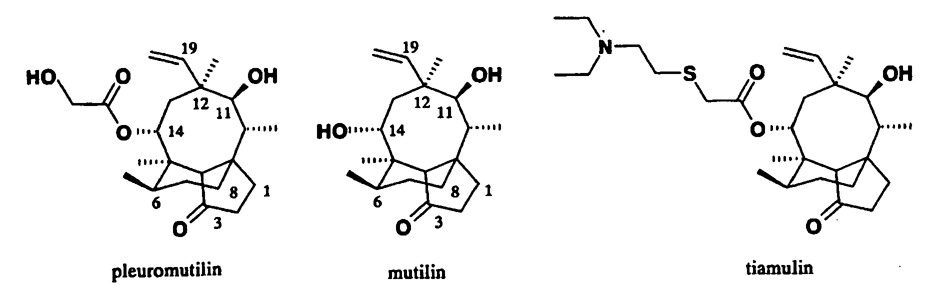

Untreatable infectious diseases caused by drug-resistant bacteria have become a worldwide concern. Studies have demonstrated that pleuromutilin exhibits antimicrobial activity by inhibiting protein synthesis by ribosomes in bacteria. Thus, pleuromutilin serves as a useful lead compound in the search for therapeutic agents for the treatment of untreatable infectious diseases. Although tiamulin (trademark), a pleuromutilin derivative, has long been used in the treatment of infectious diseases in livestock, none of pleuromutilin or mutilin derivatives have ever been used to treat infectious diseases in human.

Mutilin compounds have attracted worldwide attention due to their strong microbial activity and unique chemical structure. Several groups have recently reported novel mutilin derivatives: One mutilin derivative described in Patent Document 1 is as follows:

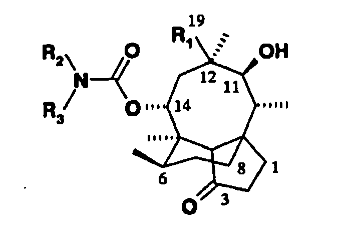

[wherein R₁ is vinyl or ethyl; and R₂ and R₃ are each independently a hydrogen atom, a substituted or unsubstituted, saturated or unsaturated lower alkyl group, a substituted or unsubstituted, saturated or unsaturated 3- to 8-membered cyclic lower alkyl group, a substituted or unsubstituted heterocyclic ring or a substituted, or unsubstituted aromatic ring, or when R₂ is any of the above-described substituents, R₃ is represented by the following chemical formula (I):

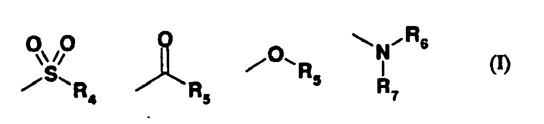

(wherein R₄ is a substituted or unsubstituted, chain or cyclic lower alkyl group, a substituted or unsubstituted heterocyclic ring, a substituted or unsubstituted aromatic ring, a substituted or unsubstituted lower alkylamino group, or a substituted or unsubstituted aromatic amino group; R₅ is a substituted or unsubstituted, chain or cyclic lower alkyl group, a substituted or unsubstituted heterocyclic ring, or a substituted or unsubstituted aromatic ring; or R₆ and R₇ are each independently a hydrogen atom, a substituted or unsubstituted, saturated or unsaturated lower alkyl group, a substituted or unsubstituted, saturated or unsaturated 3- to 8-membered cyclic alkyl group, a substituted or unsubstituted heterocyclic ring or a substituted or unsubstituted aromatic ring, with the proviso that R₆ and R₇ are not the same, or R₆ and R₇ may form, together with the nitrogen atom, a substituted or unsubstituted 3- to 8-membered ring that may contain at least one hetero atom, such as O, N and S, and that may be fused with a hydrocarbon ring, a heterocyclic ring or an aromatic ring), or R₂ and R₃ may together form a substituted or unsubstituted 3-to 8-membered cyclic lower alkyl group that may contain at least one hetero atom, such as O, N and S].

Another mutilin derivative described in Patent Document 2 is as follows:

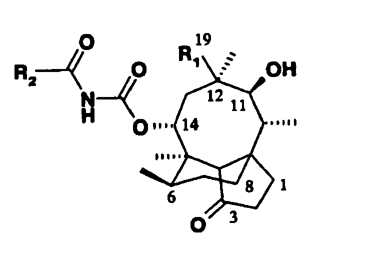

[wherein R₁ is vinyl or ethyl; and R₂ is represented by the following chemical formula (I):

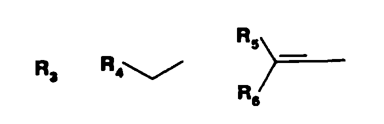

(wherein R₃ and R₄ are each an azabicyclo ring; or R₅ and R₆ may together form an azabicyclo ring)].

Another mutilin derivative described in Patent Document 3 is as follows:

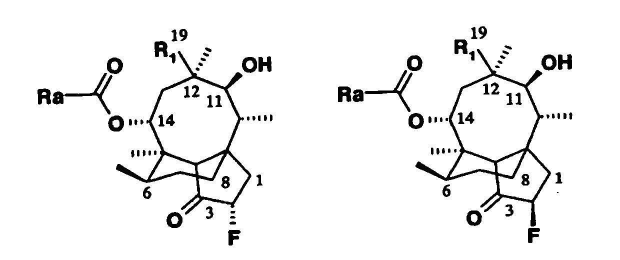

{wherein R₁ is vinyl or ethyl; Ra is represented by the following chemical formula (I):

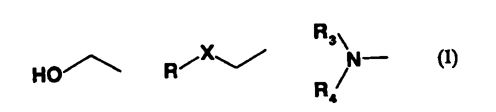

[wherein X is O, S or NR' ; R and R' are each independently an aliphatic substituent or an aromatic substituent with the proviso that R and R' are not the same; R₃ and R₄ are each independently a hydrogen atom, a substituted or unsubstituted, saturated or unsaturated lower alkyl group; a substituted or unsubstituted, saturated or unsaturated 3- to 8-membered cyclic lower alkyl group, a substituted or unsubstituted heterocyclic ring, a substituted or unsubstituted aromatic ring, or when R₃ is any of the above-described substituents, R₄ is represented by the following chemical formula (I):

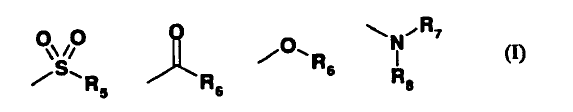

(wherein R₅ is a substituted or unsubstituted, chain or cyclic lower alkyl group, a substituted or unsubstituted heterocyclic ring, a substituted or unsubstituted aromatic ring, a substituted or unsubstituted lower alkylamino group, or a substituted or unsubstituted aromatic amino group; R₆ is a substituted or unsubstituted, chain or cyclic lower alkyl group, a substituted or unsubstituted heterocyclic ring, or a substituted or unsubstituted aromatic ring; or R₇ and R₈ are each independently a hydrogen atom, a substituted or unsubstituted, saturated or unsaturated lower alkyl group, a substituted or unsubstituted, saturated or unsaturated 3- to 8-membered cyclic lower alkyl group, a substituted or unsubstituted heterocyclic ring or a substituted or unsubstituted aromatic ring, with the proviso that R₇ and R₈ are not the same, or R₇ and R₈ may form, together with the nitrogen atom, a substituted or unsubstituted 3- to 8-membered ring that may contain at least one hetero atom, such as O, N and S, and that may be fused with a hydrocarbon ring, a heterocyclic ring or an aromatic ring), or R₃ and R₄ may together form a substituted or unsubstituted 3-to 8-membered cyclic lower alkyl group that may contain at least one hetero atom, such as O, N and S].

Another mutilin derivative described in Patent Document 4 is as follows:

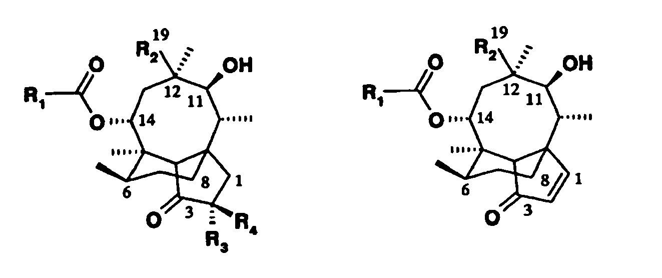

[wherein R₁ is represented by the following chemical formula (I) :

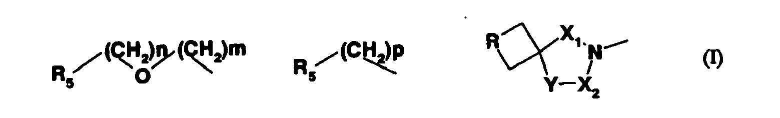

(wherein R is a spiro-monocyclic or bicyclic substituent containing 1 or 2 basic nitrogen atom, X₁ and X₂ are each independently a methylene group or a carbonyl group, and Y is a nitrogen atom, a methylene group or an ethylene group; or R₅ is a substituted or unsubstituted aromatic or heteroaromatic ring that is bound via a carbon atom, m is an integer from 1 to 3, n is an integer from 0 to 2, and p is an integer from 1 to 4); R₂ is vinyl or ethyl; R₃ is a hydrogen atom, a hydroxyl group or a fluorine atom; and R₄ is a hydrogen atom or a fluorine atom when R₃ is a hydrogen atom].

Another mutilin derivative described in Patent Document 5 is as follows:

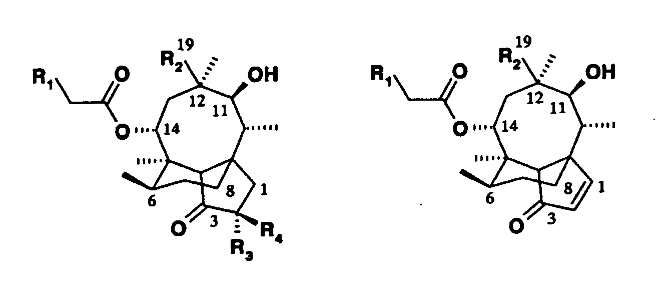

(wherein R₁ is a substituted or unsubstituted heteroaromatic ring which contains at least one nitrogen atom and which may include 5-membered heteroaromatic ring bound via a nitrogen atom; R₂ is vinyl or ethyl; R₃ is a hydrogen atom, a hydroxyl group or a fluorine atom; and R₄ is a hydrogen atom or a fluorine atom when R₃ is a hydrogen atom).

Another mutilin derivative described in Patent Document 6 is as follows:

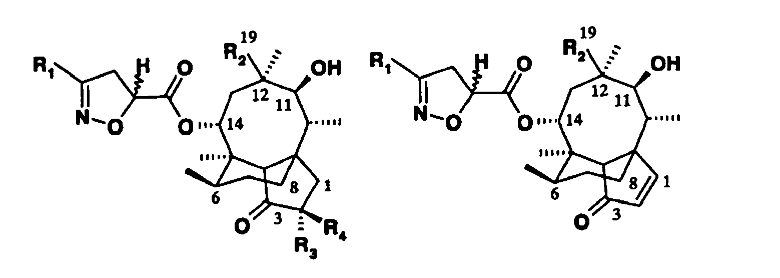

(wherein R₁ is a substituted or unsubstituted phenyl group or a heterocyclicring containing a substituted or unsubstituted nitrogen atom; R₂ is vinyl or ethyl; R₃ is a hydrogen atom, a hydroxyl group or a fluorine atom; and R₄ is a hydrogen atom or a fluorine atom when R₃ is a hydrogen atom).
Another mutilin derivative described in Patent Document 5 is as follows:

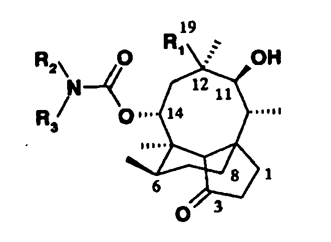

[wherein R₁ is vinyl or ethyl; and R₂ and R₃ are each independently a hydrogen atom, a substituted or unsubstituted, saturated or unsaturated lower alkyl group, a substituted or unsubstituted, saturated or unsaturated 3- to 8-membered cyclic lower alkyl group, a substituted or unsubstituted heterocyclic ring, or a substituted or unsubstituted aromatic ring; or when R₂ is any of the above-described substituents, R₃ is represented by the following chemical formula (I):

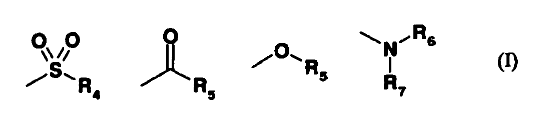

(wherein R₄ is a substituted or unsubstituted, chain or cyclic lower alkyl group, a substituted or unsubstituted heterocyclic ring, a substituted or unsubstituted aromatic ring, a substituted or unsubstituted lower alkylamino group, or a substituted or unsubstituted aromatic amino group; R₅ is a substituted or unsubstituted, chain or cyclic lower alkyl group, a substituted or unsubstituted heterocyclic ring, or a substituted or unsubstituted aromatic ring; or R₆ and R₇ are each independently a hydrogen atom, a substituted or unsubstituted, saturated or unsaturated lower alkyl group, a substituted or unsubstituted, saturated or unsaturated 3- to 8-membered cyclic lower alkyl group, a substituted or unsubstituted heterocyclic ring or a substituted or unsubstituted aromatic ring, with the proviso that R₆ and R₇ are not the same, or R₆ and R₇ may form, together with the nitrogen atom, a substituted or unsubstituted 3- to 8-membered ring that may contain at least one hetero atom, such as O, N and S, and that may be fused with a hydrocarbon ring, a heterocyclic ring or an aromatic ring), or R₂ and R₃ may together form a substituted or unsubstituted 3-to 8-membered cyclic lower alkyl group that may contain at least one hetero atom, such as O, N and S].

Another mutilin derivative described in Patent Document 8 is as follows:

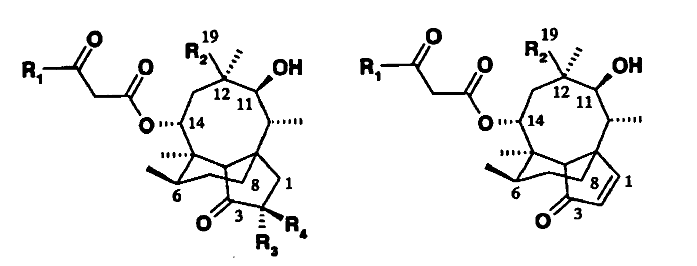

[wherein R₁ is a heterocyclic ring containing a nitrogen atom, a substituted or unsubstituted aromatic ring, a substituted or unsubstituted heteroaromatic ring, or R₁ is represented by the following general formula (I):

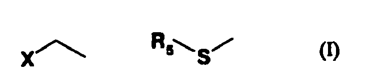

(wherein X is a halogen atom; or R₅ is a lower alkylamino group, a heterocyclic ring containing a nitrogen atom, a substituted or unsubstituted aromatic ring, or a substituted or unsubstituted heteroaromatic ring); R₂ is vinyl or ethyl; R₃ is a hydrogen atom, a hydroxyl group or a fluorine atom; and R₄ is a hydrogen atom or a fluorine atom when R₃ is a hydrogen atom].

Another mutilin derivative described in Patent Document 9 is as follows:

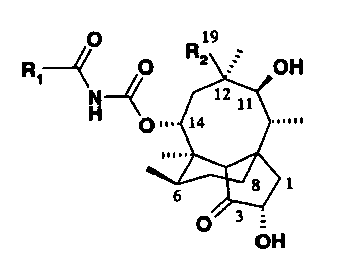

(wherein R₁ is a substituted or unsubstituted 5- or 6-membered heteroaromatic ring; and R₂ is vinyl or ethyl).

Another mutilin derivative described in Patent Document 10 is as follows:

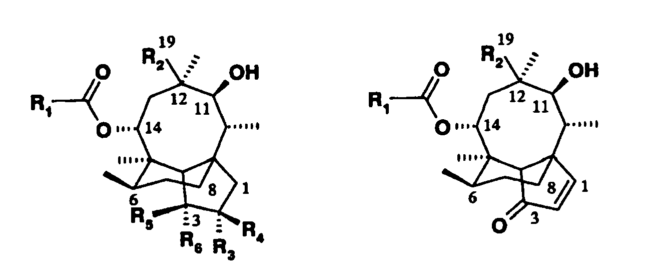

(wherein R₁ is a 5- or 6-membered aromatic or heteroaromatic ring substituted with an amino group substituted with a halogen atom, a substituted or unsubstituted lower alkoxy group, a substituted or unsubstituted lower alkylthioalkoxy group, a hydrogen atom or a substituted or unsubstituted lower alkyl group, a 5- or 6-membered dihydroheteroaromatic ring that contains one oxygen atom or one or two nitrogen atoms and may fused with a benzene ring, a 5- or 6-membered heteroaromatic ring that contains one or two nitrogen atoms, a 5- or 6-membered heterocyclic ring that may contain O, N or S, a 6-membered tetrahydroheteroaromatic ring containing one or two nitrogen atoms, or a 9- or 10-membered bicyclic heteroaromatic ring containing 1 to 4 nitrogen atoms; R₂ is vinyl or ethyl; R₃ is a hydrogen atom, a hydroxyl group or a fluorine atom; R₄ is a hydrogen atom or, when R₃ is a hydrogen atom, a fluorine atom and then R₅ and R₆ are together an oxygen atom, or when R₃ and R₄ are each a hydrogen atom, R₅ is a hydrogen atom or a hydroxyl group and R₆ is a hydrogen atom, or when R₅ is a hydrogen atom, R₆ is a hydrogen atom or a hydroxyl group).

Another mutilin derivative described in Patent Document 11 is as follows:

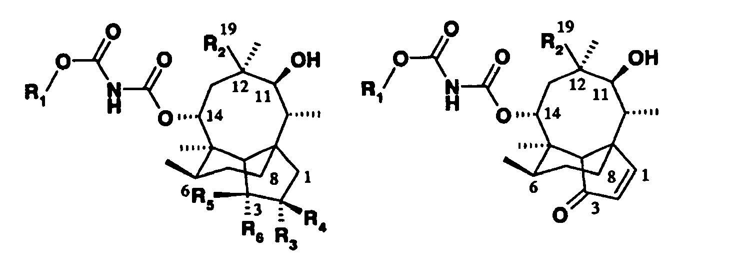

(wherein R₁ is a substituted or unsubstituted lower alkyl group, a substituted or unsubstituted 3- or 6-membered cyclic lower alkyl group, or a substituted or unsubstituted heterocyclic ring; R₂ is vinyl or ethyl; R₃ is a hydrogen atom, a hydroxyl group or a fluorine atom; and R₄ is a hydrogen atom or, when R₃ is a hydrogen atom, a fluorine atom and then R₅ and R₆ are together an oxygen atom, or when R₃ and R₄ are each a hydrogen atom, R₅ is a hydrogen atom or a hydroxyl group and R₆ is a hydrogen atom, or when R₅ is a hydrogen atom, R₆ is a hydrogen atom or a hydroxyl group).

Another mutilin derivative described in Patent Document 12 is as follows:

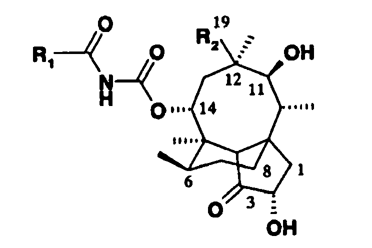

(wherein R₁ is a substituted or unsubstituted 5- or 6-membered heteroaromatic ring; and R₂ is vinyl or ethyl). Each patent disclosed in each of these patent documents claims that each of the above-described mutilin derivatives includes at position 12 a vinyl group originating from a naturally occurring pleuromutilin, or an ethyl group resulting from the reduction of the vinyl group. Thus, mutilin derivatives that have a characteristic structure at position 12 a substituent other than a vinyl or ethyl group as shown in the present invention have never been reported, nor has their antimicrobial activity been described.

The following position 12-substituted mutilin derivatives or position 12-substituted 4-epimutilin derivatives are known. Specifically, Non-Patent Document 3 describes a 4-epimutilin derivative having a desetenyl substituent at position 12, as shown below:

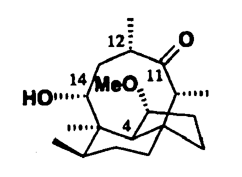

Non-Patent Document 4 describes a 4-epimutilin derivative having a dimethyl substituent at position 12, as shown below:

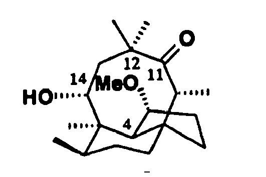

Non-Patent Document 5 describes a pleuromutilin derivative in which the substituent at position 12 has the opposite stereochemistry to that of the naturally occurring pleuromutilin, as well as a pleuromutilin derivative having a cyclopropyl substituent at position 12, as shown below:

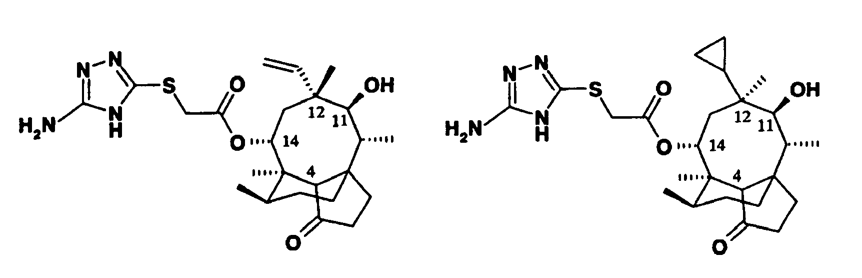

As with the compounds of the present invention, the substituents at position 12 of these mutilin derivatives or epimutilin derivatives are not a vinyl group originating from naturally occurring pleuromutilin or an ethyl group resulting from the reduction of the vinyl group. However, the compounds of the present invention include a cyclic amine structure bound via an acylcarbamoyl structure and in that sense differ from any of the previously reported compounds. Thus, the antimicrobial activity of the compounds of the present invention has never been described.

The following compounds are known as mutilin derivatives having the hydroxyl group at position 11 protected. Specifically, Non-Patent Document 6 describes a mutilin derivative as shown below:

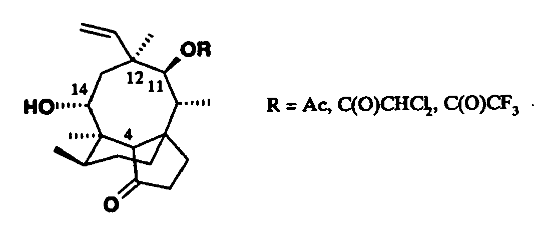

The mutilin derivatives described in this article have an acetoxy, dichloroacetoxy or trifluoroacetoxy group at position 11. These known compounds are not encompassed by the scope of the present invention. The article does not mention any compounds that share common structural features with the compounds of the present invention -- having at position 12 a substituent other than a vinyl group or an ethyl group and having at position 14 a cyclic amine structure bound via an acylcarbamoyl structure -- much less the antimicrobial activity of such compounds.

The following antimicrobial mutilin derivatives are also known. Specifically, Non-Patent Document 7 describes a mutilin derivative having a carbamate substituent at position 14, as shown below:

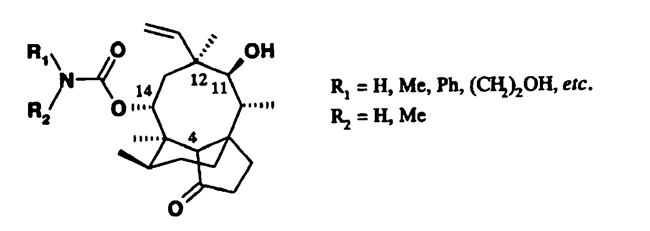

The known mutilin derivativesdescribedin Non-Patent Document 7 have at position 12 a vinyl group originating from naturally occurring pleuromutilin or an ethyl group resulting from the reduction of the vinyl group, and have a carbamoyl group at position 14. Thus, no compounds have been reported to date that have at position 12 a substituent other than a vinyl group or an ethyl group and that have at position 14 a cyclic amine structure bound via an acylcarbamoyl structure, nor has the antimicrobial activity of such compounds been described.

As described above, none of the previously described mutilin derivatives are desirable in terms of microbial activity, toxicity and kinetics within the body. Thus, there is a great need for mutilin derivatives that meet all of these requirements.
Patent Document 1 WO1997/25309 pamphlet
Patent Document 2 WO1998/05659 pamphlet
Patent Document 3 WO2000/07974 pamphlet
Patent Document 4 WO2000/27790 pamphlet
Patent Document 5 WO2000/37074 pamphlet
Patent Document 6 WO2000/73287 pamphlet
Patent Document 7 US6239175
Patent Document 8 WO2001/14310 pamphlet
Patent Document 9 WO2001/74788 pamphlet
Patent Document 10 WO2002/12199 pamphlet
Patent Document 11 US2002/30929 pamphlet
Patent Document 12 US2003/0114674 pamphlet
Non-Patent Document 1 Kavanagh, F. et al. Proc. Natl. Acad. Sci. USA 1951, 37, 570-574.
Non-Patent Document 2 Knauseder, F. et al. J. Antibiot. 1976, 29, 125-131.
Non-Patent Document 3 Berner, H. et al. Tetrahedron 1981, 37, 915-919.
Non-Patent Document 4 Berner, H. et al. Tetrahedron 1983, 39, 1745-1748.
Non-Patent Document 5 Berner, H. et al. Monatsch. Chem. 1986, 117, 1073-1080.
Non-Patent Document 6 Birch, A. J. et al. Tetrahedron 1966, Suppl.8, Part II, 359-387.
Non-Patent Document 7 Brooks, G. et al. Bioorg. Med. Chem. 2001, 9, 1221-1231.

### DISCLOSURE OF THE INVENTION

### PROBLEMS TO BE SOLVED BY THE INVENTION

It is an object of the present invention to provide a position 12-substituted mutilin derivative, a novel mutilin analogue that exhibits strong antimicrobial activity against a broad spectrum of Gram-positive or Gram-negative bacteria, including various drug-resistant bacteria, as well as intermediates for the production of the mutilin derivative. The mutilin derivative of the present invention serves as a potential treatment for infectious diseases.

### MEANS FOR SOLVING THE PROBLEMS

In the course of our studies, the present inventors have found that the novel position-12 substituted mutilin derivative has strong antimicrobial activity and have thus completed the present invention. Specifically, the present invention concerns the following:

(1) A mutilin derivative or an acid-addition salt thereof, represented by the following chemical formula (1):

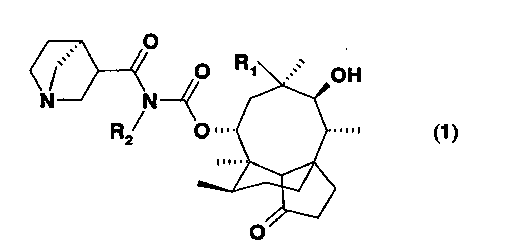

(wherein R₁ is a hydrogen atom, a formyl group, a substituted or unsubstituted lower alkyl group, an aralkyl groupwhose aromatic ring may be optionally substituted, a heteroaralkyl group whose aromatic ring may be optionally substituted or a lower alkyloxycarbonyl group; and R₂ is a hydrogen atom, a lower alkyl group or an aralkyl group whose aromatic ring may be optionally substituted) (R₁ is neither an ethyl group nor a vinyl group).

(2) A mutilin derivative or an acid-addition salt thereof, represented by the following chemical formula (1-1):

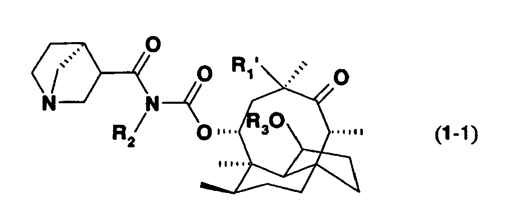

(wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or an aralkyl group whose aromatic ring may be optionally substituted; and R₃ is a protective group for hydroxyl group) (R₁' is neither an ethyl group nor a vinyl group).

(3) A mutilin derivative or an acid-addition salt thereof, represented by the following chemical formula (1-2):

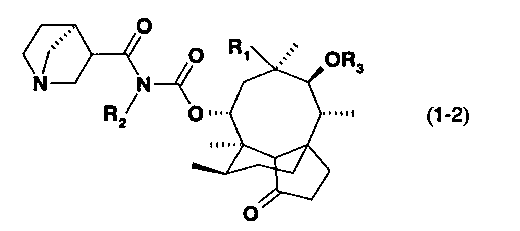

(wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or a substituted or unsubstituted aralkyl group; and R₃ is a protective group for hydroxyl group) (R₁ is neither an ethyl group nor a vinyl group).

(4) An intermediate for the production of the mutilin derivative or an acid-addition salt thereof according to 1) to 3) above, comprising a 4-epimutilin derivative represented by the following general formula (2-1):

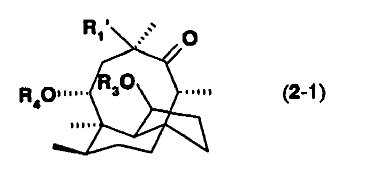

(wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₃ is a protective group for hydroxyl group; and R₄ is a hydrogen atom or a protective group for hydroxyl group) (R₁' is neither an ethyl group nor a vinyl group).

(5) An intermediate for the production of the mutilin derivative or an acid-addition salt thereof according to 1) to 4) above, comprising a mutilin derivative represented by the following general formula (2-2):

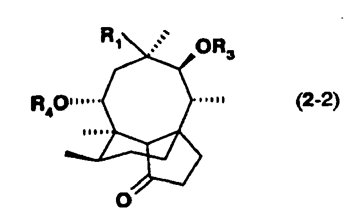

(wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; and R₃ and R₄ are each independently a hydrogen atom or a protective group for hydroxyl group) (R₁ is neither an ethyl group nor a vinyl group).

(6) A therapeutic agent for infectious diseases comprising as an active ingredient at least one mutilin derivative or an acid-addition salt thereof represented by the following general formula (1):

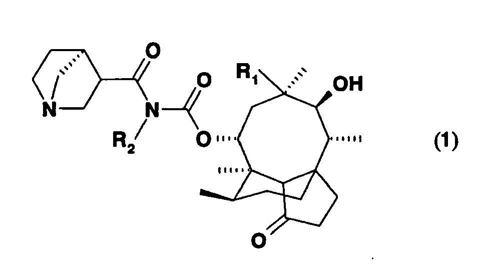

(wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; and R₂ is a hydrogen atom, a lower alkyl group or an aralkyl group whose aromatic ring may be optionally substituted) (R₁ is neither an ethyl group nor a vinyl group).

(7) A therapeutic agent for infectious diseases comprising as an active ingredient at least one mutilin derivative or an acid-addition salt thereof represented by the following general formula (1-1):

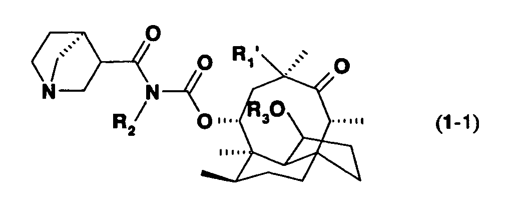

(wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or an aralkyl group whose aromatic ring may be optionally substituted; and R₃ is a protective group for hydroxyl group) (R₁' is neither an ethyl group nor a vinyl group).

(8) A therapeutic agent for infectious diseases comprising as an active ingredient at least one mutilin derivative or an acid-addition salt thereof represented by the following general formula (1-2):

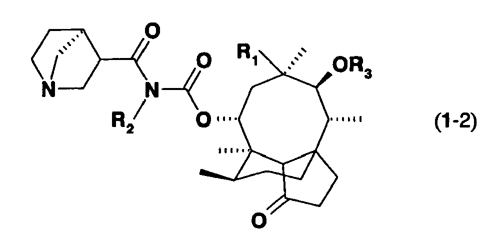

(wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or a substituted or unsubstituted aralkyl group; and R₃ is a protective group for hydroxyl group) (R₁ is neither an ethyl group nor a vinyl group).

### EFFECT OF THE INVENTION

Novel position 12-substituted mutilin derivatives having high antimicrobial activity, the compounds of the present invention can be used as an effective therapeutic agent for infectious diseases caused by Gram-positive or Gram-negative bacteria, including various drug-resistant bacteria.

### BEST MODE FOR CARRYING OUT THE INVENTION

In this description, the numbering of positions in the compound is based on the following mutilin chemistry, rather than the IUPAC nomenclature. According to literature (Tetrahedron, 1981, 37, 915-919), mutilin is named
(1S, 2R, 3S, 4S, 6R, 7R, 8R, 14R)-3,6-dihydroxy-2,4,7,14-tetramethyl-4-vinyl-tricyclo [5.4.3.0^{1,8}] tetradecan-9-one (IUPAC nomenclature).

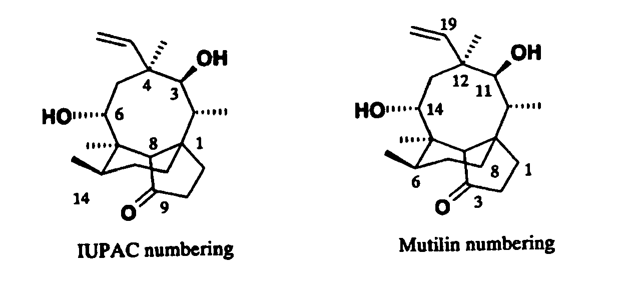

As shown in the chemical formula (3) below, the compound named (1R,2R,4S,6R,7R,8S,9R,14R)-6-hydroxy-9-methoxy-2,4,7,14-tetram ethyl-4-vinyl-1-tricyclo[5.4.3.01, 8]tetradecan-3-one according to the IUPAC nomenclature is named (3R)-3-deoxo-11-deoxy-3-methoxy-11-oxo-4-epimutilin according to mutilin chemistry.

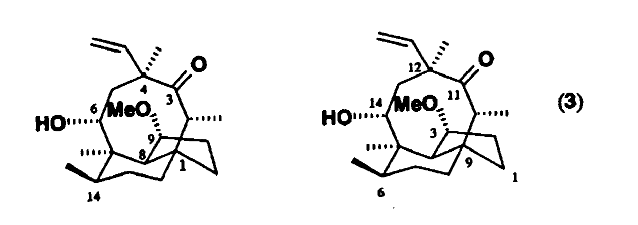

As used herein, the term "lower alkyl group" refers to a straight-chained or branched alkyl group having 1 to 6 carbon atoms, including methyl, ethyl, 1-methylethyl, 1,1-dimethylethyl, propyl and 2-methylpropyl. Its substituents include a lower alkoxy group, a halogen atom, a cyano group, a substituted or unsubstituted amino group, a substituted or unsubstituted thiol group, a lower acyloxy group, a lower alkyloxycarbonyl group, a lower alkylcarbonyl group, a lower alkylcaboxamide group, a nitro group, a 5- to 14-membered aliphatic heterocyclic ring that may have at least one substituent and that may contain at least one hetero atom such as O, N and S and 5- to 14-membered heteroaromatic ring that may have at least one substituent and that may contain at least one hetero atom such as O, N and S. The lower alkyl group may take any form: It may be straight-chained or cyclic, saturated or unsaturated.

As used herein, the term "aralkyl group" includes a benzyl group and a 1-phenylethyl group. Its substituents include a lower alkoxy group, a halogen atom, a cyano group, a substituted or unsubstituted amino group, a substituted or unsubstituted thiol group, a lower acyloxy group, a lower alkyloxycarbonyl group, a lower alkylcarbonyl group, a lower alkylcarboxamide group and a nitro group.

As used herein, the term "lower alkoxy group" refers to a straight-chained or branched alkoxy group having 1 to 6 carbon atoms, including a methoxy group, an ethoxy group, a 1-methylethoxy group, 1,1-dimethylethoxy group, a propoxy group and a 2-methylpropoxy group. The lower alkoxygroupmaybe either saturated or unsaturated.

As used herein, the term "aralkyloxy group" includes a benzyloxy group and a 1-phenylethoxy group. Its substituents include a lower alkoxy group, a halogen atom, a cyano group, a substituted or unsubstituted amino group, a substituted or unsubstituted thiol group, a lower acyloxy group, a lower alkyloxycarbonyl group, a lower alkylcarbonyl group, a lower alkylcarboxamide group and a nitro group.

As used herein, the term "lower acyloxy group" includes those having 1 to 5 carbon atoms, such as a formyl group, an acetoxy group, a propionyloxy group and 2,2-dimethylpropionyloxy group.

The term "at least one substituent" as in "5- to 14-membered aliphatic heterocyclic ring that may have at least one substituent and that may contain at least one hetero atom such as O, N and S" includes a halogen atom, a lower alkyl group, a lower alkoxy group, a lower alkylthio group, a lower alkoxycarbonyl group, a nitro group, a substituted or unsubstituted amino group and a cyano group. The term "aliphatic heterocyclic ring" in the same expression includes pyrrolidyl, piperidyl, piperadyl and morpholyl. The "amino group" in this case may be substituted with an acyl, such as acetyl, or it may be substituted with one or two lower alkyl groups. The term "at least one substituent" as in "5- to 14-membered heteroaromatic ring that may have at least one substituent and that may contain at least one hetero atom such as O, N and S" includes a halogen atom, a lower alkyl group, a lower alkoxy group, a lower alkylthio group, a lower alkoxycarbonyl group, a nitro group, an amino group and a cyano group. The term "heteroaromatic ring" in the same expression includes furanyl, thienyl, pyrazolyl, imidazolyl, oxazolyl, thiazolyl, pyridyl, pyrimidyl, pyridazyl and pyratyl. These rings may be fused with a benzene ring at any position. The "amino group" in this case may be substituted with an acyl, such as acetyl, or it may be substituted with one or two lower alkyl groups. The term "substituted or unsubstituted hydroxyl group" as used herein includes a hydroxyl group, a lower alkoxy group, a lower acyloxy group, a hydroxyl group having a protective group, an arylacyloxy group and a hydroxyl group that forms a leaving group with an oxygen atom. The term "arylacyl group" as used herein includes a benzoyl group. Its substituents include a lower alkyl group, a lower alkoxy group, a halogen atom, a cyano group and a nitro group. The protective group for hydroxyl group may be an trialkylsilyl group, such as a trimethylsilyl group and a t-butyldimethylsilyl group, an arylmethyl group, such as a benzyl group and a diphenylmethyl group, an acyl group, such as an acetyl group and a propionyl group, a lower alkoxymethyl group, such as a methoxymethyl group and an ethoxymethyl group, an aralkyloxymethyl group, such as a benzyloxymethyl group, or a tetrahydropyranyl group. These protective groups may be introduced or removed according to processes described in literature (Green, T. W.; Wuts, P. G. M. "Protective Groups in Organic Synthesis", 2ndEd., Wiley Interscience Publication, John-Weiley & Sons, New York, 1991. This literature is referred to as "Green et al.," hereinafter).

The term "leaving group formed with an oxygen atom" includes a lower alkylsulfonyloxy group and an arylsulfonyloxy group.

The term "substituted or unsubstituted thiol group" includes a thiol group, a lower thioalkoxy group, a lower acylthiooxy group, a thiol group having a protective group and an arylacylthiooxy group. The term "arylacyl group" includes a benzoyl group and the substituents include a lower alkyl group, a lower alkoxy group, a halogen atom, a cyano group and a nitro group. The protective group for thiol group may be a trialkylsilyl group, such as an trialkylsilyl group, such as a trimethylsilyl group and a t-butyldimethylsilyl group, an arylmethyl group, such as a benzyl group and a diphenylmethyl group, an acyl group, such as an acetyl group and a propionyl group, a lower alkoxymethyl group, such as a methoxymethyl group and an ethoxymethyl group, an aralkyloxymethyl group, such as a benzyloxymethyl group, or a tetrahydropyranyl group. These protective groups may be introduced or removed according to processes described in literature (Green et al.).

The term "substituted or unsubstituted amino group" includes an amino group, a lower alkylamino group, a lower acylamino group, an amino group having a protective group and an arylacylamino group. The protective group for amino group may be a lower acyl group, such as acetyl and propionyl, a lower alkoxycarbonyl group, such as ethoxycarbonyl and t-butoxycarbonyl, or a benzyl group. These protective groups may be introduced or removed according to processes described in literature (Green et al.).

The term "arylacyl group" includes a benzoyl group and the substituents include a lower alkyl group, a lower alkoxy group, a halogen atom, a cyano group and a nitro group.

Among preferred examples of the present invention are the following compounds:
14-{(3R,4S)-1-azabicyclo[2.2.1]heptane-3-carbonyl}carbamoyl-12 -desethenylmutilin,
14-{(3R,4S)-1-azabicyclo[2.2.1]heptane-3-carbonyl}carbamoyl-12 -desethenyl-12-methylmutilin,
14-{(3R,4S)-1-azabicyclo[2.2.1]heptane-3-carbonyl}carbamoyl-12 -desethenyl-12-methylmutilin hydrochloride,
14-{(3R,4S)-1-azabicyclo[2.2.1]heptane-3-carbonyl}carbamoyl-12 -desethenyl-12-(1-hydroxyethane-2-yl)mutilin,
14-{(3R,4S)-1-azabicyclo[2.2.1]heptane-3-carbonyl}carbamoyl-12 -desethenyl-12-(1-propene-3-yl)mutilin,
14-{(3R,4S)-1-azabicyclo[2.2.1]heptane-3-carbonyl}carbamoyl-12 -desethenyl-12-(1-propene-3-yl)mutilin hydrochloride,
14-{(3R,4S)-1-azabicyclo[2.2.1]heptane-3-carbonyl}carbamoyl-12 -desethenyl-12-phenylmethylmutilin,
14-{(3R,4S)-1-azabicyclo[2.2.1]heptane-3-carbonyl}carbamoyl-12 -desethenyl-12-phenylmethylmutilin hydrochloride,
14-{(3R,4S)-1-azabicyclo[2.2.1]heptane-3-carbonyl}carbamoyl-12 -desethenyl-12-(1-propyne-3-yl)mutilin,
14-{(3R,4S)-1-azabicyclo[2.2.1]heptane-3-carbonyl}carbamoyl-12 -desethenyl-12-(1-propyne-3-yl)mutilin hydrochloride,
12-(2-propyne-3-yl)-14-{(3R,4S)-1-azabicyclo[2.2.1]heptane-3-c arbonyl}carbamoyl-12-desethenylmutilin,
12- (2-butyne-4-yl) -14-{(3R, 4S) -1-azabicyclo [2.2.1] heptane-3-carbonyl}carbamoyl-12-desethenylmutilin,
12-(2-butyne-4-yl)-14-{(3R,4S)-1-azabicyclo[2.2.1]heptane-3-ca rbonyl}carbamoyl-12-desethenylmutilin hydrochloride,
14-{(3R,4S)-1-azabicyclo[2.2.1]heptane-3-carbonyl}carbamoyl-12 -desethenyl-12-(3-pentyne-5-yl)mutilin,
14-{(3R,4S)-1-azabicyclo[2.2.1]heptane-3-carbonyl}carbamoyl-12 -desethenyl-12-(1-fluoroethane-2-yl)mutilin,
14-{(3R,4S)-1-azabicyclo[2.2.1]heptane-3-carbonyl}carbamoyl-12 -desethenyl-12-(1,1,1-trifluoroethane-2-yl)mutilin,
14-{(3R,4S)-1-azabicyclo[2.2.1]heptane-3-carbonyl}carbamoyl-12 -desethenyl-12-[(2-methyl)ethane-2-yl]mutilin,
14-{(3R,4S)-1-azabicyclo[2.2.1]heptane-3-carbonyl}carbamoyl-12 -desethenyl-12-fluoromethylmutilin,
14-{(3R,4S)-1-azabicyclo[2.2.1]heptane-3-carbonyl}carbamoyl-12 -desethenyl-12-fluoromethylmutilin hydrochloride,
14-{(3R,4S)-1-azabicyclo[2.2.1]heptane-3-carbonyl}carbamoyl-12 -desethenyl-12-(4-pyridyl)methylmutilin hydrochloride,
14-{(3R,4S)-1-azabicyclo[2.2.1]heptane-3-carbonyl}carbamoyl-12 -desethenyl-12-[2-propene-3-yl]mutilin,
14-{(3R,4S)-1-azabicyclo[2.2.1]heptane-3-carbonyl}carbamoyl-12 -desethenyl-12-[2-propene-3-yl]mutilin hydrochloride,
14-{(3R,4S)-1-azabicyclo[2.2.1]heptane-3-carbonyl}carbamoyl-12 -desethenyl-N-methyl-12-[2-propene-3-yl]mutilin,
14-{(3R,4S)-1-azabicyclo[2.2.1]heptane-3-carbonyl}carbamoyl-12 -desethenyl-12-propylmutilin,
14-{(3R,4S)-1-azabicyclo[2.2.1]heptane-3-carbonyl}carbamoyl-12 -desethenyl-12-propylmutilin hydrochloride,
12-butyl-14-{(3R,4S)-1-azabicyclo[2.2.1]heptane-3-carbonyl}car bamoyl-12-desethenylmutilin,
14-{(3R,4S)-1-azabicyclo[2.2.1]heptane-3-carbonyl}carbamoyl-12 -(1-chloropropane-3-yl)-12-desethenylmutilin,
14-{(3R,4S)-1-azabicyclo[2.2.1]heptane-3-carbonyl}carbamoyl-12 -[(E)-2-butene-4-yl]-12-desethenylmutilin,
14-{(3R,4S)-1-azabicyclo[2.2.1]heptane-3-carbonyl}carbamoyl-12 -desethenyl-12-[(2-methyl)propane-3-yl]mutilin,
14-{(3R,4S)-1-azabicyclo[2.2.1]heptane-3-carbonyl}carbamoyl-12 -cyclohexyl-12-desethenylmutilin,
14-{(3R,4S)-1-azabicyclo[2.2.1]heptane-3-carbonyl}carbamoyl-12 -desethenyl-12-(1-methoxyethane-2-yl)mutilin,
14-{(3R,4S)-1-azabicyclo[2.2.1]heptane-3-carbonyl}carbamoyl-12 -(1-chloropropene-3-yl)-12-desethenylmutilin,
14-{(3R,4S)-1-azabicyclo[2.2.1]heptane-3-carbonyl}carbamoyl-12 -desethenyl-12-ethoxymethylmutilin,
14-{(3R,4S)-1-azabicyclo[2.2.1]heptane-3-carbonyl}carbamoyl-12 -desethenyl-12-(1-fluoropropene-3-yl)mutilin,
14-{(3R,4S)-1-azabicyclo[2.2.1]heptane-3-carbonyl}carbamoyl-12 -desethenyl-12-(1-fluoropropane-3-yl)mutilin,
14-{(3R,4S)-1-azabicyclo[2.2.1]heptane-3-carbonyl}carbamoyl-12 -desethenyl-12-[(E)-1-fluoroethene-2-yl]mutilin,
14-{(3R,4S)-1-azabicyclo[2.2.1]heptane-3-carbonyl}carbamoyl-12 -desethenyl-12-[(Z)-1-fluoroethene-2-yl]mutilin,
14-{(3R,4S)-1-azabicyclo[2.2.1]heptane-3-carbonyl}carbamoyl-12 -cyclopentyl-12-desethenylmutilin,
14-{(3R,4S)-1-azabicyclo[2.2.1]heptane-3-carbonyl}carbamoyl-12 -(2-cyclohexene-1-yl)-12-desethenylmutilin,
14-{(3R,4S)-1-azabicyclo[2.2.1]heptane-3-carbonyl}carbamoyl-12 -desethenyl-12-(3-methyl-1-propene-3-yl)mutilin,
14-{(3R,4S)-1-azabicyclo[2.2.1]heptane-3-carbonyl}carbamoyl-12 -desethenyl-12-(3-methyl-1-propyne-3-yl)mutilin,
(3R)-14-{(3R,4S)-1-azabicyclo[2.2.1]heptane-3-carbonyl}carbamo yl-12-cyclopropylmethyl-3-deoxo-11-deoxy-12-desethenyl-3-metho xy-11-oxo-4-epimutilin,
14-{(3R,4S)-1-azabicyclo[2.2.1]heptane-3-carbonyl}carbamoyl-12 -desethenyl-12-(1,1,1-trifluoro-2-propene-3-yl)mutilin,
14-{(3R,4S)-1-azabicyclo[2.2.1]heptane-3-carbonyl}carbamoyl-12 -(1-butyne-4-yl)-12-desethenylmutilin,
4-{(3R,4S)-1-azabicyclo[2.2.1]heptane-3-carbonyl}carbamoyl-12-desethenyl-12-methoxycarbonylmutilin,
14-{(3R,4S)-1-azabicyclo[2.2.1]heptane-3-carbonyl}carbamoyl-12 -desethenyl-12-[1-(E)-(ethoxycarbonyl)ethene-2-yl]mutilin hydrochloride,
14-{(3R,4S)-1-azabicyclo[2.2.1]heptane-3-carbonyl}carbamoyl-12 -desethenyl-12-(1-chloroethene-2-yl)mutilin,
14-{(3R,4S)-1-azabicyclo[2.2.1]heptane-3-carbonyl}carbamoyl-12 -desethenyl-12-(1-chloroethene-2-yl)mutilin hydrochloride,
14-{(3R,4S)-1-azabicyclo[2.2.1]heptane-3-carbonyl}carbamoyl-12 -desethenyl-12-methoxyiminomethylmutilin,
12-[(E)-1-butene-1-yl]-14-{(3R,4S)-1-azabicyclo[2.2.1]heptane-3-carbonyl}carbamoyl-12-desethenylmutilin,
12-[(E)-1-butene-1-yl]-14-{(3R,4S)-1-azabicyclo[2.2.1]heptane-3-carbonyl}carbamoyl-12-desethenylmutilin hydrochloride,
14-{(3R,4S)-1-azabicyclo[2.2.1]heptane-3-carbonyl}carbamoyl-12 -desethenyl-12-[(E)-1-pentene-1-yl]mutilin,
14-{(3R,4S)-1-azabicyclo[2.2.1]heptane-3-carbonyl}carbamoyl-12 -desethenyl-12-(1-phenylethene-2-yl)mutilin,
14-{(3R,4S)-1-azabicyclo[2.2.1]heptane-3-carbonyl}carbamoyl-12 -(buta-1,3-diene-1-yl)-12-desethenylmutilin,
14-{(3R,4S)-1-azabicyclo[2.2.1]heptane-3-carbonyl}carbamoyl-12 -[(Z)-2-butene-4-yl]-12-desethenylmutilin, and
14-{(3S,4S)-1-azabicyclo[2.2.1]heptane-3-carbonyl}carbamoyl-12 -desethenyl-12-[2-propene-3-yl]mutilin hydrochloride.

The compounds of the present invention may form pharmaceutically acceptable salts. For example, they may form inorganic salts with hydrochloric acid, hydrobromic acid, sulfuric acid, nitric acid or phosphoric acid, or organic salts with acetic acid, maleic acid, fumaric acid, succinic acid, lactic acid, malic acid, tartaric acid, citric acid, methanesulfonic acid, p-toluenesulfonic acid, benzenesulfonic acid, salicylic acid, stearic acid, palmitic acid or trifluoroacetic acid.

The compounds of the present invention include a plurality of asymmetric carbon atoms and may thus have a corresponding number of optical isomers. These optical isomers, as well as mixtures containing these isomers in any proportion, are also encompassed by the scope of the present invention.

The compounds of the present invention represented by the chemical formula (1) and salts thereof include any of intramolecular salts, adducts, solvates or hydrates thereof.

The compounds of the present invention or salts thereof can be used as a pharmaceutical composition either alone or in combination with one or more pharmaceutically acceptable auxiliary agents. Specifically, the compounds may be mixed with pharmaceutically acceptable carriers/excipients (such as starches, lactose, calcium phosphate and calcium carbonate), lubricants (such as magnesium stearate, calcium stearate talc and stearic acid), binders (such as starches, crystalline cellulose, carboxymethylcellulose, gum arabic, polyvinylpyrrolidone and alginic acid), disintegrating agents (such as talc and carboxymethylcellulose calcium) or diluents (such as physiological saline and aqueous solutions of glucose, mannitol and lactose) and may be formulated as tablets, capsules, granules, powders, fine powders, ampoules or injections for oral or parenteral administration. While the dose of the compounds of the chemical formula (1) according to the present invention may vary depending on the type of the compounds or salts thereof and the route of administration, as well as age, weight and the symptoms of patients, the compounds of the.chemical formula (1) or salts thereof are typically administered to mammals, including humans, at a dose of 0.0001 to 1000 mg/kg/day in single or multiple doses.

The compounds of the chemical formula (1) according to the present invention can be produced, for example, by the following process involving the compound of the chemical formula (3) as a key intermediate:

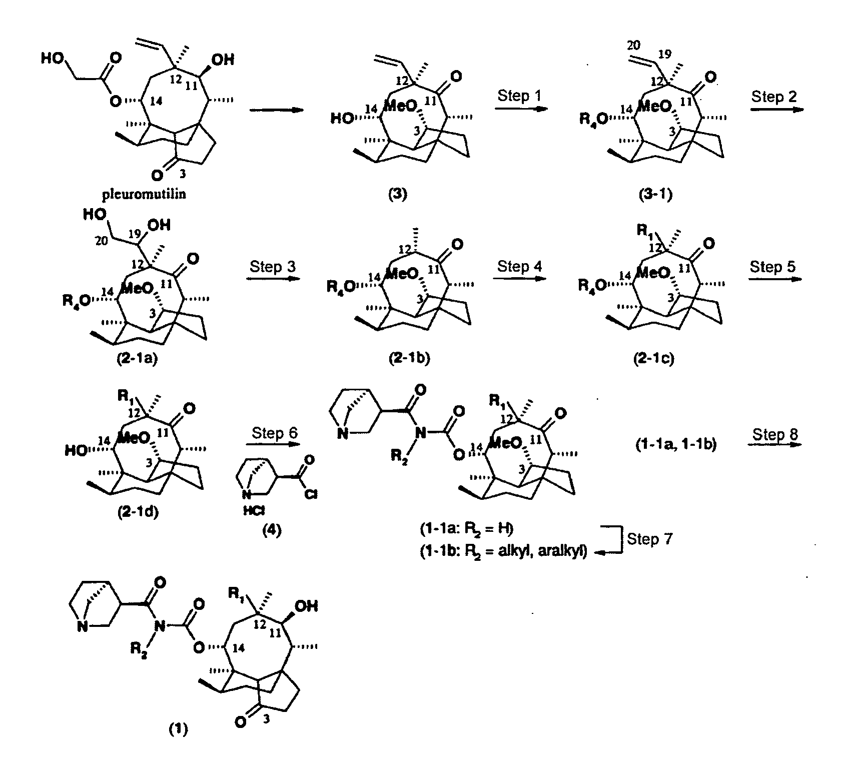

The compound shown by the chemical formula (4) is a known compound and can be produced by processes described in Patent Document (EP 257741 (Japanese Patent Publication No. Sho 63-39879), EP398617 (Japanese Patent Publication No. Hei 03-63272), EP 398629 (Japanese Patent Publication No. Hei 03-63280)) and Non-Patent Document (J. Med. Chem. 1991, 34, 2726-2735; Tetrahedron Lett. 1991, 32, 1241-1244; J. Med. Chem. 1992, 35, 911; J. Chem. Soc. Perkin I. 1991, 1091-1097; J. Org. Chem. 2001, 66, 2526-2529.).

### (Step I)

In Step I, a 4-epimutilin derivative represented by the chemical formula (3) is produced frompleuromutilin by a known process (See, for example, Tetrahedron 1980, 36, 1807-1811.). A protective group is then introduced into the 4-epimutilin derivative at the hydroxyl group at position 14 to form a 4-epimutilin derivative represented by the chemical formula (3-1) protected at the hydroxyl group at position 14.

The introduction of the protective group into hydroxyl group can be carried out by processes described in literature (Green et al.). Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, and aprotic polar solvents, such as acetonitrile, propionitrile, nitromethane, nitroethane, N,N-dimethylformamide, N,N-dimethylacetamide and dimethylsulfoxide. Typically the reaction proceeds smoothly at -20°C to 200°C.

### (Step II)

Step I gives a 4-epimutilin derivative represented by the chemical formula (3-1) that is protected at the hydroxyl group at position 14. In Step II, the double bond at position 12 of the 4-epimutilin derivative represented by the chemical formula (3-1) is converted to a diol to form a 4-epimutilin 19, 20-diol derivative represented by the chemical formula (2-1a).

The process typically uses a catalytic amount of an osmium derivative and an equal or excess amount of an oxidizing agent.
Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, aprotic polar solvents, such as N,N-dimethylformamide, N,N-dimethylacetamide and dimethylsulfoxide, and mixed solvents of water and these solvents. The reaction proceeds smoothly at 0°C to 200°C.

### (Step III)

Step II gives a 4-epimutilin 19, 20-diol derivative represented by the chemical formula (2-1a). In Step III, the diol moiety of the 4-epimutilin 19,20-diol derivative is eliminated by a retro-aldol reaction to form a 12-desethenyl-4-epimutilin derivative represented by the chemical formula (2-1b).

The reaction generally requires the presence of a particular reagent. Examples of such reagents include alkali metal alkoxides, such as sodium methoxide and sodium ethoxide, alkali metal hydrides, such as sodium hydride and potassium hydride, alkali metal organic bases, such as n-butyllithium, lithium bis(trimethylsilyl)amide, sodium bis(trimethylsilyl)amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodiumbicarbonate. When necessary, a Lewis acid may be added, such as zinc chloride, zinc bromide, zinc iodide, boron trifluoride, aluminum chloride, tin tetrachloride, boron trichloride-diethyl ether complex and lithium perchlorate. Any solvent that is not involved in the reaction may be used in this process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, and ether solvents, such as diethyl ether, tetrahydrofuran, 1,4-dioxane and dimethoxyethane. The reaction proceeds smoothly at -110°C to 100°C.

### (Step IV)

Step III gives a 12-desethenyl-4-epimutilin derivative represented by the chemical formula (2-1b). In Step IV, an appropriate electrophile is reacted with the 12-desethenyl-4-epimutilin derivative at position 12 in the presence of a base to form a 12-R₁ substituted 4-epimutilin derivative represented by the chemical formula (2-1c).

The reaction generally requires the presence of a particular reagent and an equal or excess amount of an electrophile. Examples of such reagents include alkali metal alkoxides, such as sodium methoxide and sodium ethoxide, alkali metal hydrides, such as sodium hydride and potassium hydride, alkali metal organic bases, such as n-butyllithium, lithium diisopropylamide, lithium bis(trimethylsilyl)amide, sodium bis(trimethylsilyl)amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodiumbicarbonate. When necessary, a Lewis acid may be added, such as zinc chloride, zinc bromide, zinc iodide, boron trifluoride, aluminum chloride, tin tetrachloride, boron trichloride-diethyl ether complex and lithium perchlorate. Any solvent that is not involved in the reaction may be used in this process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, and aprotic polar solvents, such as N,N-dimethylformamide, N,N-dimethylacetamide and dimethylsulfoxide. The reaction proceeds smoothly at -110°C to 100°C.

### (Step V)

Step IV gives a 12-R₁ substituted 4-epimutilin derivative represented by the chemical formula (2-1c) In Step V, the protective group for the hydroxyl group at position 14 of the 12-R₁ substituted 4-epimutilin derivative is removed to form a 14-hydroxy-4-epimutilin derivative represented by the chemical formula (2-1d) . The removal of the protective group for hydroxyl group can be carried out by processes described in literature (Green et al.) . Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol. The reaction proceeds smoothly at -110°C to 100°C.

### (Step VI)

Step V gives a 14-hydroxy-4-epimutilin derivative represented by the chemical formula (2-1d). In Step VI, a carboxylic acid chloride derivative of the bicyclic amine represented by the general formula (4) is reacted with the 14-hydroxy-4-epimutilin derivative under appropriate reaction conditions to add an acylcarbamoyl group to the hydroxyl group at position 14 to form a 14-acylcarbamoyl-4-epimutilin derivative represented by the chemical formula (1-1a).

This step can be carried out by any of the following processes described in literature: (A) reacting a carboxylic acid chloride derivative of the bicyclic amine represented by the general formula (4) and silver cyanate with the 14-hydroxy-4-epimutilin derivative represented by the chemical formula (2-1d) in the presence of a base (J. Org. Chem. 1962, 27, 3317.), or using tributyltin isocyanate (Chem. Ber. 1986, 119, 83.); (B) adding a carbamoyl group to the hydroxyl group at position 14 of the 14-hydroxy-4-epimutilin derivative represented by the chemical formula (2-1d) under normal reaction conditions, and binding a carboxylic acid chloride derivative of the bicyclic amine represented by the general formula (4) in the presence of a base; (C) reacting the 14-hydroxy-4-epimutilin derivative represented by the chemical formula (2-1d) with trimethylsilyl isocyanate and a carboxylic acid chloride derivative of the bicyclic amine represented by the general formula (4) in the presence of a base (J. Gen. Chem. USSR, 1977, 2061-2067.); or (D) amidating the carboxylic acid of a carboxylic acid chloride derivative of the bicyclic amine represented by the general formula (4) in the presence of a base under normal reaction conditions, and reacting the 14-hydroxy-4-epimutilin derivative represented by the chemical formula (2-1d) with a carbonyl source, such as oxalyl chloride, phosgene or 1,1,-carbonyldiimidazole, in the presence of a base such as a bis (trimethylsilyl) amide salt (18) J. Org. Chem. 1962, 27, 3742.). These processes are generally carried out in the presence of a base. Examples of such bases include alkali metal alkoxides, such as sodiummethoxide and sodiumethoxide, alkali metal hydrides, such as sodium hydride and potassium hydride, alkali metal organic bases, such as n-butyllithium, lithium diisopropylamide, lithium bis(trimethylsilyl)amide, sodium bis(trimethylsilyl)amide and potassium bis (trimethylsilyl) amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodium bicarbonate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, and ether solvents, such as diethyl ether, tetrahydrofuran, 1,4-dioxane and dimethoxyethane. Each reaction proceeds smoothly at -110°C to 100°C.

### (Step VII)

Step VI gives a 14-acylcarbamoyl-4-epimutilin derivative represented by the chemical formula (1-1a). In Step VII, a substituent R₂ (except a hydrogen atom) is introduced into the nitrogen atom at the spacer site (position 14) of the 14-acylcarbamoyl-4-epimutilin derivative to form a 14-acylcarbamoyl-4-epimutilin derivative represented by the chemical formula (1-1b) . Examples of the base used in the process include alkali metal alkoxides, such as sodium methoxide and sodium ethoxide, alkali metal hydrides, such as sodium hydride and potassium hydride, alkali metal organic bases, such as n-butyllithium, lithium diisopropylamide, lithium bis(trimethylsilyl)amide, sodium bis(trimethylsilyl)amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodium bicarbonate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, and aprotic polar solvents, such as N,N-dimethylformamide, N,N-dimethylacetamide and dimethylsulfoxide. The reaction proceeds smoothly at -110°C to 100°C.

### (Step VIII)

Step VII gives a 14-acylcarbamoyl-4-epimutilin derivative represented by the chemical formula (1-1a, 1-1b). In Step VIII, the protective group at position 3 of the 14-acylcarbamoyl-4-epimutilin derivative is removed to form a 14-acylcarbamoyl mutilin derivative represented by the general formula (1). The removal of the protective group can be carried out by processes described in literature (Green et al.) and preferably involves the use of hydrochloric acid or zinc chloride-hydrochloric acid (Lucas reagent). Any solvent that is not involved in the reaction may be used in this process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, aprotic polar solvents, such as acetonitrile, propionitrile, nitromethane, nitroethane, N,N-dimethylformamide, N,N-dimethylacetamide and dimethylsulfoxide, and mixed solvents of water and these solvents. The process may be carried out either in the presence or in the absence of these solvents. Typically the reaction proceeds smoothly at -20°C to 200°C.

Alternatively, the compounds represented by the chemical formula (1) according to the present invention may be produced by the following process using pleuromutilin as a starting material:

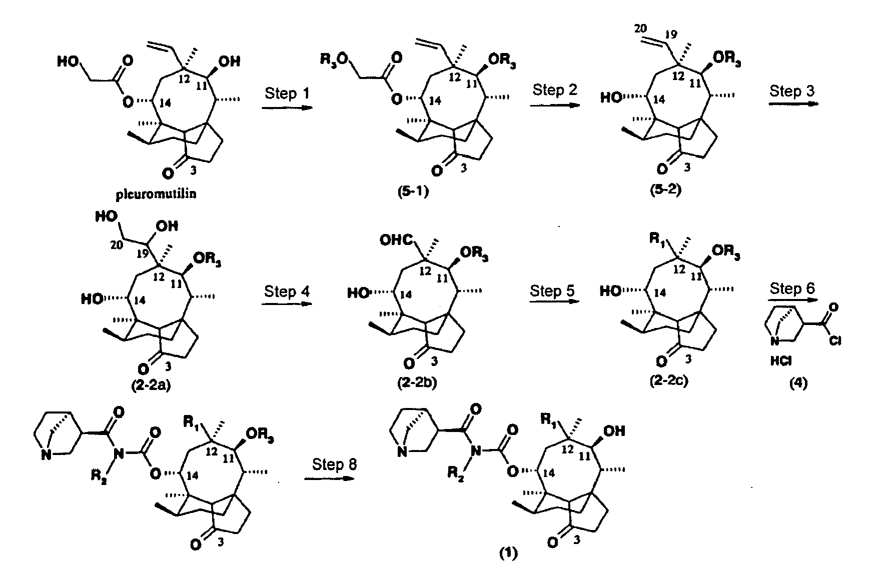

### (Step I)

In Step I, two protective groups are introduced into preuromutilin at the hydroxyl group of the glycolate moiety at position 14 and the hydroxyl group at position 11, respectively, to form a preuromutilin derivative having two protected hydroxyl groups, as represented by the chemical formula (5-1).

The introduction of the hydroxyl groups in this process can be carried out by processes described in literature (Green et al.). Any solvent that is not involved in the reaction may be used in the process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, and aprotic polar solvents, such as acetonitrile, propionitrile, nitromethane, nitroethane, N,N-dimethylformamide, N,N-dimethylacetamide and dimethylsulfoxide, and mixed solvents of water and these solvents . The process may be carried out either in the presence or in the absence of theses solvents. Typically the reaction proceeds smoothly at -20°C to 200°C.

### (Step II)

Step I gives a preuromutilin derivative represented by the chemical formula (5-1) having two protected hydroxyl groups. In Step II, the glycolate moiety at position 14 of the pleuromutilin derivative is hydrolyzed to form a mutilin derivative represented by the chemical formula (5-2) with only the hydroxyl group at position 11 protected.

The reaction generally requires the presence of a particular reagent. Examples of such reagents include alkali metal alkoxides, such as sodium methoxide and sodium ethoxide, alkali metal hydrides, such as sodium hydride and potassium hydride, alkali metal organic bases, such as n-butyllithium, lithium diisopropylamide, lithium bis(trimethylsilyl)amide, sodium bis(trimethylsilyl)amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodium bicarbonate. When necessary, a Lewis acid may be added, such as zinc chloride, zinc bromide, zinc iodide, boron trifluoride, aluminum chloride, tin tetrachloride, boron trichloride-diethyl ether complex and lithium perchlorate. Alternatively, hydrochloric acid may be added. Any solvent that is not involved in the reaction may be used in this process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, aprotic polar solvents, such as N,N-dimethylformamide, N, N-dimethylacetamide and dimethylsulfoxide, and mixed solvents of water and these solvents. The process may be carried out either in the presence or in the absence of these solvents. The reaction proceeds smoothly at -110°C to 100°C.

### (Step 3)

Step II gives a mutilin derivative represented by the chemical formula (5-2) with the hydroxyl group at position 11 protected. In Step (III), the double bond at positions 19, 20 is converted to a diol to form a mutilin 19,20-diol derivative represented by the chemical formula (2-2a) with the hydroxyl group at position 11 protected.

The process typically uses a catalytic amount of an osmium derivative and an equal or excess amount of an oxidizing agent. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, and mixed solvents of water and these solvents. The reaction proceeds smoothly at 0°C to 200°C.

### (Step IV)

Step (III) gives a mutilin 19,20-diol derivative represented by the chemical formula (2-2a) with the hydroxyl group at position 11 protected. In Step (IV), the mutilin 19,20-diol derivative is subjected to diol cleavage to form a 12-formyl-mutilin derivative represented by the chemical formula (2-2b) with the hydroxyl group at position 11 protected.

The process typically uses sodium periodate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, and mixed solvents of water and these solvents. The reaction proceeds smoothly at -110°C to 100°C.

### (Step V)

Step (IV) gives a 12-formyl-mutilin derivative represented by the chemical formula (2-2b) with the hydroxyl group at position 11 protected. In Step (V), the 12-formyl-mutilin derivative is subjected to various conversion processes that convert the formyl group at position 12 to form a 12-R₁ substituted mutilin derivative represented by the chemical formula (2-2c) with the hydroxyl group at position 11 protected. For example, the 12-formyl-mutilin derivative may be subjected to the Wittig reaction involving a phosphonium salt. The phosphonium salt may be methyltriphenylphosphonium chloride, methyltriphenylphosphonium bromide, methyltriphenylphosphonium iodide or (ethyl)triphenylphosphonium bromide. The process may be carried out in the presence of a base. Such a base may be an alkali metal alkoxide, such as sodium methoxide and sodium ethoxide, an alkali metal hydride, such as sodium hydride and potassium hydride, an alkali metal organic base, such as n-butyllithium, lithium diisopropylamide, lithium bis(trimethylsilyl)amide, sodium bis(trimethylsilyl)amide and potassium bis (trimethylsilyl) amide, a tertiary organic base, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, or an inorganic base, such as potassium carbonate and sodium bicarbonate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol. The reaction proceeds smoothly at -110°C to 100°C. Alternatively, the 12-formyl-mutilin derivative may be converted to a carboxylic acid derivative by oxidizing the formyl group. The process is carried out in the presence of an oxidizing agent, such as sodium bromate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, and mixed solvents of water and these solvents. The reaction proceeds smoothly at -110°C to 100 °C. The 12-formyl-mutilin derivative may be reacted with a hydroxylamine derivative to produce an oxime derivative. The process uses hydroxylamine chloride or its derivative. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, aprotic polar solvents, such as N,N-dimethylformamide, N,N-dimethylacetamide and dimethylsulfoxide, and mixed solvents of water and these solvents. The reaction proceeds smoothly at -110°C to 100°C.

### Step (VI)

Step (V) gives a 12-R₁ substituted mutilin derivative represented by the chemical formula (2-2c) with the hydroxyl group at position 11 protected. In Step (VI), a carboxylic acid chloride derivative of the bicyclic amine of the general formula (4) is reacted with the 12-R₁ substitutedmutilin derivative to add an acylcarbamoyl group to the hydroxyl group at position 14 to form a 14-acylcarbamoyl-mutilin derivative represented by the chemical formula (1-2a) with the hydroxyl group at position 11 protected.

This step can be carried out by any of the following processes described in literature: (A) reacting, in the presence of a base, a carboxylic acid chloride derivative of the bicyclic amine represented by the general formula (4) and silver cyanate with the 14-hydroxy-mutilin derivative represented by the chemical formula (2-2c) (with the hydroxyl group at position 11 protected) (J. Org. Chem. 1962, 27, 3317.), or using tributyltin isocyanate (Chem. Ber. 1986, 119, 83.) ; (B) adding a carbamoyl group to the hydroxyl group at position 14 of the 14-hydroxy-mutilin derivative represented by the chemical formula (2-2c) (with the hydroxyl group at position 11 protected) under normal reaction conditions, and binding a carboxylic acid chloride derivative of the bicyclic amine represented by the general formula (4) in the presence of a base; (C) reacting the 14-hydroxy-mutilin derivative represented by the chemical formula (2-2c) (with the hydroxyl group at position 11 protected) with trimethylsilyl isocyanate and a carboxylic acid chloride derivative of the bicyclic amine represented by the general formula (4) in the presence of a base (J. Gen. Chem. USSR, 1977, 2061-2067.); or (D) amidating the carboxylic acid of a carboxylic acid chloride derivative of the bicyclic amine represented by the general formula (4) in the presence of a base under normal reaction conditions, and reacting the 14-hydroxy-mutilin derivative represented by the chemical formula (2-2c) (with the hydroxyl group at position 11 protected) with a carbonyl source, such as oxalyl chloride, phosgene or 1,1,-carbonyldiimidazole, in the presence of a base such as a bis(trimethylsilyl)amide salt (J. Org. Chem. 1962, 27, 3742.). These processes are generally carried out in the presence of a base. Examples of such bases include alkali metal alkoxides, such as sodium methoxide and sodium ethoxide, alkali metal hydrides, such as sodium hydride and potassium hydride, alkali metal organic bases, such as n-butyllithium, lithium diisopropylamide, lithium bis(trimethylsilyl)amide, sodium bis(trimethylsilyl)amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodium bicarbonate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, and ether solvents, such as diethyl ether, tetrahydrofuran, 1,4-dioxane and dimethoxyethane. Each reaction proceeds smoothly at -110°C to 100°C.

### (Step VII)

Step VI gives a 14-acylcarbamoyl-mutilin derivative represented by the chemical formula (1-2a) with the hydroxyl group at position 11 protected. In Step VII, a substituent R₂ (except hydrogen atom) is introduced into the nitrogen atom at the spacer site (position 14) of the 14-acylcarbamoyl-mutilin derivative to form a 14-acylcarbamoyl-mutilin derivative represented by the chemical formula (1-2b) with the hydroxyl group at position 11 protected. Examples of the base used in the process include alkali metal alkoxides, such as sodiummethoxide and sodium ethoxide, alkali metal hydrides, such as sodium hydride and potassium hydride, alkali metal organic bases, such as n-butyllithium, lithium diisopropylamide, lithium bis(trimethylsilyl)amide, sodium bis (trimethylsilyl) amide and potassium bis(trimethylsilyl)amide, tertiary organic bases, such as triethylamine, diisopropylethylamine, pyridine, N-methylmorpholine, imidazole, pyrrolidine, piperidine, 1,5-diazabicyclo[4.3.0]nona-5-ene and 1,8-diazabicyclo[5.4.0]unde-7-cene, and inorganic bases, such as potassium carbonate and sodium bicarbonate. Any solvent that is not involved in the reaction may be used in this process. Examples of such solvents include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, alcohol solvents, such as methanol, ethanol, 1-propanol, 2-propanol, 1-butanol, 2-butanol, 2-methyl-1-propanol and 2-methyl-2-propanol, and aprotic polar solvents, such as N,N-dimethylformamide, N,N-dimethylacetamide and dimethylsulfoxide. The reaction proceeds smoothly at -110°C to 100°C.

### (Step VIII)

Step VII gives a 14-acylcarbamoyl-mutilin derivative represented by the chemical formula (1-2a, 1-2b). In Step VIII, the protective group for the hydroxyl group at position 11 of the 14-acylcarbamoyl-mutilin derivative is removed to form a 14-acylcarbamoyl mutilin derivative represented by the general formula (1). The removal of the protective group can be carried out by processes described in literature (Green et al.) and preferably involves the use of hydrochloric acid or zinc chloride-hydrochloric acid (Lucas reagent). Any solvent that is not involved in the reaction may be used in this process. Examples include hydrocarbon solvents, such as pentane, hexane, cyclohexane, benzene, toluene and xylene, halogenated hydrocarbon solvents, such as dichloromethane, 1,2-dichloroethane, chloroform and carbon tetrachloride, ether solvents, such as diethyl ether, tetrahydrofuran, 1,4-dioxane and dimethoxyethane, aprotic polar solvents, such as acetonitrile, propionitrile, nitromethane, nitroethane, N,N-dimethylformamide, N,N-dimethylacetamide and dimethylsulfoxide, and mixed solvents of water and these solvents. The process may be carried out either in the presence or in the absence of these solvents. Typically the reaction proceeds smoothly at -20°C to 200°C.

### Examples

The present invention will now be described with reference to Examples and Reference Examples, which are not intended to limit the scope of the invention in any way.

### (Reference Example 1)

### (3R)-3-deoxo-11-deoxy-3-methoxy-14-O-methoxymethyl-11-oxo-4-ep imutilin

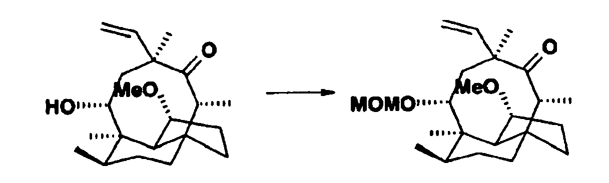

According to a two-step process described in literature (Tetrahedron 1980, 36, 1807-1811.), 4-epimutilin was produced from pleuromutilin. 2.00 g (5.98 mmol) of this product was dissolved in methylene chloride (50 mL). To this solution,2.08 mL (12.0 mmol) diisopropylethylamine and 0.91 mL (12.0 mmol) chloromethyl methyl ether were sequentially added at 0°C in an argon atmosphere. The resulting mixture was stirred at room temperature for 60 hours. Subsequently, the reaction mixture was poured into cold diluted aqueous citric acid. The solvent was evaporated under reduced pressure and the residue was extracted with ethyl acetate (20 mL x 3) . The organic layers were combined, washed with saturated brine (20 mL), and dried over anhydrous magnesium sulfate. The dried product was then filtered and the solvent was removed. Purification of the residue by silica gel column chromatography (hexane:ethyl acetate = 8:1) afforded 2.30 g of the title compound as a colorless oil (100% yield).
MS (FAB) (m/z): 379 (MH⁺).
HRMS (FAB) (m/z) : Calcd. for C₂₃H₃₉O₄(MH⁺) : 379.2848. Found, 379.2883.

### (Reference Example 2)

(3R)-3-deoxo-11-deoxy-19,20-dihydroxy-3-methoxy-14-O-methoxyme thyl-11-oxo-4-epimutilin

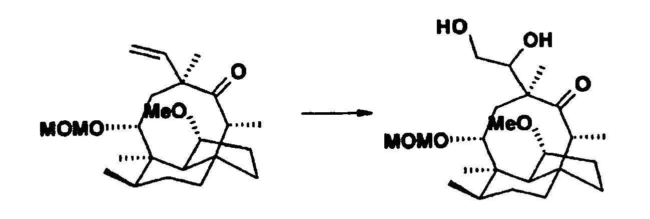

60.3 g (0.52 mol) of 4-methylmorpholine N-oxide, along with a catalytic amount of osmium tetraoxide (in 5% t-butanol solution), was added to a solution of 130 g (0.34 mol) of the compound of Reference Example 1 in aqueous acetone (800 mL). The mixture was refluxed for 60 hours. Subsequently, the reaction mixture was evaporated under reduced pressure and diluted aqueous acetic acid was added to the residue. The resulting mixture was extracted with ethyl acetate (500 mL x 3) . The organic layers were combined, washed with saturated brine (500 mL) and dried over anhydrous sodium sulfate. The dried product was then filtered and the solvent was removed. Purification of the residue by silica gel column chromatography (hexane:ethyl acetate = 1:1, followed by ethyl acetate:methanol = 10:1) afforded 135 g of the title compound as a yellow oil (95% yield).
MS (FAB) (m/z): 413 (MH⁺).
HRMS (FAB) (m/z) : Calcd. for C₂₃H₄₁O₆(MH⁺) : 413.2903. Found, 413.2933.

### (Reference Example 3)

(3R)-3-deoxo-11-deoxy-12-desethenyl-3-methoxy-14-O-methoxymeth yl-11-oxo-4-epimutilin

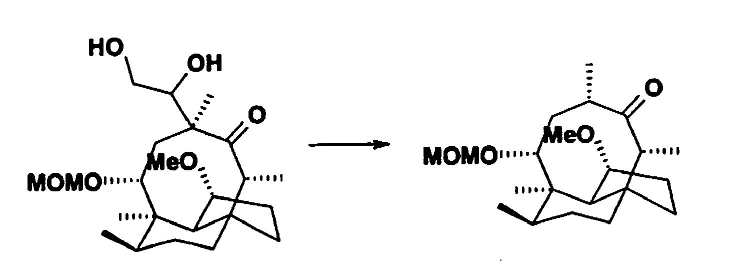

At 0°C, 63.4 g (0.46 mol) of potassium carbonate was added to a solution of 94.6 g (0.23 mol) of the compound of Reference Example 2 in acetone (1000 mL). The reaction mixture was stirred at room temperature for 4 hours. Subsequently, the mixture was filtered through Celite and the residue was washed with ethyl acetate.
The filtrate was evaporated under reduced pressure. To the resulting residue, diluted aqueous citric acid (1000 mL) was added and the mixture was extracted with ethyl acetate (500 mL x 3). The organic layers were combined, washed with saturated brine (500 mL) and dried over anhydrous sodium sulfate. The dried product was filtered and the solvent was removed. Purification of the resulting residue by silica gel column chromatography (hexane:ethyl acetate = 8:1) afforded 70.2 g of the title compound as a yellow powder (87% yield). MS (FAB) (m/z) : 353 (MH⁺) .
HRMS (FAB) (m/z) : Calcd. for C₂₁H₃₇O₄ (MH⁺) : 353.2692. Found, 353.2720.

### (Reference Example 4)

(3R)-3-deoxo-11-deoxy-12-desethenyl-3-methoxy-11-oxo-4-epimuti lin

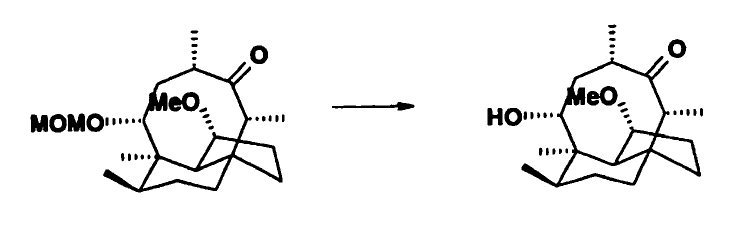

523 mg (2.75 mmol) of p-toluenesulfonic acid was added to a solution of 970 mg (2.75 mmol) of the compound of Reference Example 3 in methylene chloride (30 mL). The reaction mixture was stirred at room temperature for 24 hours. Subsequently, the mixture was evaporated under reduced pressure, followed by addition of water and extraction with ethyl acetate (20 mL x 3). The organic layers were combined, washed with saturated brine (20 mL) and dried over anhydrous magnesium sulfate. The dried product was filtered and the solvent was removed. Purification of the resulting residue by silica gel column chromatography (hexane:ethyl acetate = 2:1) afforded 772 mg of the title compound as a colorless powder (91% yield).
MS (FAB) (m/z) : 291 (MH⁺-H₂O).
HRMS (FAB) (m/z): Calcd. for C₁₉H₃₁O₂(MH⁺-H₂O) : 291.2324. Found, 291.2317.

### (Reference Example 5)

### Step I

(3R)-3-deoxo-11-deoxy-12-desethenyl-3-methoxy-12,14-O,O-dimeth oxymethyl-11-oxo-4-epimutilin

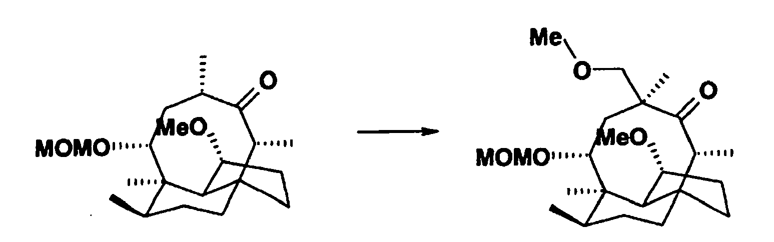

According to Step I of Example 2, 2.00 g (5.67 mmol) of the compound of Reference Example 3, 0.52 mL (6.81 mmol) chloromethyl methyl ether and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 4:1) to afford 1.80 g of the title compound as a yellow oil (80% yield).
MS (FAB) (m/z) : 397 (MH⁺) .
HRMS (FAB) (m/z) : Calcd. for C₂₃H₄₁O₅(MH⁺): 397.2954. Found, 397.2926.

### Step II

(3R)-3-deoxo-11-deoxy-12-desethenyl-3-methoxy-12-methoxymethyl -11-oxo-4-epimutilin

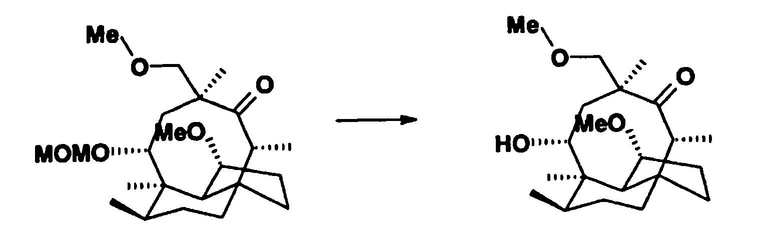

According to Reference Example 4, 1.80 g (4.54 mmol) of the compound of Step I and 0.86 g (4.54 mmol) p-toluenesulfonic acid were used in the reaction. The resulting residue was purified by silica gel column chromatography (hexane:ethyl acetate = 1:1) to afford 1.30 g of the title compound as a colorless powder (81% yield). MS (FAB) (m/z): 353 (MH⁺) .
HRMS (FAB) (m/z) : Calcd. for C₂₁H₃₇O₄(MH⁺): 353.2692. Found, 353.2656.

### (Reference Example 6)

(3R)-12-acetoxymethyl-3-deoxo-11-deoxy-12-desethenyl-3-methoxy -11-oxo-4-epimutilin

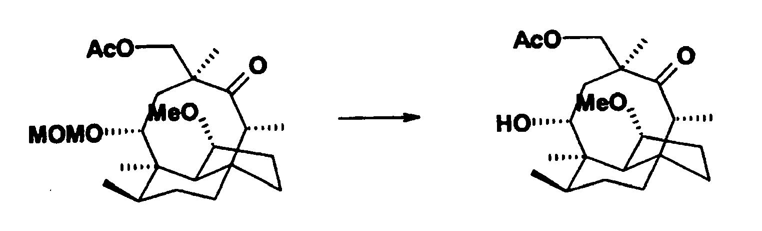

According to Reference Example 4, 600 mg (1.41 mmol) (3R)-3-deoxo-11-deoxy-12-desethenyl-12-acetoxymethyl-3-methoxy -14-O-methoxymethyl-11-oxo-4-epimutilin and 26.9 mg (0.14 mmol) p-toluenesulfonic acid were used in the reaction. The resulting residue was purified by silica gel column chromatography (hexane:ethyl acetate = 2:1) to afford 156 mg of the title compound as a colorless oil (29% yield).
MS (FAB) (m/z) : 381 (MH⁺).
HRMS (FAB) (m/z): Calcd. for C₂₂H₃₇O₅ (MH⁺) : 381.2641. Found, 381.2656.

### (Reference Example 7)

### Step I

(3R)-12-cyanomethyl-3-deoxo-11-deoxy-12-desethenyl-3-methoxy-1 4-O-methoxymethyl-11-oxo-4-epimutilin

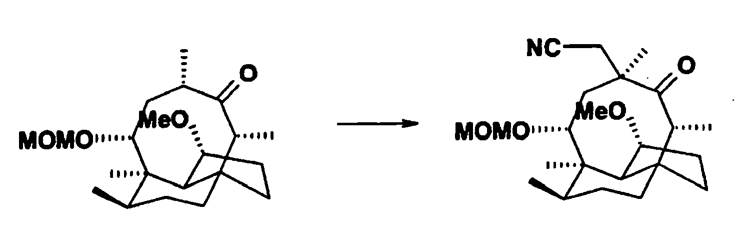

According to Step I of Example 2, 3.00 g (8.51 mmol) of the compound of Reference Example 3, 0.65 mL (10.2 mmol) chloroacetonitrile and 20.4 mL (10.2 mmol) potassium bis(trimethylsilyl)amide (0.5 mol/L toluene solution) were used in the reaction. The resulting residue was purified by silica gel column chromatography (hexane:ethyl acetate = 5:1, followed by hexane:ethyl acetate = 2:1) to afford 2.10 g of the title compound as a yellow oil (2.10 g, 63% yield). MS (FAB) (m/z): 330 (MH⁺-HOCH₂OCH₃).
HRMS (FAB) (m/z) : Calcd. for C₂₁H₃₂NO₂ (MH⁺-HOCH₂OCH₃) : 330.2433. Found, 330.2426.

### Step II

(3R)-12-cyanomethyl-3-deoxo-11-deoxy-12-desethenyl-3-methoxy-1 1-oxo-4-epimutilin

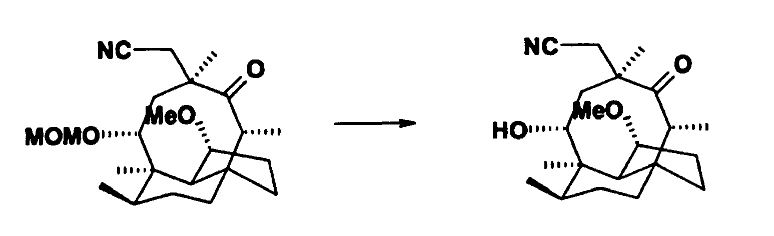

According to Reference Example 4, 2.10 g (5.36 mmol) of the compound of Step I and 1.02 g (5.36 mmol) p-toluenesulfonic acid were used in the reaction. The resulting residue was purified by silica gel column chromatography (hexane:ethyl acetate = 2:1) to afford 1.31 g of the title compound as a colorless powder (70% yield) .
MS (FAB) (m/z) : 330 (MH⁺-H₂O).
Rf = 0.23 (hexane:ethyl acetate = 2:1)

### (Reference Example 8)

(3R)-3-deoxo-11-deoxy-12-desethenyl-3-methoxy-11-oxo-12-[1-(1, 2,3-triazol-1-yl)ethyl]-4-epimutilin

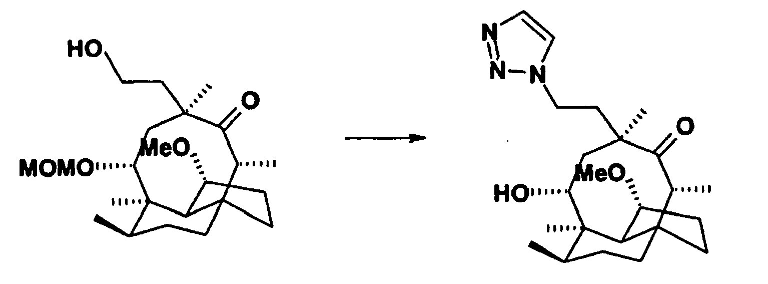

In an argon atmosphere, 4.74 mL (34.0 mmol) triethylamine and 1. 32 mL (17.0 mmoL) methanesulfonyl chloride were sequentially added to a solution of 4.49 g (11.3 mmol) (3R)-3-deoxo-11-deoxy-12-desethenyl-12-(1-hydroxyethane-2-yl)-3-methoxy-14-O-methoxymethyl-11-oxo-4-epimutilin in ice-cooled methylene chloride (100 mL). The mixture was then stirred for 2 hours as it was allowed to warm to room temperature. Subsequently, the reaction mixture was poured into diluted aqueous citric acid. The solvent was evaporated under reduced pressure and the residue was extracted with ethyl acetate (30 mL x 3) . The organic layers were combined, washed with saturated brine (30 mL) and dried over anhydrous magnesium sulfate. The dried product was filtered and the solvent was removed. Purification of the resulting residue by column chromatography (hexane:ethyl acetate = 2:1) gave 4.35 g of a colorless oil (81% yield).

2.30 g (4.85 mmol) of this product was dissolved in N,N-dimethylforamide (30 mL) . To this solution, 0.47 g (7.27 mmol) sodium azide was added and the mixture was stirred for 4 hours while heated at 80°C. After cooling, the reaction mixture was poured into cold water and was extracted with ethyl acetate (30 mL x 3) . The organic layers were combined, washed with saturated brine (30 mL) and dried over anhydrous magnesium sulfate. Filtering the dried product followed by removal of the solvent gave a crude azide product. To this product, 5.23 mL (48.5 mmol) bicyclo [2.2. 1] hepta-2, 5-diene was added and the mixture was stirred for 1 hour while heated at 80°C. After cooling, the reaction mixture was diluted with dioxane (20 mL) and was refluxed for 1 hour. Subsequently, the mixture was allowed to cool and evaporated under reduced pressure. Purification of the resulting residue by column chromatography (hexane:ethyl acetate = 1:1, followed by hexane:ethyl acetate = 1:2, followed by hexane: ethyl acetate = 1 : 4) gave 1.50 g of a pale yellow powder (69% yield).
MS (FAB) (m/z): 386 (MH⁺-HOCH₂OCH₃).
HRMS (FAB) (m/z) : Calcd. for C₂₃H₃₆N₃O₂ (MH⁺-HOCH₂OCH₃) : 386.2808. Found, 386.2849.

This product was dissolved in methanol (50 mL). To this solution, 1.27 g (6.70 mmol) p-toluenesulfonic acid was added and the resulting mixture was left for 24 hours at room temperature. Subsequently, the mixture was concentrated, followed by addition of a diluted aqueous sodium bicarbonate solution and extraction with ethyl acetate (30 mL x 3). The organic layers were combined, washed with saturated brine (20mL) and dried over anhydrous magnesium sulfate. The dried product was filtered and the solvent was removed. Purification of the resulting residue by column chromatography (hexane:ethyl acetate = 1: 2, ethyl acetate, followed by hexane:ethyl acetate = 10:1) afforded 1.03 g of the title compound as a colorless powder (76% yield).
MS (FAB) (m/z) : 404 (MH⁺) .
HRMS (FAB) (m/z): Calcd. for C₂₃H₃₈N₃O₃ (MH⁺) : 404.2913. Found, 404.2903.

### (Reference Example 9)

### Step I

(3R)-3-deoxo-11-deoxy-12-desethenyl-3-methoxy-14-O-methoxymeth yl-12-(2-methyl-1-propene-3-yl)-11-oxo-4-epimutilin

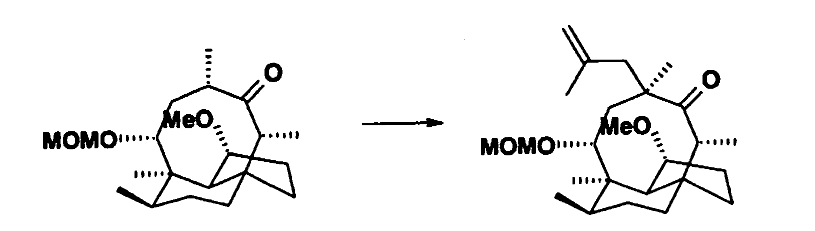

According to Step I of Example 2, 2.00 g (5.67 mmol) of the compound of Reference Example 3, 0.67mL (6.81 mmol) 3-chloro-2-methylpropene and 13.6 mL (6.81 mmol) potassium bis(trimethylsilyl)amide (0.5 mol/L toluene solution) were used in the reaction. Purification of the resulting residue by silica gel column chromatography (hexane:ethyl acetate = 8:1) afforded 1.97 g of the title compound as a colorless oil (85% yield). MS (FAB) (m/z) : 345 (MH⁺-HOCH₂OCH₃).
HRMS (FAB) (m/z) : Calcd. for C₂₃H₃₇O₂ (MH⁺-HOCH₂OCH₃) : 345.2794. Found, 345.2814.

### Step II

(3R)-3-deoxo-11-deoxy-12-desethenyl-3-methoxy-12-(2-methyl-1-p ropene-3-yl)-11-oxo-4-epimutilin

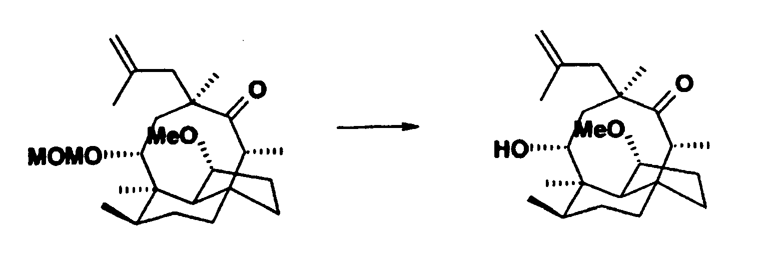

According to Reference Example 4, 1.97 g (4.85 mmol) of the
compound of Step I and 0.92 g (4.85 mmol) p-toluenesulfonic acid were used in the reaction. The resulting residue was purified by silica gel column chromatography (hexane:ethyl acetate = 4:1) to afford 1.41 g of the title compound as a colorless powder (80% yield). MS (FAB) (m/z): 345 (MH⁺-H₂O).
HRMS (FAB) (m/z): Calcd. for C₂₃H₃₇O₂(MH⁺-H₂O) : 345.2794. Found, 345.2814.

### (Reference Example 10)

### Step I

(3R)-12-(1-chloroethane-2-yl)-3-deoxo-11-deoxy-12-desethenyl-3 -methoxy-14-O-methoxymethyl-11-oxo-4-epimutilin

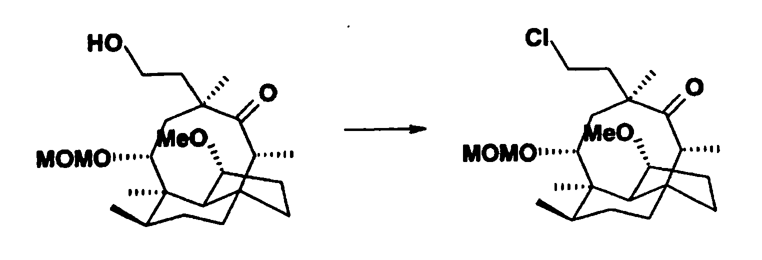

0.66 g (2.52 mmol) triphenylphosphine was added to a solution of 1.00 g (2.52 mmol) (3R)-3-deoxo-11-deoxy-12-desethenyl-12-(1-hydroxyethane-2-yl)-3-methoxy-14-O-methoxymethyl-11-oxo-4-epimutilin in carbon tetrachloride (30 mL). The reaction mixture was refluxed for 15 hours. After cooling, the mixture was filtered through Celite and the residue was washed with carbon tetrachloride. The combined filtrate was evaporated under reduced pressure and the resulting residue was purified by silica gel column chromatography (hexane:ethyl acetate = 10:1) to afford 474 mg of the title compound as a colorless oil (45% yield). MS (FAB) (m/z): 415 (MH⁺).
HRMS (FAB) (m/z): Calcd. for C₂₃H₄₀ClO₄(MH⁺): 415.2615. Found, 415.2616.

### Step II

(3R)-12-(1-chloroethane-2-yl)-3-deoxo-11-deoxy-12-desethenyl-3 -methoxy-11-oxo-4-epimutilin

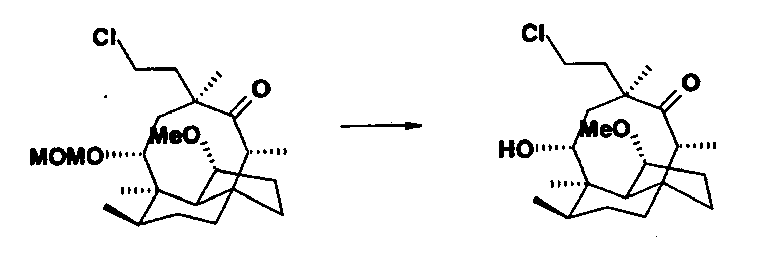

According to Reference Example 4, 474 mg (1.14 mmol) of the
compound of Step I and 217 mg (1.14 mmol) p-toluenesulfonic acid were used in the reaction. The resulting residue was purified by silica gel column chromatography (hexane:ethyl acetate = 4:1) to afford 279 mg of the title compound as a colorless oil (66% yield). MS (EI) (m/z): 370 (M⁺).
HRMS (EI) (m/z) : Calcd. for C₂₁H₃₅ClO₃(M⁺) : 370.2275. Found, 370.2303.

### (Reference Example 11)

(3R)-12-acetoxyethyl-3-deoxo-11-deoxy-12-desethenyl-3-methoxy-11-oxo-4-epimutilin

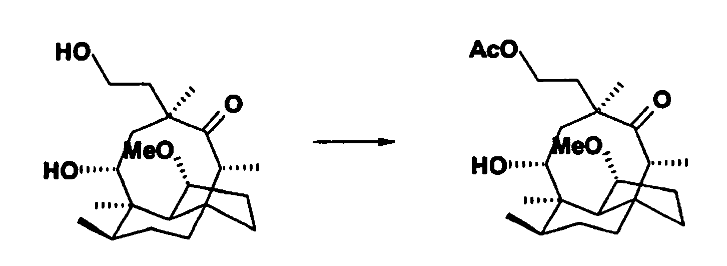

359 mg (1.02 mmol) of (3R)-3-deoxo-11-deoxy-12-desethenyl-12-(1-hydroxyethane-2-yl)-3-methoxy-11-oxo-4-epimutilin produced in Step II of Example 3, 1 mL acetic anhydride and 1 mL pyridine were used in the reaction. The resulting residue was purified by silica gel column chromatography (hexane:ethyl acetate = 2:1) to afford 210 mg of the title compound as a colorless powder (80% yield). MS (FAB) (m/z): 377 (MH⁺).
HRMS (FAB) (m/z) : Calcd. for C₂₃H₃₇O₄ (MH⁺) : 377.2692. Found, 377.2701.

### (Reference Example 12)

11-O-methoxymethylmutilin

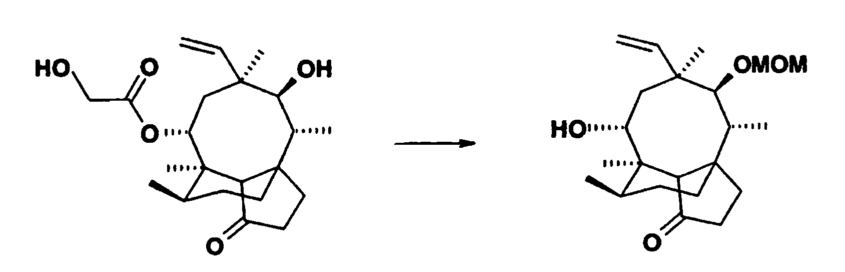

In an argon atmosphere at 0°C, 40.1 mL (0.53 mol) chloromethyl methyl ether was added dropwise to a methylene chloride solution (500 mL) of 50.0 g (0.13 mol) pleuromutilin and 138mL (0.79 mol) N,N-diisopropylethylamine in a 2L egg-shaped flask. The reaction mixture was left for 21 days at room temperature. Subsequently, 33.2 mL (0.26 mol) 3-(dimethylamino)propylamine was added and the mixture was evaporated under reduced pressure, followed by addition of diluted aqueous citric acid and extraction with ethyl acetate (200 mL x 3) . The organic layers were combined, washed with saturated brine (100 mL) and dried over anhydrous sodium sulfate. The dried product was filtered and the solvent was removed to give a crude compound. This product was dissolved in 500 ml of a 2 mol/L potassium hydroxide-methanol solution and the solution was refluxed for 3 hours. After cooling, the solvent was evaporated under reduced pressure, followed by addition of diluted aqueous citric acid and extraction with ethyl acetate (300 mL x 3). The organic layers were combined, washed with saturated brine (300 mL) and dried over anhydrous sodium sulfate. The dried product was filtered and the solvent was removed. Purification of the resulting residue by silica gel column chromatography (hexane:ethyl acetate = 4:1) afforded 42.0 g of the title compound as a colorless powder (87% yield in the two step process). MS (FAB) (m/z): 365 (MH⁺).
HRMS (FAB) (m/z) : Calcd. for C₂₂H₃₇O₄ (MH⁺) : 365.2692. Found, 365.2665.

### (Example 1)

### Step I

(3R)-14-{(3R,4S)-1-azabicyclo[2.2.1]heptane-3-carbonyl}carbamo yl-3-deoxo-11-deoxy-12-desethenyl-3-methoxy-11-oxo-4-epimutili n

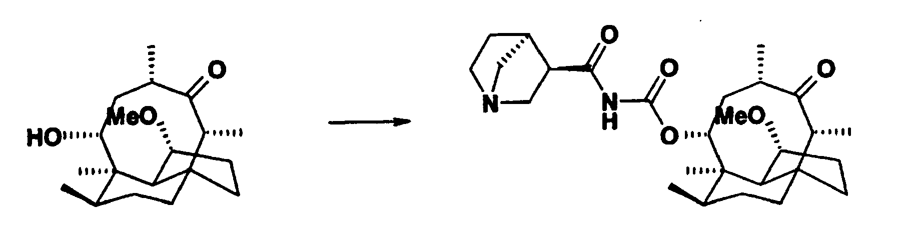

According to the process described in literature (J. Chem. Soc. Perkin Trans. I 1991, 1091-1097.), an acid chloride was prepared from 174 mg (0.98 mmol) (3R,4S)-1-azabicyclo[2.2.1]heptane-3-carboxlic acid hydrochloride. 244 mg (1. 63 mmol) silver cyanide, the acid chloride and 0.14 mL (0.98 mmol) triethylamine were added to a solution of 200 mg (0.65 mmol) of the compound of Reference Example 4 in methylene chloride (6 mL). The mixture was stirred for 22 hours at room temperature in a dark environment. Subsequently, the reaction mixture was filtered through Celite and the residue was washed with methylene chloride. The combined organic layer was evaporated under reduced pressure, followed by addition of a saturated aqueous sodium bicarbonate solution and extraction with ethyl acetate (10 mL x 3). The organic layers were combined, washed with saturated brine (10mL) and dried over anhydrous magnesium sulfate. The dried product was then filtered and the solvent was removed. Purification of the resulting residue by silica gel column chromatography (NH, ethyl acetate, and then ethyl acetate:methanol = 20:1) afforded 159 mg of the title compound as a colorless powder (52% yield).
MS (FAB) (m/z): 475 (MH⁺).
HRMS (FAB) (m/z): Calcd. for C₂₇H₄₃N₂O₅(MH⁺): 475.3172. Found, 475.3174.

### Step II

14-{(3R,4S)-1-azabicyclo[2.2.1]heptane-3-carbonyl}carbamoyl-12 -desethenylmutilin

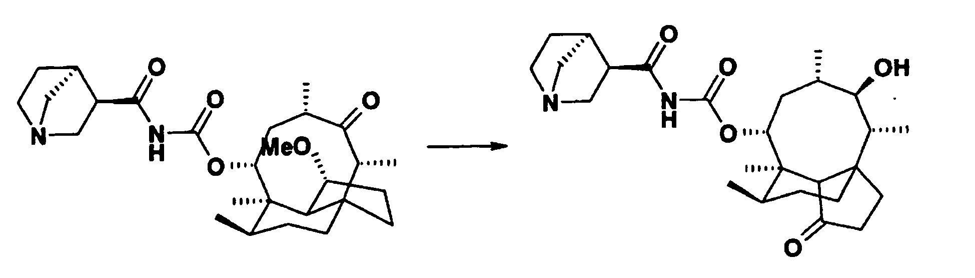

1. 00 mL concentrated hydrochloric acid was added to a solution of 100 mg (0.21 mmol) of the compound of Step I in ice-cooled dioxane (1 mL) while the solution was being stirred. The mixture was further stirred for about 13 hours as it was allowed to warm to room temperature. Subsequently, the reaction mixture was made basic by adding it to a 10% aqueous sodium hydroxide solution and the aqueous layer was extracted with ethyl acetate (10 mL x 3). The organic layers were combined, washed with saturated brine (10mL) and dried over anhydrous magnesium sulfate. The dried product was filtered and the solvent was removed. Purification of the resulting residue by silica gel column chromatography (NH, ethyl acetate:methanol = 30:1) afforded 67.9 mg of the title compound as a colorless powder (70% yield).
MS (FAB) (m/z): 461 (MH⁺) .
HRMS (FAB) (m/z): Calcd. for C₂₆H₄₁N₂O₅(MH⁺) : 461.3015. Found, 461.2988.

### (Example 2)

### Step I

(3R)-3-deoxo-11-deoxy-12-desethenyl-3-methoxy-14-O-methoxymeth yl-12-methyl-11-oxo-4-epimutilin

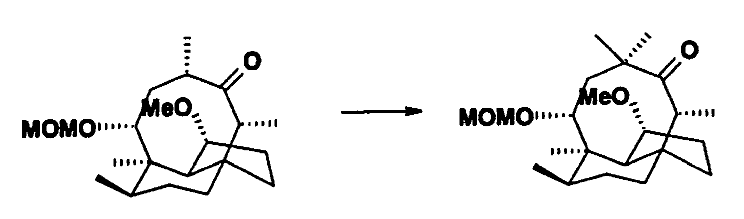

In an argon atmosphere at -70°C, 34.0 mL (17.0 mmol) potassium bis(trimethylsilyl)amide (0.5 mol/L toluene solution) was added to a solution of 5.00 g (14.2 mmol) of the compound of Reference Example 3 in anhydrous tetrahydrofuran (200 mL). The mixture was stirred for 0.5 hours. Under the same conditions, 1.06 mL (17.0 mmol) methyl iodide was added and the mixture was further stirred for 4 hours as it was allowed to warm to -50°C. Subsequently, diluted aqueous citric acid was added and the reaction mixture was evaporated under reduced pressure. The resulting residue was extracted with ethyl acetate (50 mL x 3). The organic layers were combined, washed with saturated brine (50 mL) and dried over anhydrous magnesium sulfate. The dried product was filtered and the solvent was removed. Purification of the resulting residue by silica gel column chromatography (hexane:ethyl acetate = 4:1) afforded 5.20 g of the title compound as a yellow oil (100% yield). MS (FAB) (m/z): 367 (MH⁺).
HRMS (FAB) (m/z) : Calcd. for C₂₂H₃₉O₄(MH⁺) : 367.2848. Found, 367.2884.

### Step II

(3R)-3-deoxo-11-deoxy-12-desethenyl-3-methoxy-12-methyl-11-oxo -4-epimutilin

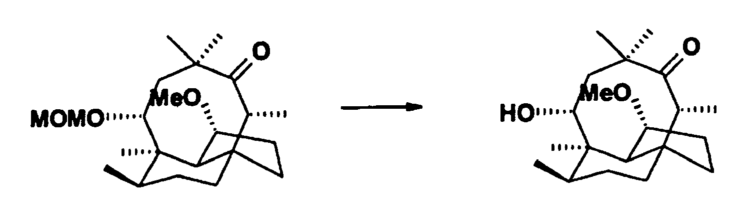

According to Reference Example 4, 5.20 g (14.2 mmol) of the
compound of Step I and 2.70 g (14.2 mmol) p-toluenesulfonic acid were used in the reaction. The resulting residue was purified by silica gel column chromatography (hexane:ethyl acetate = 4:1) to afford 3.70 g of the title compound as a colorless powder (81% yield) . MS (FAB) (m/z) : 323 (MH⁺).
HRMS (FAB) (m/z) : Calcd. for C₂₀H₃SO₃ (MH⁺) : 323.2586. Found, 323.2609.

### Step III

(3R)-14-{(3R,4S)-1-azabicyclo[2.2.1]heptane-3-carbonyl}carbamo yl-3-deoxo-11-deoxy-12-desethenyl-3-methoxy-12-methyl-11-oxo-4 -epimutilin

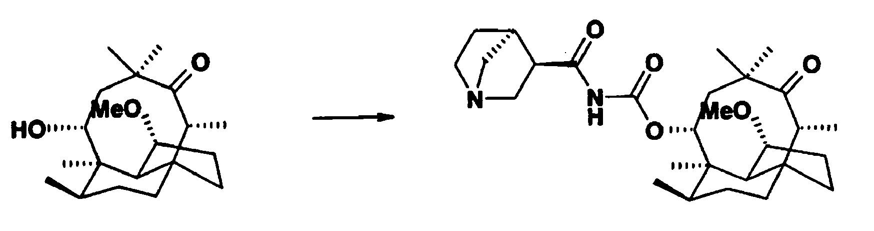

According to Step I of Example 1, the following compounds were used in the reaction: 500 mg (1.55 mmol) of the compound of Step II, an acid chloride prepared from 469 mg (2.64 mmol) of carboxylic acid, 580 mg (3.88 mmol) silver cyanide and 0.37 mL (2.64 mmol) triethylamine. The resulting residue was purified by silica gel column chromatography (NH, hexane: ethyl acetate = 1: 2, ethyl acetate, and then ethyl acetate:methanol = 10:1) to afford 590 mg of the title compound as a colorless powder (78% yield). MS (FAB) (m/z): 489 (MH⁺).
HRMS (FAB) (m/z): Calcd. for C₂₈H₄₅N₂O₅(MH⁺) : 489.3328. Found, 489.3314.

### Step IV

14-{(3R,4S)-1-azabicyclo[2.2.1]heptane-3-carbonyl}carbamoyl-12 -desethenyl-12-methylmutilin

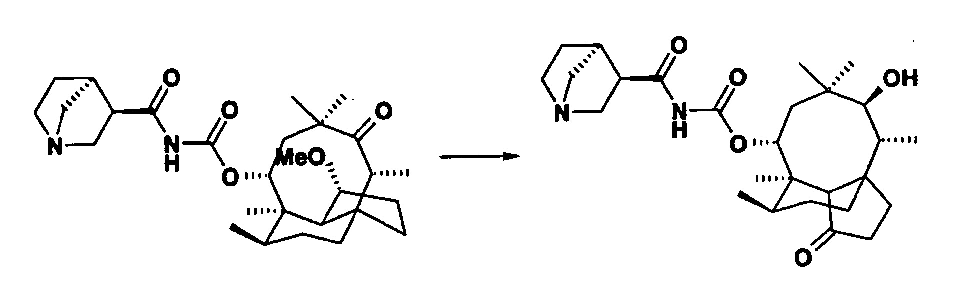

According to Step I of Example 1, 590 mg (1.21 mmol) of the compound of Step III was used in the reaction. The resulting residue was purified by silica gel column chromatography (NH, ethyl acetate:methanol = 20:1) to afford 460 mg of the title compound as a colorless powder (80% yield). MS (FAB) (m/z) : 475 (MH⁺) .
HRMS (FAB) (m/z): Calcd. for C₂₇H₄₃N₂O₅(MH⁺): 475.3172. Found, 475.3153.

### Step V

14-{(3R,4S)-1-azabicyclo[2.2.1]heptane-3-carbonyl}carbamoyl-12 -desethenyl-12-methylmutilin hydrochloride

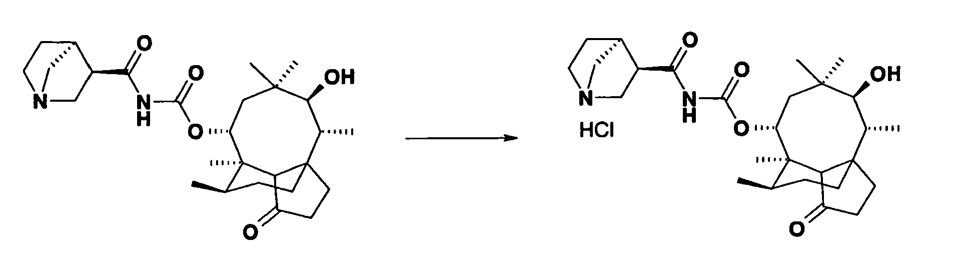

4 mol/L hydrogen chloride (2.00 mL) was added to a solution of 200 mg (0.42 mmol) of the compound of Step IV in ethyl acetate (2.00mL) and dioxane (2.00mL). The resulting crystals were filtered and washed with diethyl ether to afford 167 mg of the title compound (78% yield).
MS (FAB) (m/z): 475 (MH⁺ for free form).
HRMS (FAB) (m/z) : Calcd. for C₂₇H₄₃N₂O₅(MH⁺ for free form) : 475.3172. Found, 475.3171.

### (Example 3)

### Step I

(3R)-12-(1-acetoxyethane-2-yl)-3-deoxo-11-deoxy-12-desethenyl-3-methoxy-14-O-methoxymethyl-11-oxo-4-epimutilin

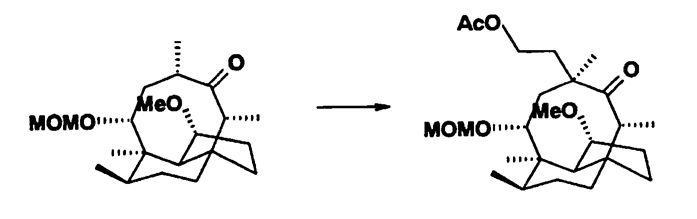

According to Step I of Example 2, 5.00 g (14.2 mmol) of the compound of Reference Example 3, 1.88 mL (17.0 mmol) ethyl bromoacetate and 34.0 mL (17.0 mmol) potassium bis(trimethylsilyl)amide (0.5 mol/L toluene solution) were used in the reaction. The resulting residue was purified by silica gel column chromatography (hexane:ethyl acetate = 5:1) to afford 5.47 g of the title compound as a pale yellow oil (88% yield). MS (FAB) (m/z) : 377 (MH⁺-HOCH₂OCH₃).
HRMS (FAB) (m/z) : Calcd. for C₂₃H₃₇O₄ (MH⁺-HOCH₂OCH₃) : 377.2692. Found, 377.2725.

### Step II

(3R)-12-(1-t-butyldimethylsilyloxyethane-2-yl)-3-deoxo-11-deox y-12-desethenyl-3-methoxy-11-oxo-4-epimutilin

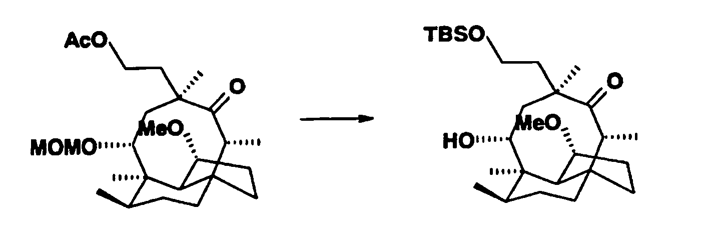

According to Reference Example 4, 5.18 g (11.8 mmol) of the compound of Step I and 2.25 g (14.2mmol) p-toluenesulfonic acid were used in the reaction. The resulting residue was purified by silica gel column chromatography (hexane:ethyl acetate = 1:2, followed by hexane:ethyl acetate = 1:4) to give 3.15 g of (3R)-3-deoxo-11-deoxy-12-desethenyl-12-(1-hydroxyethane-2-yl)-3-methoxy-11-oxo-4-epimutilin as a colorless powder (76% yield). 1.23 g (3. 49 mmol) of this product was dissolved in methylene chloride and was reacted with 0. 63 g (4.19 mmol) t-butyldimethylsilyl chloride and 1.22 mL (10.5 mmol) 2, 6-lutidine in an argon atmosphere at 0°C. The mixture was subsequently stirred for 31 hours as it was allowed to warm to room temperature. This was followed by addition of 439 µL (3.49 mmol) 3-(dimethylamino)propylamine and removal of the solvent under reduced pressure. Diluted aqueous citric acid was added to the residue and the solution was extracted with ethyl acetate (20 mL x 3). The organic layers were combined, washed with saturated brine (20 mL) and dried over anhydrous magnesium sulfate. The dried product was filtered and the solvent was removed. Purification of the resulting residue by silica gel column chromatography (hexane:ethyl acetate = 4:1) afforded 1.24 g of the title compound as a colorless oil (76% yield). MS (FAB) (m/z): 449 (MH⁺-H₂O).
HRMS (FAB) (m/z): Calcd. for C₂₇H₄₉O₃Si (MH⁺-H₂O) : 449.3451. Found, 449.3447.

### Step III

(3R)-14-{(3R,4S)-1-azabicyclo[2.2.1]heptane-3-carbonyl}carbamo yl-12-(1-t-butyldimethylsilyloxyethane-2-yl)-3-deoxo-11-deoxy-12-desethenyl-3-methoxy-11-oxo-4-epimutilin

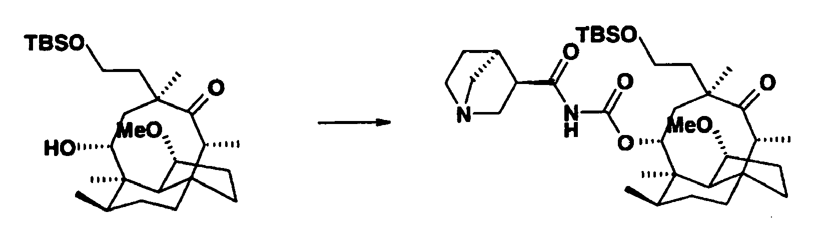

According to Step I of Example 1, the following compounds were used in the reaction: 500 mg (1.07 mmol) of the compound of Step II, an acid chloride prepared from 286 mg (1.61 mmol) carboxylic acid, 402 mg (2.68 mmol) silver cyanide and 0.22 mL (1.61 mmol) triethylamine. The resulting residue was purified by silica gel column chromatography (NH, ethyl acetate, and then ethyl acetate:methanol = 20:1) to afford 408 mg of the title compound as a colorless powder (60% yield). MS (FAB) (m/z): 633 (MH⁺) .
HRMS (FAB) (m/z) : Calcd. for C₃₅H₆₁N₂O₆Si (MH⁺) : 633.4299. Found, 633.4264.

### Step IV

14-{(3R,4S)-1-azabicyclo[2.2.1]heptane-3-carbonyl}carbamoyl-12 -desethenyl-12-(1-hydroxyethane-2-yl)mutilin

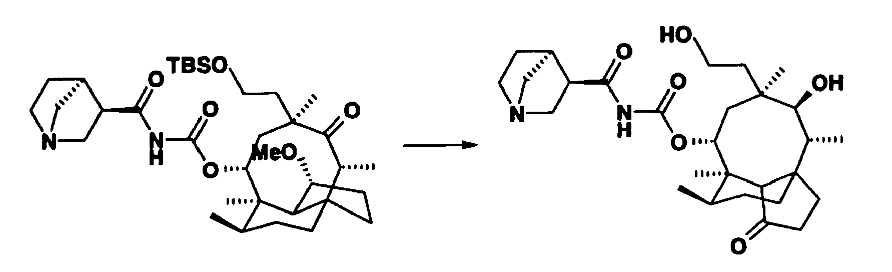

According to Step II of Example 1, 408 mg (0.64 mmol) of the compound of Step III was used in the reaction. The resulting residue was purified by silica gel column chromatography (NH, ethyl acetate:methanol = 20:1) to afford 210 mg of the title compound as a colorless powder (65% yield). MS (FAB) (m/z): 505 (MH⁺) .
HRMS (FAB) (m/z): Calcd. for C₂₈H₄₅N₂O₆ (MH⁺) : 505.3278. Found, 505.3284.

### (Example 4)

### Step I

(3R)-3-deoxo-11-deoxy-12-desethenyl-3-methoxy-14-O-methoxymeth yl-11-oxo-12-(1-propane-3-yl)-4-epimutilin

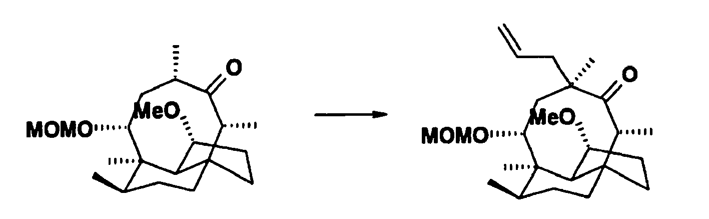

According to Step I of Example 2, 5.00 g (14.2 mmol) of the compound of Reference Example 3, 1.47 mL (17.0 mmol) allyl iodide and 34.0 mL (17.0 mmol) potassium bis(trimethylsilyl)amide (0.5 mol/L toluene solution) were used in the reaction. The resulting residue was purified by silica gel column chromatography (hexane: ethyl acetate = 10:1) to afford 2.52 g of the title compound as a yellow oil (45% yield). MS (FAB) (m/z): 393 (MH⁺).
HRMS (FAB) (m/z) : Calcd. for C₂₄H₄₁O₄(MH⁺) : 393.3005. Found, 393.2977.

### Step II

(3R)-3-deoxo-11-deoxy-12-desethenyl-3-methoxy-11-oxo-12-(1-pro pene-3-yl)-4-epimutilin

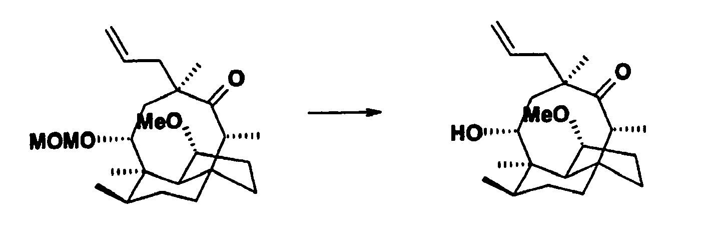

According to Reference Example 4, 2.52 g (6.42 mmol) of the
compound of Step I and 1.22 g (6.42 mmol) p-toluenesulfonic acid were used in the reaction. The resulting residue was purified by silica gel column chromatography (hexane:ethyl acetate = 4:1) to afford 1.76 g of the title compound as a colorless powder (79% yield) . MS (FAB) (m/z): 349 (MH⁺) .
HRMS (FAB) (m/z): Calcd. for C₂₂H₃₇O₃(MH⁺) : 349.2743. Found, 349.2788.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-(1-prope ne-3-yl)-4-epimutilin

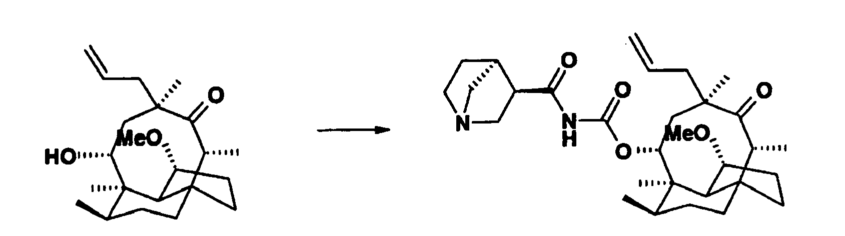

According to Step I of Example 1, the following compounds were used in the reaction: 500 mg (1.43 mmol) of the compound of Step II, an acid chloride prepared from 382 mg (2.15 mmol) carboxylic acid, 537 mg (3.58 mmol) silver cyanide and 0.30 mL (2.15 mmol) triethylamine. The resulting residue was purified by silica gel column chromatography (NH, ethyl acetate, and then ethyl acetate:methanol = 20:1) to afford 452 mg of the title compound as a colorless powder (61% yield). MS (FAB) (m/z): 515 (MH⁺) .
HRMS (FAB) (m/z): Calcd. for C₃₀H₄₇N₂O₅ (MH⁺) : 515.3485. Found, 515.3471.

### Step IV

14-{(3R,4S)-1-azabicyclo[2.2.1]heptane-3-carbonyl}carbamoyl-12 -desethenyl-12-(1-propene-3-yl)mutilin

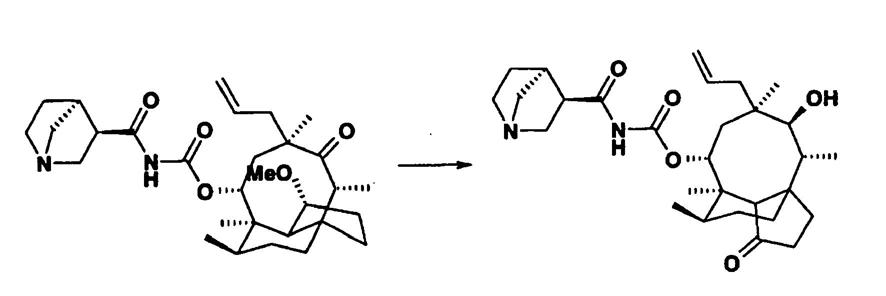

According to Step II of Example 1, 452 mg (0.88 mmol) of the compound of Step III was used in the reaction. The resulting residue was purified by silica gel column chromatography (NH, ethyl acetate:methanol = 20:1) to afford 348 mg of the title compound as a colorless powder (79% yield). MS (FAB) (m/z): 501 (MH⁺).
HRMS (FAB) (m/z): Calcd. for C₂₉H₄₅N₂O₅(MH⁺): 501.3328. Found, 501.3314.

### Step V

14-{(3R,4S)-1-azabicyclo[2.2.1]heptane-3-carbonyl}carbamoyl-12 -desethenyl-12-(1-propene-3-yl)mutilin hydrochloride

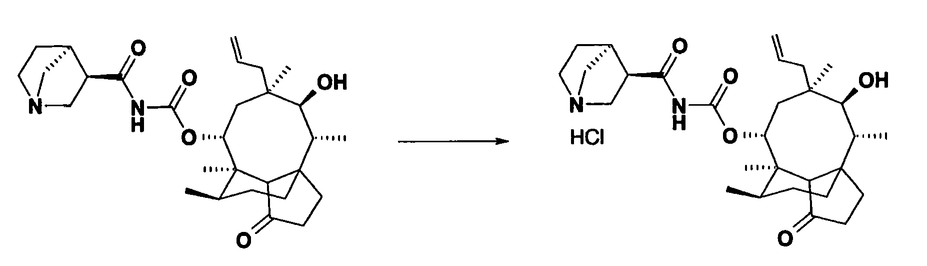

According to Step V of Example 2, 100 mg (0.20 mmol) of the
compound of Step IV was used in the reaction to afford 60.6 mg of the title compound (56% yield). MS (FAB) (m/z): 501 (MH⁺ for free form).
HRMS (FAB) (m/z) : Calcd. for C₂₉H₄₅N₂O₅(MH⁺ for free form) : 501.3328. Found, 501.3312.

### (Example 5)

### Step I

(3R)-3-deoxo-11-deoxy-12-desethenyl-3-methoxy-14-O-methoxymeth yl-11-oxo-12-phenylmethyl-4-epimutilin

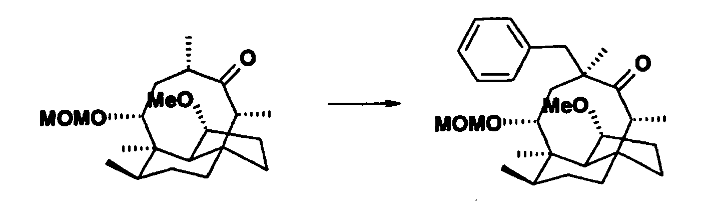

According to Step I of Example 2, 2.00 g (5.67 mmol) of the compound of Reference Example 3, 0.81 mL (6.81 mmol) benzyl bromide and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 4 : 1) to afford 2.51 g of the title compound as a colorless oil (100% yield).
MS (FAB) (m/z) : 381 (MH⁺-HOCH₂OCH₃).
HRMS (FAB) (m/z) : Calcd. for C₂₆H₃₇O₂ (MH⁺-HOCH₂OCH₃) : 381.2794. Found, 381.2817.

### Step II

(3R)-3-deoxo-11-deoxy-12-desethenyl-3-methoxy-11-oxo-12-phenyl methyl-4-epimutilin

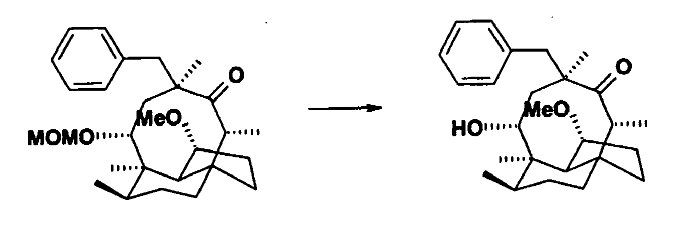

According to Reference Example 4, 2.51 g (5.67 mmol) of the
compound of Step I and 1.08 g (5.67 mmol) p-toluenesulfonic acid were used in the reaction. The resulting residue was purified by silica gel column chromatography (hexane:ethyl acetate = 4:1) to afford 1. 55 g of the title compound as a colorless powder (69% yield). MS (FAB) (m/z): 399 (MH⁺).
HRMS (FAB) (m/z) : Calcd. for C₂₆H₃₉O₃(MH⁺): 399.2899. Found, 399.2900.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-phenylme thyl-4-epimutilin

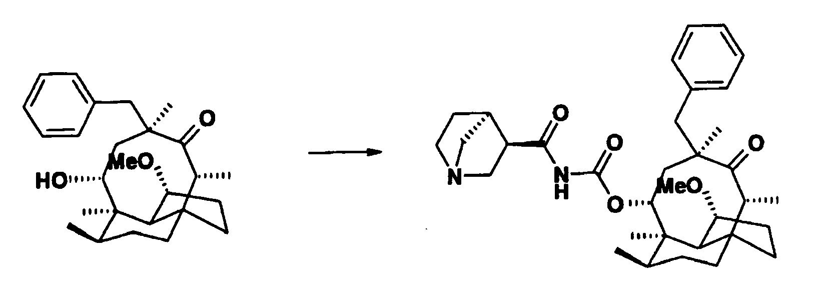

According to Step I of Example 1, the following compounds were used in the reaction: 500 mg (1.25 mmol) of the compound of Step II, an acid chloride prepared from 334 mg (1.88 mmol) carboxylic acid, 469 mg (3.13 mmol) silver cyanide and 0.26 mL (1.88 mmol) triethylamine. The resulting residue was purified by silica gel column chromatography (NH, ethyl acetate, and then ethyl acetate:methanol = 10:1) to afford 362 mg of the title compound as a colorless powder (51% yield). MS (FAB) (m/z): 565 (MH⁺) .
HRMS (FAB) (m/z): Calcd. for C₃₄H₄₉N₂O₅ (MH⁺) : 565.3641. Found, 565.3646.

### Step IV

14-{(3R,4S)-1-azabicyclo[2.2.1]heptane-3-carbonyl}carbamoyl-12 -desethenyl-12-phenylmethylmutilin

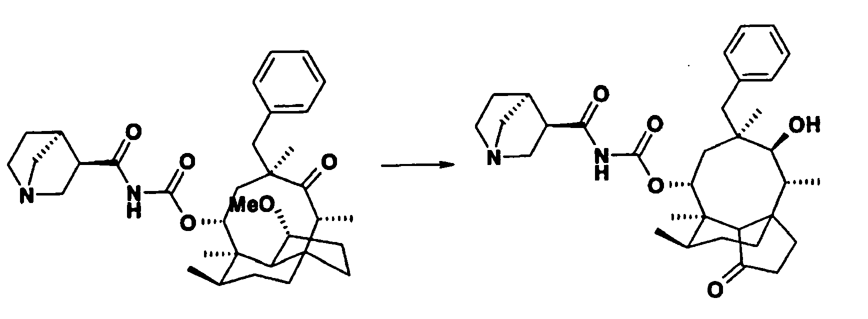

According to Step II of Example 1, 362 mg (0.64 mmol) of the
compound of Step III was used in the reaction. The resulting residue was purified by silica gel column chromatography (NH, ethyl acetate, and then ethyl acetate:methanol = 20:1) to afford 266 mg of the title compound as a colorless powder (75% yield). MS (FAB) (m/z): 551 (MH⁺) .
HRMS (FAB) (m/z): Calcd. for C₃₃H₄₇N₂O₅ (MH⁺) : 551.3485. Found, 551.3499.

### Step V

14-{(3R,4S)-1-azabicyclo[2.2.1]heptane-3-carbonyl}carbamoyl-12 -desethenyl-12-phenylmethylmutilin hydrochloride

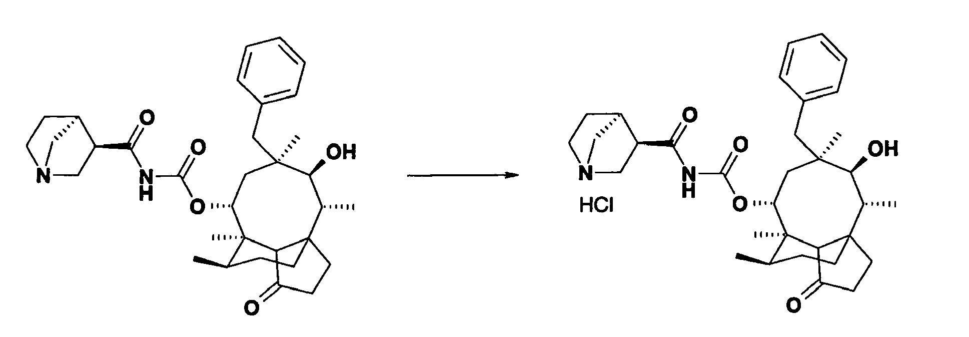

According to Step V of Example 2, 50.0 mg (90.8 mmol) of the
compound of Step IV was used in the reaction to afford 36.2 mg of the title compound (68% yield). MS (FAB) (m/z) : 551 (MH⁺ for free form).
HRMS (FAB) (m/z) : Calcd. for C₃₃H₄₇N₂O₅ (MH⁺ for free form) : 551.3485. Found, 551.3504.

### (Example 6)

### Step I

(3R)-3-deoxo-11-deoxy-12-desethenyl-3-methoxy-14-O-methoxymeth yl-11-oxo-12-(1-propyne-3-yl)-4-epimutilin

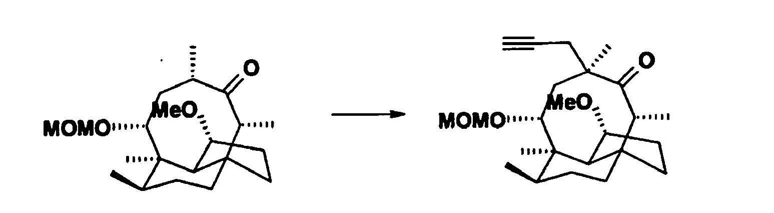

According to Step I of Example 2, 2.00 g (5.67 mmol) of the compound of Reference Example 3, 0.51 mL (6.81 mmol) propargyl bromide and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 4:1) to afford 2.32 g of the title compound as a yellow oil (100% yield). MS (FAB) (m/z) : 329 (MH⁺-HOCH₂OCH₃) .
HRMS (FAB) (m/z) : Calcd. for C₂₂H₃₃O₂ (MH⁺-HOCH₂OCH₃) : 329.2481. Found, 329.2467.

### Step II

(3R)-3-deoxo-11-deoxy-12-desethenyl-3-methoxy-11-oxo-12-(1-pro pyne-3-yl)-4-epimutilin

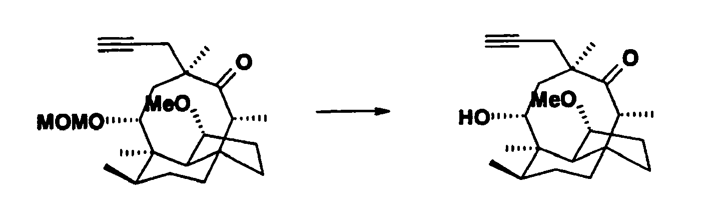

According to Reference Example 4, 2.32 g (5.67 mmol) of the compound of Step I and 1.08 g (5.67 mmol) p-toluenesulfonic acid were used in the reaction. The resulting residue was purified by silica gel column chromatography (hexane:ethyl acetate = 2:1) to afford 1.61g of the title compound as a colorless powder (82% yield). MS (FAB) (m/z): 347 (MH⁺).
HRMS (FAB) (m/z) : Calcd. for C₂₂H₃₅O₃ (MH⁺) : 347.2586. Found, 347.2600.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-(1-propy ne-3-yl)-4-epimutilin

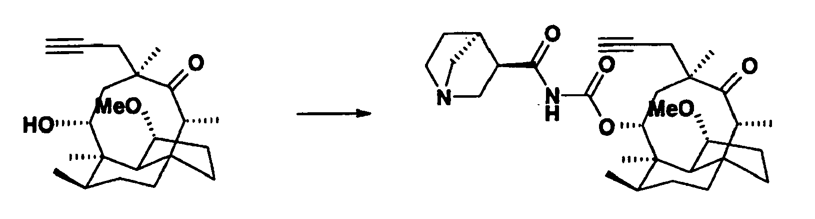

According to Step I of Example 1, the following compounds were used in the reaction: 500 mg (1.44 mmol) of the compound of Step II, an acid chloride prepared from 384 mg (2.16 mmol) carboxylic acid, 540 mg (3.60 mmol) silver cyanide and 0.30 mL (2.16 mmol) triethylamine. The resulting residue was purified by silica gel column chromatography (NH, ethyl acetate, and then hexane:ethyl acetate = 20: 1) to afford 368 mg of the title compound as a colorless powder (50% yield).
MS (FAB) (m/z): 513 (MH⁺).
HRMS (FAB) (m/z): Calcd. for C₃₀H₄₅N₂O₅(MH⁺) : 513.3328. Found, 513.3285.

### Step IV

14-{(3R,4S)-1-azabicyclo[2.2.1]heptane-3-carbonyl}carbamoyl-12 -desethenyl-12-(1-propyne-3-yl)mutilin

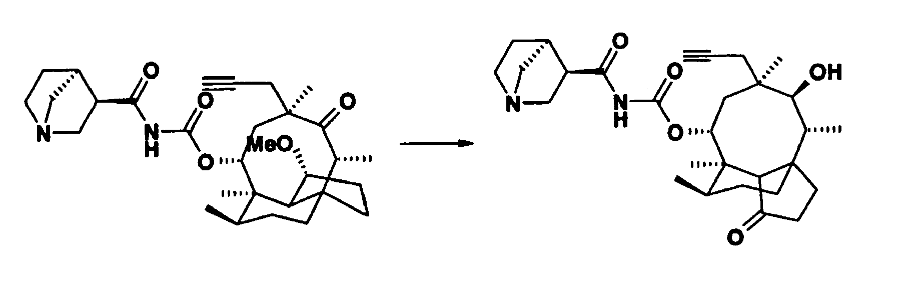

According to Step 2 of Example 1, 368 mg (0.72 mmol) of the compound of Step III was used in the reaction. The resulting residue was purified by silica gel column chromatography (NH, ethyl acetate:methanol = 20:1) to afford 306 mg of the title compound as a colorless powder (85% yield). MS (FAB) (m/z): 499 (MH⁺) .
HRMS (FAB) (m/z): Calcd. for C₂₉H₄₃N₂O₅(MH⁺): 499.3172. Found, 499.3192.

### Step V

14-{(3R,4S)-1-azabicyclo[2.2.1]heptane-3-carbonyl}carbamoyl-12 -desethenyl-12-(1-propyne-3-yl)mutilin hydrochloride

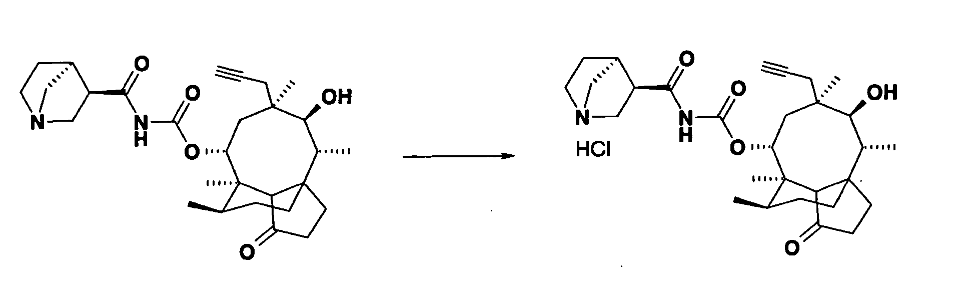

According to Step V of Example 2, 100 mg (0.20 mmol) of the
compound of Step IV was used in the reaction to afford 74.3 mg of the title compound (69% yield). MS (FAB) (m/z): 499 (MH⁺ for free form).
HRMS (FAB) (m/z) : Calcd. for C₂₉H₄₃N₂O₅ (MH⁺ for free form) : 499.3172. Found, 499.3188.

### (Example 7)

### Step I

(3R)-12-(2-propyne-3-yl)-3-deoxo-11-deoxy-12-desethenyl-3-meth oxy-14-O-methoxymethyl-11-oxo-4-epimutilin

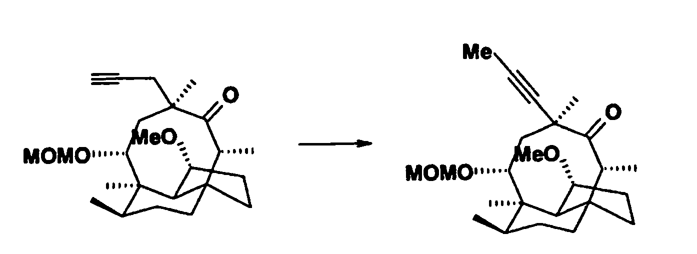

3. 82 g (34. 0 mmol) potassium t-butoxide was added to a solution of 13.3g (34.0 mmol) of the compound of Step I of Example 6 in ice-cooled tetrahydrofuran (150 mL). The reaction mixture was stirred for 12 hours as it was allowed to warm to room temperature. Subsequently, the mixture was poured into diluted aqueous citric acid and the solvent was removed under reduced pressure. The residue was then extracted with ethyl acetate (100 mL × 3). The organic layers were combined, washed with saturated brine (50 mL) and dried over anhydrous magnesium sulfate. The dried product was filtered and the solvent was removed. Purification of the resulting residue by silica gel column chromatography (hexane:ethyl acetate = 8:1) afforded 13.2 g of the title compound as a yellow oil (99% yield). MS (FAB) (m/z): 391 (MH⁺) .
HRMS (FAB) (m/z) : Calcd. for C₂₄H₃₉O₄ (MH⁺) : 391.2848. Found, 391.2871.

### Step II

(3R)-12-(2-propyne-3-yl)-3-deoxo-11-deoxy-12-desethenyl-3-meth oxy-11-oxo-4-epimutilin

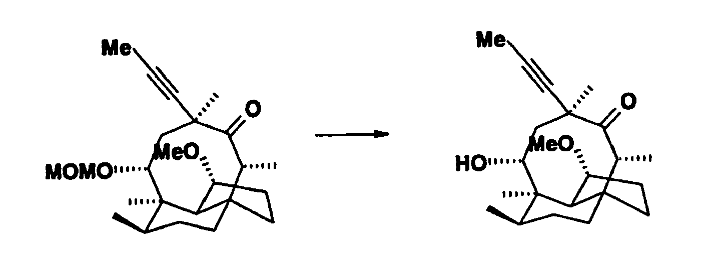

According to Reference Example 4, 3.09 g (7.91 mmol) of the compound of Step I and 1.50 g (7.91 mmol) p-toluenesulfonic acid were used in the reaction. The resulting residue was purified by silica gel column chromatography (hexane:ethyl acetate = 4:1) to afford 2.12 g of the title compound as a yellow oil (77% yield). MS (FAB) (m/z): 329 (MH⁺-H₂O) .
HRMS (FAB) (m/z): Calcd. for C₂₂H₃₃O₂ (MH⁺-H₂O) : 329.2481. Found, 329.2477.

### Step III

(3R)-12-(2-propyne-3-yl)-14-{(3R,4S)-1-azabicyclo[2.2.1]heptan e-3-carbonyl}carbamoyl-3-deoxo-11-deoxy-12-desethenyl-3-methox y-11-oxo-4-epimutilin

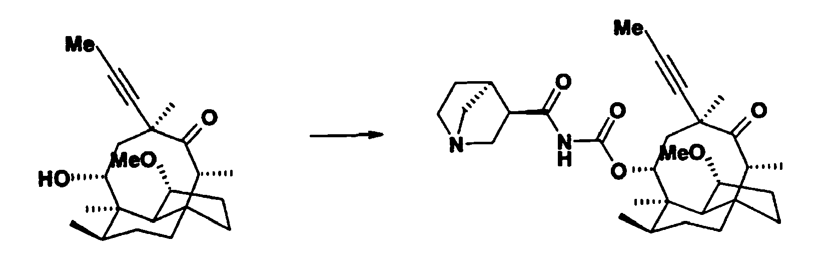

According to Step I of Example 1, the following compounds were used in the reaction: 200 mg (0.58 mmol) of the compound of Step II, an acid chloride prepared from 154 mg (0.87 mmol) carboxylic acid, 217 mg (1.45 mmol) silver cyanide and 0.12 mL (0.87 mmol) triethylamine. The resulting residue was purified by silica gel column chromatography (NH, ethyl acetate, and then ethyl acetate:methanol = 20:1) to afford 135 mg of the title compound as a colorless powder (45% yield). MS (FAB) (m/z) : 513 (MH⁺).
HRMS (FAB) (m/z): Calcd. for C₃₀H₄₅N₂O₅(MH⁺) : 513.3328. Found, 513.3358.

### Step IV

12-(2-propyne-3-yl)-14-{(3R,4S)-1-azabicyclo[2.2.1]heptane-3-c arbonyl}carbamoyl-12-desethenylmutilin

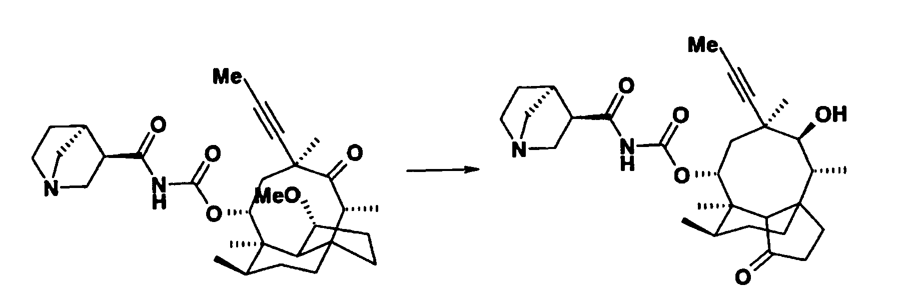

According to Step II of Example 1, 100 mg (0.20 mmol) of the
compound of Step III was used in the reaction. Purification of the resulting residue by silica gel column chromatography (NH, ethyl acetate:methanol = 30:1) afforded 80.5 mg of the title compound as a colorless powder (81% yield). MS (FAB) (m/z): 499 (MH⁺).
HRMS (FAB) (m/z): Calcd. for C₂₉H₄₃N₂O₅(MH⁺): 499.3172. Found, 499.3184.

### (Example 8)

### Step I

(3R)-12-(2-butyne-4-yl)-3-deoxo-11-deoxy-12-desethenyl-3-metho xy-14-O-methoxymethyl-11-oxo-4-epimutilin

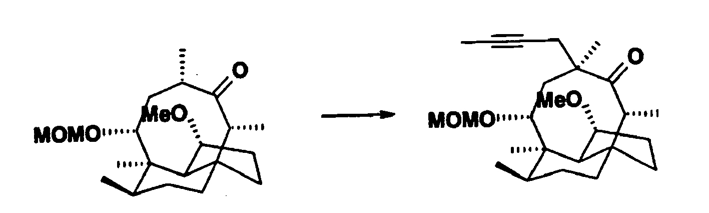

According to Step I of Example 2, 5.00 g (14.2 mmol) of the compound of Reference Example 3, 1. 4 9 mL (17.0 mmol) 1-bromo-2-butyne and 34.0 mL (17.0 mmol) potassium bis(trimethylsilyl)amide (0.5 mol/L toluene solution) were used in the reaction. The resulting residue was purified by silica gel column chromatography (hexane:ethyl acetate = 4:1) to afford 5.74 g of the title compound as a yellow oil (100% yield). MS (FAB) (m/z) : 343 (MH⁺-HOCH₂OCH₃).
HRMS (FAB) (m/z): Calcd. for C₂₃H₃₅O₂ (MH⁺-HOCH₂OCH₃) : 343.2637. Found, 343.2639.

### Step II

(3R)-12-(2-butyne-4-yl)-3-deoxo-11-deoxy-12-desethenyl-3-metho xy-11-oxo-4-epimutilin

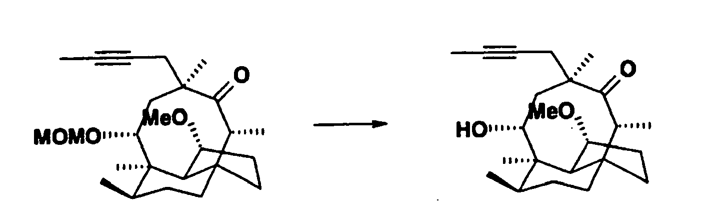

According to Reference Example 4, 5.74 g (14.2 mmol) of the
compound of Step I and 2.70 g (14.2 mmol) p-toluenesulfonic acid were used in the reaction. The resulting residue was purified by silica gel column chromatography (hexane:ethyl acetate = 4:1) to afford 4.05 g of the title compound as a colorless powder (79% yield) . MS (FAB) (m/z) : 343 (MH⁺-H₂O) .
HRMS (FAB) (m/z): Calcd. for C₂₃H₃₅O₂ (MH⁺-H₂O) : 343.2637. Found, 343.2617.

### Step III

(3R)-12-(2-butyne-4-yl)-14-{(3R,4S)-1-azabicyclo[2.2.1]heptane -3-carbonyl}carbamoyl-3-deoxo-11-deoxy-12-desethenyl-3-methoxy -11-oxo-4-epimutilin

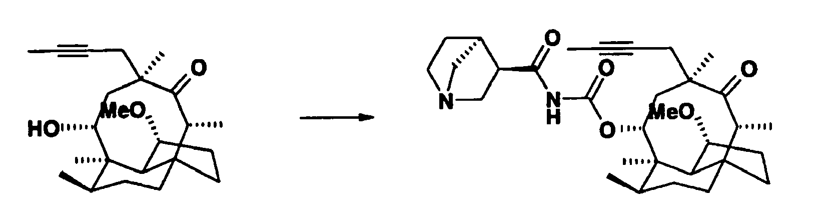

According to Step I of Example 1, the following compounds were used in the reaction: 500 mg (1.39 mmol) of the compound of Step II, an acid chloride prepared from 371 mg (2.09 mmol) carboxylic acid, 522 mg (3.48 mmol) silver cyanide and 0.29 mL (2.09 mmol) triethylamine. The resulting residue was purified by silica gel column chromatography (NH, ethyl acetate, and then ethyl acetate:methanol = 20:1) to afford 354 mg of the title compound as a colorless powder (48% yield). MS (FAB) (m/z): 527 (MH⁺).
HRMS (FAB) (m/z): Calcd. for C₃₁H₄₇N₂O₅(MH⁺): 527.3485. Found, 527.3498.

### Step IV

12-(2-butyne-4-yl)-14-{(3R,4S)-1-azabicyclo[2.2.1]heptane-3-ca rbonyl}carbamoyl-12-desethenylmutilin

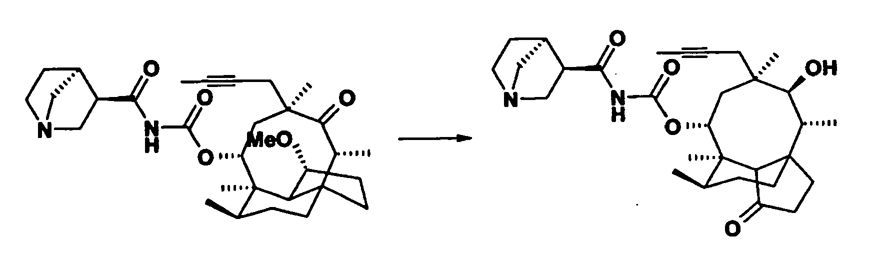

Using Step II of Example 1, 354 mg (0.67 mmol) of the compound of Step III was used in the reaction. The resulting residue was purified by silica gel column chromatography (NH, ethyl acetate, and then ethyl acetate:methanol = 20:1) to afford 284 mg of the title compound as a colorless powder (83% yield). MS (FAB) (m/z): 513 (MH⁺) .
HRMS (FAB) (m/z): Calcd. for C₃₀H₄₅N₂O₅(MH⁺) : 513.3328. Found, 513.3305.

### Step V

12-(2-butyne-4-yl)-14-{(3R,4S)-1-azabicyclo[2.2.1]heptane-3-ca rbonyl}carbamoyl-12-desethenylmutilin hydrochloride

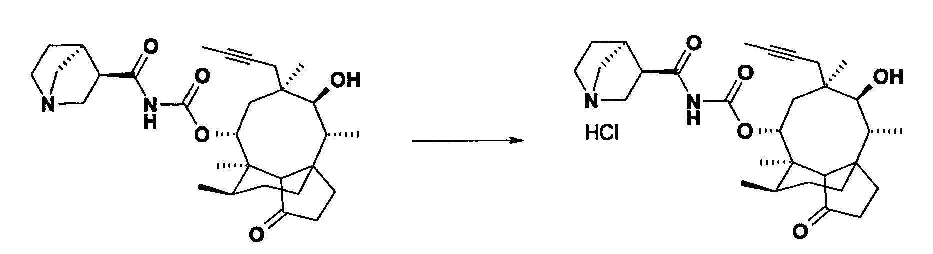

According to Step V of Example 2, 100 mg (0.20 mmol) of the
compound of Step IV was used in the reaction to afford 84.0 mg of the title compound (76% yield). MS (FAB) (m/z): 513 (MH⁺ for free form).
HRMS (FAB) (m/z) : Calcd. for C₃₀H₄₅N₂O₅ (MH⁺ for free form) : 513.3328. Found, 513.3350.

### (Example 9)

### Step I

(3R)-3-deoxo-11-deoxy-12-desethenyl-3-methoxy-14-0-methoxymeth yl-11-oxo-12-(3-pentyne-5-yl)-4-epimutilin

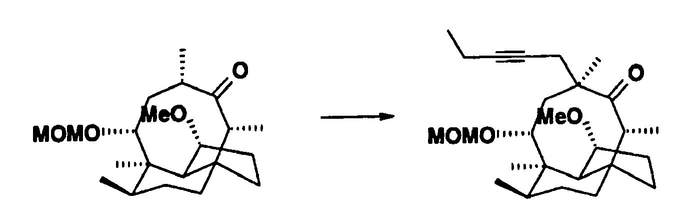

According to Step I of Example 2, 5.00 g (14.2 mmol) of the compound of Reference Example 3, 1.74 mL (17.0 mmol) 1-bromo-2-pentyne and 34.0 mL (17.0 mmol) potassium bis(trimethylsilyl)amide (0.5 mol/L toluene solution) were used in the reaction to afford 5. 94 g crude product of the title compound as a yellow oil (100% yield). MS (FAB) (m/z) : 357 (MH⁺-HOCH₂OCH₃).
HRMS (FAB) (m/z): Calcd. for C₂₄H₃₇O₂(MH⁺-HOCH₂OCH₃): 357.2794. Found, 357.2802.

### Step II

(3R)-3-deoxo-11-deoxy-12-desethenyl-3-methoxy-11-oxo-12-(3-pen tyne-5-yl)-4-epimutilin

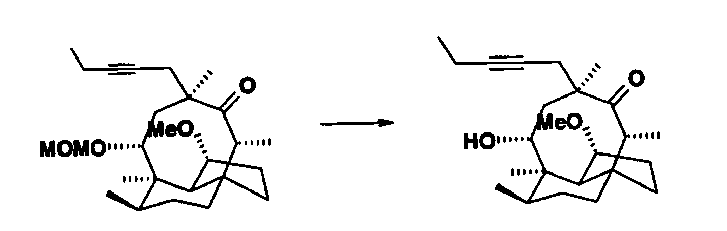

According to Reference Example 4, 5.94 g (14.2 mmol) of the compound of Step I and 2.70 g (14.2 mmol) p-toluenesulfonic acid were used in the reaction. The resulting residue was purified by silica gel column chromatography (hexane:ethyl acetate = 4:1) to afford 3.99 g of the title compound as a pale yellow powder (75% yield) .
MS (FAB) (m/z) : 357 (MH⁺-H₂O).
HRMS (FAB) (m/z): Calcd. for C₂₄H₃₇O₂ (MH⁺-H₂O) : 357.2794. Found, 357.2774.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-(3-penty ne-5-yl)-4-epimutilin

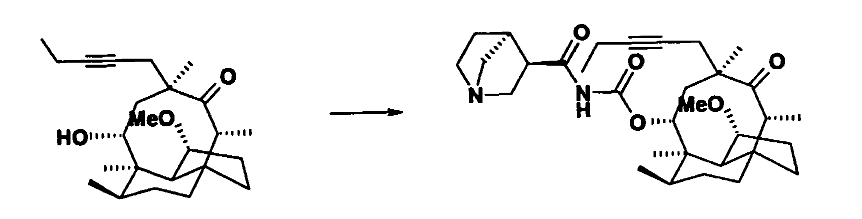

According to Step I of Example 1, the following compounds were used in the reaction: 500 mg (1.33 mmol) of the compound of Step II, an acid chloride prepared from 355 mg (2.00 mmol) carboxylic acid, 499 mg (3.33 mmol) silver cyanide and 0.28 mL (2.00 mmol) triethylamine. The resulting residue was purified by silica gel column chromatography (NH, ethyl acetate, and then ethyl acetate:methanol = 20:1) to afford 300 mg of the title compound as a colorless powder (42% yield). MS (FAB) (m/z): 541 (MH⁺) .
HRMS (FAB) (m/z): Calcd. for C₃₂H₄₉N₂O₅ (MH⁺): 541.3641. Found, 541.3653.

### Step IV

14-{(3R,4S)-1-azabicyclo[2.2.1]heptane-3-carbonyl}carbamoyl-12 -desethenyl-12-(3-pentyne-5-yl)mutilin

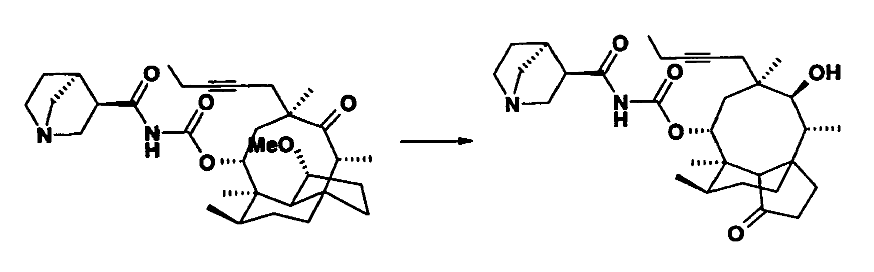

According to Step II of Example 1, 300 mg (0.55 mmol) of the compound of Step III was used in the reaction. The resulting residue was purified by silica gel column chromatography (NH, ethyl acetate, and then ethyl acetate:methanol = 20:1) to afford 105 mg of the title compound as a colorless powder (36% yield). MS (FAB) (m/z): 527 (MH⁺).
HRMS (FAB) (m/z): Calcd. for C₃₁H₄₇N₂O₅(MH⁺) : 527.3485. Found, 527.3498.

### (Example 10)

### Step I

(3R)-3-deoxo-11-deoxy-12-desethenyl-12-(1-fluoroethane-2-yl)-3 -methoxy-14-O-methoxymethyl-11-oxo-4-epimutilin

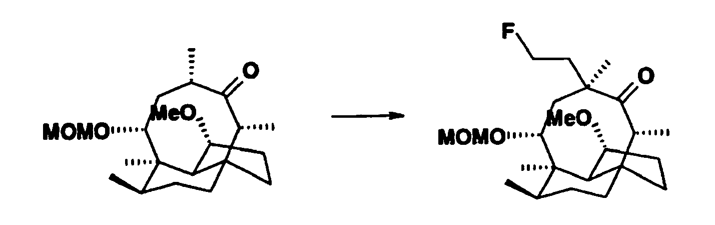

According to Step I of Example 2, 5.00 g (14.2 mmol) of the compound of Reference Example 3, 2.16 g (17.0 mmol) 1-bromo-2-fluoroethane and 34.0 mL (17.0 mmol) potassium bis(trimethylsilyl)amide (0.5 mol/L toluene solution) were used in the reaction. The resulting residue was purified by silica gel column chromatography (hexane:ethyl acetate = 4:1) to afford 5.29 g of the title compound as a pale yellow oil (94% yield).
MS (FAB) (m/z): 399 (MH⁺). HRMS (FAB) (m/z): Calcd. for C₂₃H₄₀FO₄ (MH⁺) : 399.2911. Found, 399.2916.

### Step II

(3R)-3-deoxo-11-deoxy-12-desethenyl-12-(1-fluoroethane-2-yl)-3 -methoxy-11-oxo-4-epimutilin

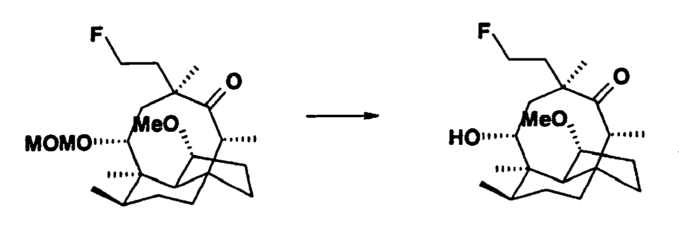

According to Reference Example 4, 5.29 g (13.3 mmol) of the compound of Step I and 2.52 g (13.3 mmol) p-toluenesulfonic acid were used in the reaction. The resulting residue was purified by silica gel column chromatography (hexane:ethyl acetate = 4:1, followed by hexane:ethyl acetate = 2:1) to afford 4.07 g of the title compound as a colorless powder (86% yield). MS (FAB) (m/z): 355 (MH⁺) .
HRMS (FAB) (m/z): Calcd. for C₂₁H₃₆FO₃ (MH⁺) : 355.2648. Found, 355.2672.

### Step III

(3R)-14-{(3R,4S)-1-azabicyclo[2.2.1]heptane-3-carbonyl}carbamo yl-3-deoxo-11-deoxy-12-desethenyl-12-(1-fluoroethane-2-yl)-3-m ethoxy-11-oxo-4-epimutilin

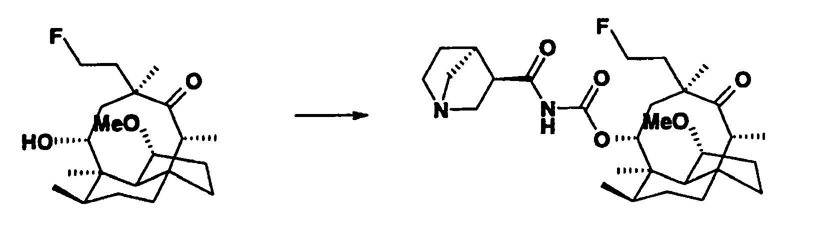

According to Step I of Example 1, the following compounds were used in the reaction: 500 mg (1.41 mmol) of the compound of Step II, an acid chloride prepared from 377 mg (2.12 mmol) carboxylic acid, 529 mg (3.53 mmol) silver cyanide and 0.30 mL (2.12 mmol) triethylamine. The resulting residue was purified by silica gel column chromatography (NH, ethyl acetate, and then ethyl acetate:methanol = 20:1) to afford 490 mg of the title compound as a colorless powder (67% yield). MS (FAB) (m/z): 521 (MH⁺).
HRMS (FAB) (m/z): Calcd. for C₂₉H₄₆FN₂O₅ (MH⁺) : 521. 3391. Found, 521.3430.

### Step IV

14-{(3R,4S)-1-azabicyclo[2.2.1]heptane-3-carbonyl}carbamoyl-12 -desethenyl-12-(1-fluoroethane-2-yl)mutilin

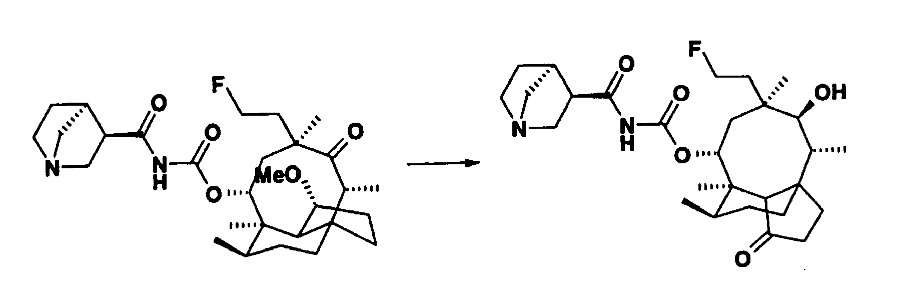

According to Step II of Example 1, 2,000 mg (3.84 mmol) of the compound of Step III was used in the reaction. The resulting residue was purified by silica gel column chromatography (NH, methylenechloride:methanol: 25% aqueous ammonia = 70 : :10:1) to afford 558 mg of the title compound as a colorless powder (29% yield). MS (FAB) (m/z): 507 (MH⁺).
HRMS (FAB) (m/z): Calcd. for C₂₈H₄₄FN₂O₅(MH⁺) : 507.3234. Found, 507.3231.

### (Example 11)

### Step I

(3R)-3-deoxo-11-deoxy-12-desethenyl-3-methoxy-14-O-methoxymeth yl-11-oxo-12-(1,1,1-trifluoroethane-2-yl)-4-epimutilin

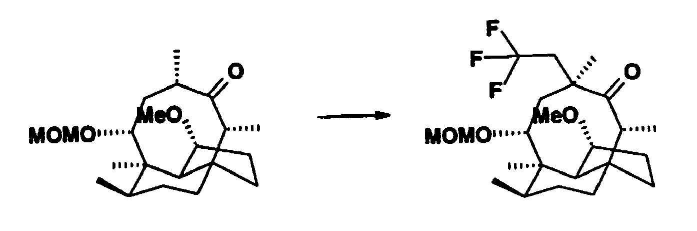

According to Step I of Example 2, 600 mg (1.70 mmol) of the compound of Reference Example 3, 201 µL (2.04 mmol) trifluoroethyl iodide and 4.08 mL (2.04 mmol) potassium bis (trimethylsilyl) amide (0.5 mol/L toluene solution) were used in the reaction. The resulting residue was purified by silica gel column chromatography (hexane:ethyl acetate = 10:1) to afford 563 mg of he title compound as a colorless oil (78% yield). MS (FAB) (m/z): 391 (MH⁺-MeOH).
HRMS (FAB) (m/z): Calcd. for C₂₁H₃₄F₃O₃(MH⁺-MeOH) : 391.2460. Found, 391.2459.

### Step II

(3R)-3-deoxo-11-deoxy-12-desethenyl-3-methoxy-11-oxo-12-(1,1,1 -trifluoroethane-2-yl)-4-epimutilin

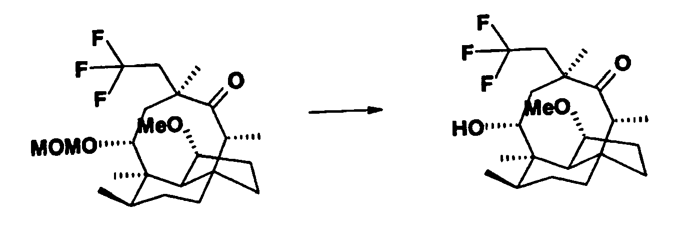

According to Reference Example 4, 563 mg (1.33 mmol) of the compound of Step I and 253 mg (1.33 mmol) p-toluenesulfonic acid were used in the reaction. The resulting residue was purified by silica gel column chromatography (hexane:ethyl acetate = 4:1) to afford 396 mg of the title compound as a yellow powder (79% yield) .
MS (CI) (m/z): 391 (MH⁺) .
HRMS (CI) (m/z) : Calcd. for C₂₁H₃₉F₃O₃ (MH⁺) : 391.2460. Found, 391.2479.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-(1,1,1-t rifluoroethane-2-yl)-4-epimutilin

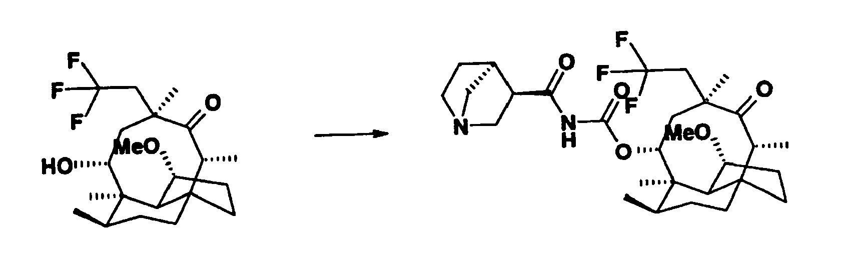

According to Step I of Example 1, the following compounds were used in the reaction: 250 mg (0.64 mmol) of the compound of Step II, an acid chloride prepared from 170 mg (0.96 mmol) carboxylic acid, 240 mg (1.60 mmol) silver cyanide and 130 µL (0.96 mmol) triethylamine. The resulting residue was purified by silica gel column chromatography (NH, ethyl acetate, and then ethyl acetate:methanol = 20:1) to afford 129 mg of the title compound as a colorless powder (36% yield).
MS (FAB) (m/z): 557 (MH⁺) .
HRMS (FAB) (m/z): Calcd. for C₂₉H₄₄F₃N₂O₅(MH⁺): 557.3202. Found, 557.3179.

### Step IV

14-{(3R,4S)-1-azabicyclo[2.2.1]heptane-3-carbonyl}carbamoyl-12 -desethenyl-12-(1,1,1-trifluoroethane-2-yl)mutilin

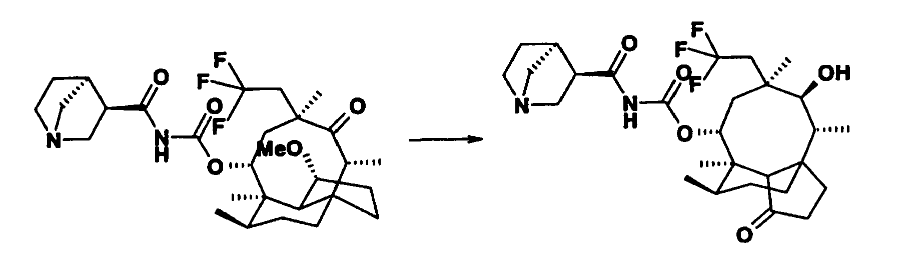

According to Step I I of Example 1, 129 mg (0.23 mmol) of the
compound of Step III was used in the reaction. The resulting residue was purified by silica gel column chromatography (NH, ethyl acetate, and then ethyl acetate:methanol = 20:1) to afford 26.2 mg of the title compound as a colorless powder (21% yield).
MS (FAB) (m/z): 543 (MH⁺).
HRMS (FAB) (m/z): Calcd. for C₂₈H₄₂F₃N₂O₅(MH⁺) : 543.3046. Found, 543.3048.

### (Example 12)

### Step I

(3R)-3-deoxo-11-deoxy-12-desethenyl-3-methoxy-14-O-methoxymeth yl-12-[(2-methyl)ethane-2-yl]-11-oxo-4-epimutilin

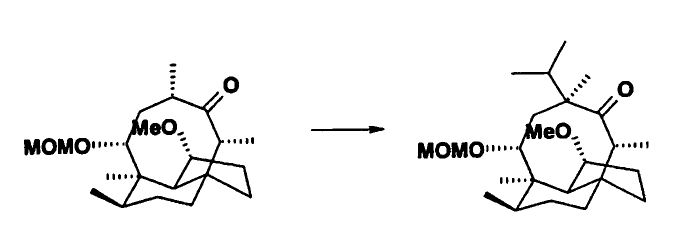

According to Step I of Example 2, 2.00 g (5.67 mmol) of the compound of Reference Example 3, 0.68 mL (6.81 mmol) 2-iodopropane and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 8:1) to afford 1.87 g of the title compound as a brown powder (84% yield).
MS (FAB) (m/z): 333 (MH⁺-HOCH₂OCH₃).
HRMS (FAB) (m/z) : Calcd. for C₂₂H₃₇O₂(MH⁺-HOCH₂OCH₃) : 333.2794. Found, 333.2802.

### Step II

(3R)-3-deoxo-11-deoxy-12-desethenyl-3-methoxy-12-[(2-methyl)et hane-2-yl]-11-oxo-4-epimutilin

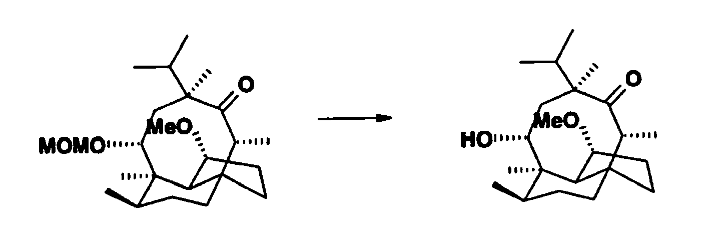

According to Reference Example 4, 1.87 g (4.74 mmol) of the
compound of Step I and 0.90 g (4.74 mmol) p-toluenesulfonic acid were used in the reaction. The resulting residue was purified by silica gel column chromatography (hexane:ethyl acetate = 4:1) to afford 1.38 g of the title compound as a colorless powder (83% yield). MS (FAB) (m/z) : 351 (MH⁺).
HRMS (FAB) (m/z) : Calcd. for C₂₂H₃₉O₃ (MH⁺) : 351.2899. Found, 351.2933.

### Step III

(3R)-14-{(3R,4S)-1-azabicyclo[2.2.1]heptane-3-carbonyl}carbamo yl-3-deoxo-11-deoxy-12-desethenyl-3-methoxy-12-[(2-methyl)etha ne-2-yl]-11-oxo-4-epimutilin

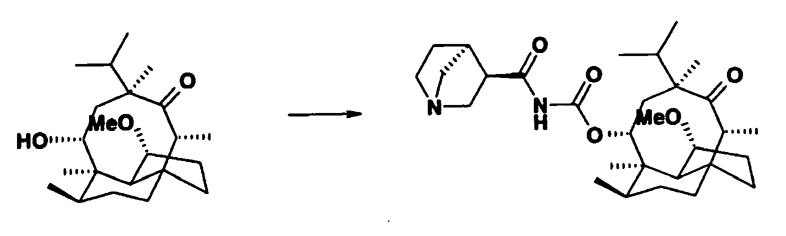

According to Step I of Example 1, the following compounds were used in the reaction: 300 mg (0.86 mmol) of the compound of Step II, an acid chloride prepared from 229 mg (1.29 mmol) of carboxylic acid, 322 mg (2.15 mmol) silver cyanide and 0.18 mL (1.29 mmol) triethylamine. The resulting residue was purified by silica gel column chromatography (NH, ethyl acetate, and then ethyl acetate:methanol = 20:1) to afford 248 mg of the title compound as a colorless powder (56% yield).
MS (FAB) (m/z): 517 (MH⁺) .
HRMS (FAB) (m/z): Calcd. for C₃₀H₄₉N₂O₅(MH⁺): 517.3641. Found, 517.3645.

### Step IV

14-{(3R,4S)-1-azabicyclo[2.2.1]heptane-3-carbonyl}carbamoyl-12 -desethenyl-12-[(2-methyl)ethane-2-yl]mutilin

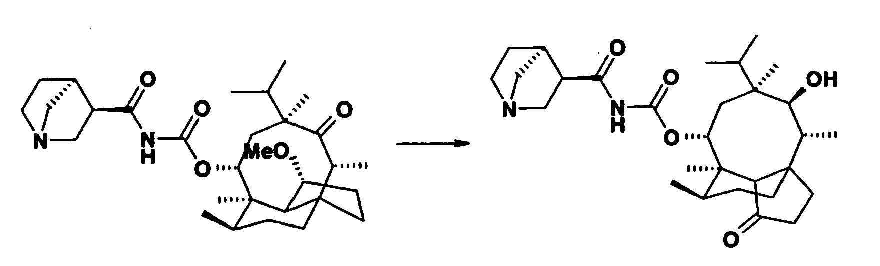

According to Step II of Example 1, 248 mg (0.48 mmol) of the
compound of Step III was used in the reaction. The resulting residue was purified by silica gel column chromatography (NH, ethyl acetate, and then ethyl acetate:methanol = 20:1) to afford 153 mg of the title compound as a colorless powder (63% yield).
MS (FAB) (m/z): 503 (MH⁺) .
HRMS (FAB) (m/z): Calcd. for C₂₉H₄₇N₂O₅ (MH⁺) : 503.3485. Found, 503.3467.

### (Example 13)

### Step I

(3R)-3-deoxo-11-deoxy-12-desethenyl-12-fluoromethyl-3-methoxy-14-0-methoxymethyl-11-oxo-4-epimutilin

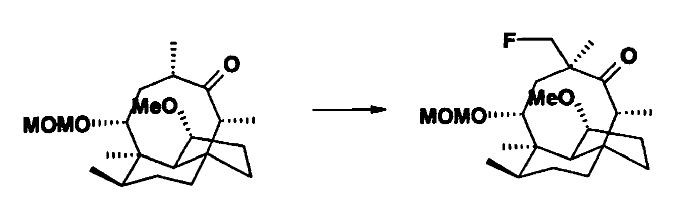

According to Step I of Example 2, 5.00 g (14.2 mmol) of the compound of Reference Example 3, 1.67 mL (17.0 mmol) methyl bromoacetate and 34.0 mL (17.0 mmol) potassium bis(trimethylsilyl)amide (0.5 mol/L toluene solution) were used in the reaction. The resulting residue was purified by silica gel column chromatography (hexane:ethyl acetate = 4:1) to give 3.93 g of (3R)-3-deoxo-11-deoxy-12-desethenyl-12-acetoxymethyl-3-methoxy -14-O-methoxymethyl-11-oxo-4-epimutilin as a colorless oil (65% yield).
MS (FAB) (m/z) : 363 (MH⁺-HOCH₂OCH₃).
HRMS (FAB) (m/z): Calcd. for C₂₂H₃₅O₄ (MH⁺-HOCH₂OCH₃) : 363.2535. Found, 363.2552.
To 2.00 g (4.71 mmol) of this compound, methanol and 1.30 g (9.42 mmol) of potassium carbonate were sequentially added and the mixture was stirred at room temperature for 2 hours. Subsequently, the reaction mixture was filtered through Celite. The residue was then washed with ethyl acetate and the solvent was evaporated under reduced pressure. Diluted aqueous citric acid was added to the residue and the mixture was extracted with ethyl acetate (10 mL x3) . The organic layers were combined and were washed with saturated brine (10 mL), followed by filtration and drying over anhydrous magnesium sulfate to remove the solvent. Purification of the resulting residue by silica gel column chromatography (hexane:ethyl acetate = 4:1, followed by hexane:ethyl acetate = 1:2) gave 1.13 g of (3R)-3-deoxo-11-deoxy-12-desethenyl-12-hydroxymethyl-3-methoxy -14-O-methoxymethyl-11-oxo-4-epimutilin as a colorless powder (63% yield).
MS (FAB) (m/z): 383 (MH⁺).
HRMS (FAB) (m/z) : Calcd. for C₂₂H₃₉O₅(MH⁺): 383.2797. Found, 383.2824.

1.13 g (2.95 mmol) of this compound was dissolved in toluene. The solution was chilled on ice and 1.33 mL (8.86 mmol) 1,8-diazabicyclo[5.4.0]unde-7-cene and 1.22 mL (4.43 mmol) perfluorooctanesulfonyl fluoride were sequentially added dropwise in an argon atmosphere. The mixture was then stirred for 15 hours as it was allowed to warm to room temperature. Subsequently, diluted aqueous citric acid was added and the mixture was extracted with ethyl acetate (10 mL x 3). The organic layers were combined and were washed with saturated brine (10 mL), followed by filtration and drying over anhydrous magnesium sulfate to remove the solvent. Purification of the resulting residue by silica gel column chromatography (hexane: ethyl acetate = 8:1) afforded 1.00 g of the title compound as a yellow powder (88% yield).
MS (FAB) (m/z) : 323 (MH⁺-H₂O).
Rf = 0.41 (hexane:ethyl acetate = 4:1).

### Step II

(3R)-3-deoxo-11-deoxy-12-desethenyl-12-fluoromethyl-3-methoxy-11-oxo-4-epimutilin

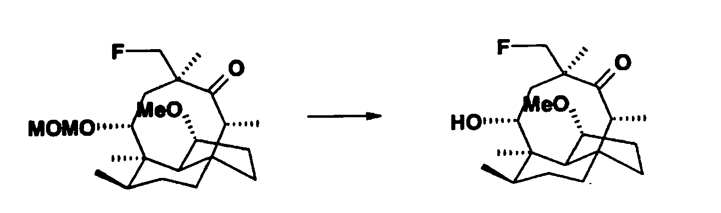

According to Reference Example 4, 1.00 g (2.60 mmol) of the compound of Step I and 0.49 g (2.60 mmol) p-toluenesulfonic acid were used in the reaction. The resulting residue was purified by silica gel column chromatography (hexane:ethyl acetate = 4:1, followed by hexane:ethyl acetate = 2:1) to afford 526 mg of the title compound as a colorless oil (59% yield).
MS (FAB) (m/z): 341 (MH⁺).
HRMS (FAB) (m/z): Calcd. for C₂₀H₃₄FO₃(MH⁺) : 341.2492. Found, 341.2502.

### Step III

(3R)-14-{(3R,4S)-1-azabicyclo[2.2.1]heptane-3-carbonyl}carbamo yl-3-deoxo-11-deoxy-12-desethenyl-12-fluoromethyl-3-methoxy-11 -oxo-4-epimutilin

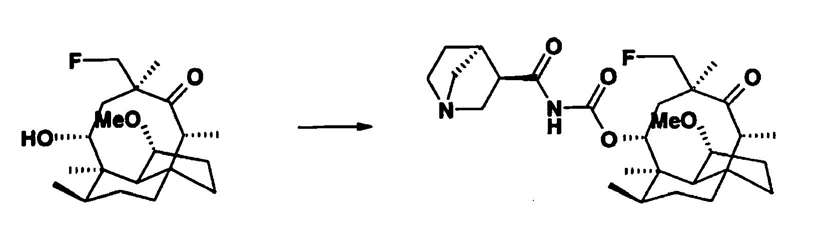

According to Step I of Example 1, the following compounds were
used in the reaction: 357 mg (1.05 mmol) of the compound of Step II, an acid chloride prepared from 281 mg (1.58 mmol) carboxylic acid, 394 mg (2.63 mmol) silver cyanide and 0.22 mL (1.58 mmol) triethylamine. The resulting residue was purified by silica gel column chromatography (NH, hexane:ethylacetate =2:1, ethyl acetate alone, and then ethyl acetate:methanol = 20:1) to afford 283 mg of the title compound as a colorless powder (53% yield).
MS (FAB) (m/z): 507 (MH⁺) .
HRMS (FAB) (m/z) : Calcd. for C₂₈H₄₄FN₂O₅(MH⁺): 507.3234. Found, 507.3255.

### Step IV

14-{(3R,4S)-1-azabicyclo[2.2.1]heptane-3-carbonyl}carbamoyl-12 -desethenyl-12-fluoromethylmutilin

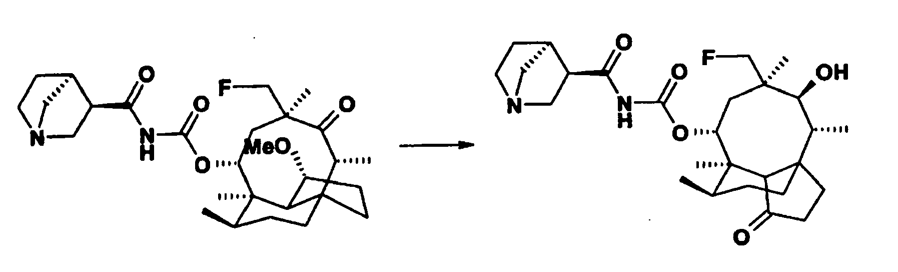

According to Step II of Example 1, 262 mg (0.52 mmol) of the
compound of Step III was used in the reaction. The resulting residue was purified by silica gel column chromatography (NH, ethyl acetate, and then ethyl acetate:methanol = 20:1) to afford 190 mg of the title compound as a colorless powder (74% yield).
MS (FAB) (m/z): 493 (MH⁺).
HRMS (FAB) (m/z) : Calcd. for C₂₇H₄₂FN₂O₅(MH⁺) : 493.3078. Found, 493.3079.

### Step V

14-{(3R,4S)-1-azabicyclo[2.2.1]heptane-3-carbonyl}carbamoyl-12 -desethenyl-12-fluoromethylmutilin hydrochloride

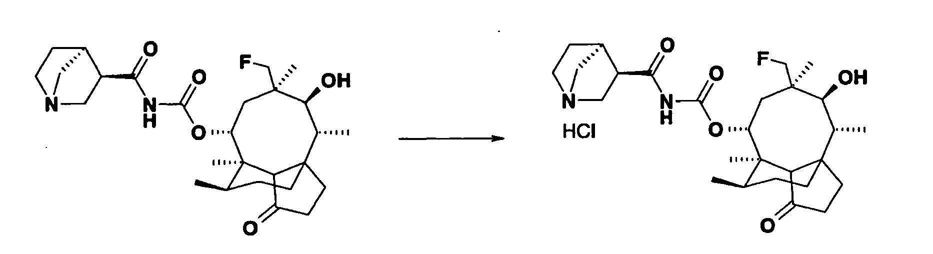

According to Step V of Example 2, 70.0 mg (0.14 mmol) of the
compound of Step IV was used in the reaction to afford 53.8 mg of the title compound (73% yield).
MS (FAB) (m/z): 493 (MH⁺ for free form).
HRMS (FAB) (m/z) : Calcd. for C₂₇H₄₂FN₂O₅ (MH⁺ for free form) : 493.3078. Found, 493.3071.

### (Example 14)

### Step I

(3R)-3-deoxo-11-deoxy-12-desethenyl-3-methoxy-14-O-methoxymeth yl-11-oxo-12-(4-pyridyl)methyl-4-epimutilin

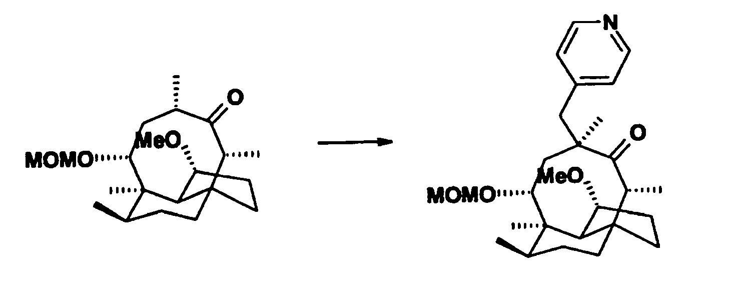

According to Step I of Example 2, 2.00 g (5.67 mmol) of the compound of Reference Example 3, 1.01 g (6.81 mmol) 4-chloromethylpyridine hydrochloride and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 4:1, followed by hexane:ethyl acetate =1:4, followed by ethyl acetate) to afford 2.06 g of the title compound as a brown powder (82% yield). MS (FAB) (m/z): 444 (MH⁺).
HRMS (FAB) (m/z): Calcd. for C₂₇H₄₂NO₄(MH⁺) : 444.3114. Found, 444.3122.

### Step II

(3R)-3-deoxo-11-deoxy-12-desethenyl-3-methoxy-11-oxo-12-(4-pyr idyl)methyl-4-epimutilin

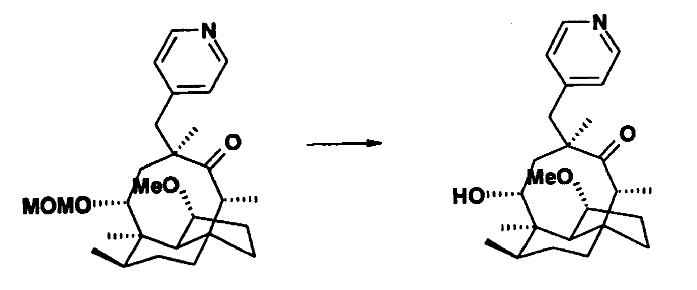

According to Reference Example 4, 2.06 g (4.64 mmol) of the compound of Step I and 1.77 g (9.29 mmol) p-toluenesulfonic acid were used in the reaction. The resulting residue was purified by silica gel column chromatography (ethyl acetate, and then ethyl acetate:methanol = 10:1) to afford 1.32 g of the title compound as a colorless powder (71% yield).
MS (FAB) (m/z): 400 (MH⁺).
HRMS (FAB) (m/z): Calcd. for C₂₅H₃₈NO₃(MH⁺) : 400.2852. Found, 400.2872.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-(4-pyrid yl)methyl-4-epimutilin

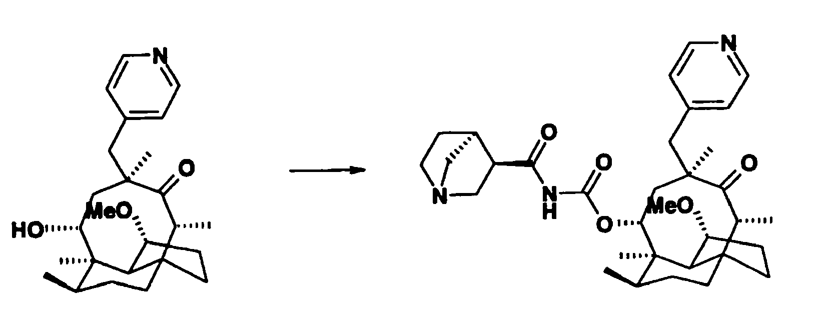

According to Step I of Example 1, the following compounds were used in the reaction: 300 mg (0.75 mmol) of the compound of Step II, an acid chloride prepared from 201 mg (1.13 mmol) carboxylic acid, 282 mg (1.88 mmol) silver cyanide and 0.16 mL (1.13 mmol) triethylamine. The resulting residue was purified by silica gel column chromatography (NH, ethyl acetate, and then ethyl acetate:methanol = 20:1) to afford 228 mg of the title compound as a colorless powder (54% yield). MS (FAB) (m/z): 566 (MH⁺) .
HRMS (FAB) (m/z) : Calcd. for C₃₃H₄₈N₃O₅ (MH⁺) : 566.3594. Found, 566.3592.

### Step IV

14-{(3R,4S)-1-azabicyclo[2.2.1]heptane-3-carbonyl}carbamoyl-12 -desethenyl-12-(4-pyridyl)methylmutilin hydrochloride

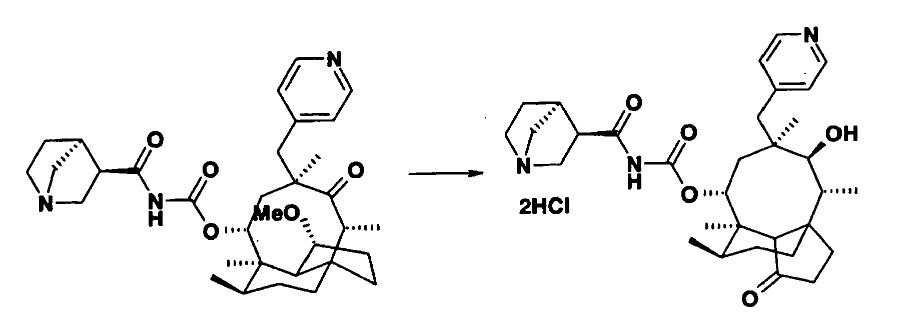

According to Step II of Example 1, 228 mg (0.40 mmol) of the compound of Step III were used in the reaction. The resulting residue was purified by silica gel column chromatography (NH, ethyl acetate, and then ethyl acetate:methanol = 20:1) to give 171 mg of 14-{(3R,4S)-1-azabicyclo[2.2.1]heptane-3-carbonyl}carbamoyl-12 -desethenyl-12-(4-pyridyl)methylmutilin as a colorless powder (77% yield). 171 mg of this product was treated with 4 mol/L hydrogen chloride-dioxane to afford 147 mg of the title compound as a colorless powder (76% yield).
MS (FAB) (m/z): 552 (MH⁺ for free form).
HRMS (FAB) (m/z) : Calcd. for C₃₂H₄₆N₃O₅(MH⁺ for free form) : 552.3437. Found, 552.3405.

### (Example 15)

### Step I

(3R)-3-deoxo-11-deoxy-12-desethenyl-3-methoxy-14-O-methoxymeth yl-11-oxo-12-[2-propene-3-yl]-4-epimutilin

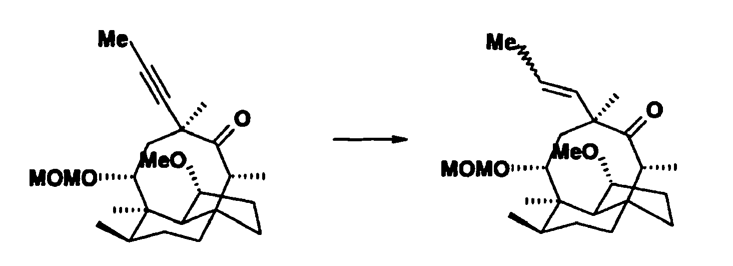

1.30 g Lindlar catalyst (10% by weight) was added to 13.2 g (33.8 mmol) of the compound of Step I of Example 7 in toluene (250 mL). The mixture was subjected to catalytic reduction at room temperature under 98.1 KPa for 5 hours. Subsequently, the reaction mixture was filtered through Celite and the residue was washed with ethyl acetate. The filtrates were combined and evaporated under reduced pressure to afford 13. 3 g of the title compound as a colorless oil (100% yield).
MS (FAB) (m/z): 393 (MH⁺).
HRMS (FAB) (m/z) : Calcd. for C₂₄H₄₁O₄ (MH⁺) : 393.3005. Found, 393.3010.

### Step II

(3R)-3-deoxo-11-deoxy-12-desethenyl-3-methoxy-11-oxo-12-[2-pro pene-3-yl]-4-epimutilin

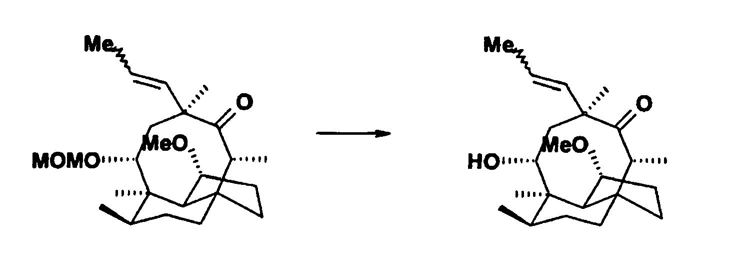

According to Reference Example 4, 13.3 g (33.9 mmol) of the
compound of Step I and 6.44 g (33.9 mmol) p-toluenesulfonic acid were used in the reaction. The resulting residue was purified by silica gel column chromatography (hexane:ethyl acetate = 4:1) to afford 10.1 g of the title compound as a colorless powder (86% yield). MS (FAB) (m/z): 331 (MH⁺-H₂O).
HRMS (FAB) (m/z): Calcd. for C₂₂H₃₅O₂(MH⁺-H₂O) : 331.2637. Found, 331.2645.

### Step III

14-{(3R,4S)-1-azabicyclo[2.2.1]heptane-3-carbonyl}carbamoyl-12 -desethenyl-3-methoxy-11-0-methoxymethyl-11-oxo-12-[2-propene-3-yl]-4-epimutilin and 14-{(3S,4S)-1-azabicyclo[2.2.1]heptane-3-carbonyl}carbamoyl-12 -desethenyl-3-methoxy-11-O-methoxymethyl-11-oxo-12-[2-propene-3-yl]-4-epimutilin

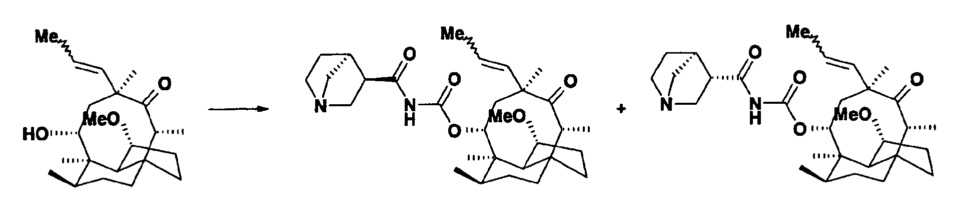

According to Step I of Example 1, the following compounds were used in the reaction: 30.0 g (86.1 mmol) of the compound of Step II, an acid chloride prepared from 22.9 g (129 mmol) carboxylic acid, 32.3 g (215 mmol) silver cyanide and 18.0 mL (129 mmol) triethylamine. The resulting residue was purified by silica gel column chromatography (NH, hexane: ethyl acetate= 1:5, and then ethyl acetate:methanol = 5:1) to afford 18.1 g of the title compound as a colorless powder (41% yield), along with 593 mg of its epi-form 14-{(3S,4S)-1-azabicyclo[2.2.1]heptane-3-carbonyl}carbamoyl-12 -desethenyl-3-methoxy-11-O-methoxymethyl-11-oxo-12-[2-propene-3-yl]-4-epimutilin (1% yield).
MS (FAB) (m/z) : 515 (MH⁺).
HRMS (FAB) (m/z): Calcd. for C₃₀H₄₇N₂O₅(MH⁺): 515.3485. Found, 515.3505.

14-{(3S,4S)-1-azabicyclo[2.2.1]heptane-3-carbonyl}carbamoyl-12 -desethenyl-3-methoxy-11-O-methoxymethyl-11-oxo-12-[2-propene-3-yl]-4-epimutilin
MS (FAB) (m/z): 515 (MH⁺) .
HRMS (FAB) (m/z): Calcd. for C₃₀H₄₇N₂O₅(MH⁺): 515.3485. Found, 515.3487.

### Step IV

14-{(3R,4S)-1-azabicyclo[2.2.1]heptane-3-carbonyl}carbamoyl-12 -desethenyl-12-[2-propene-3-yl]mutilin

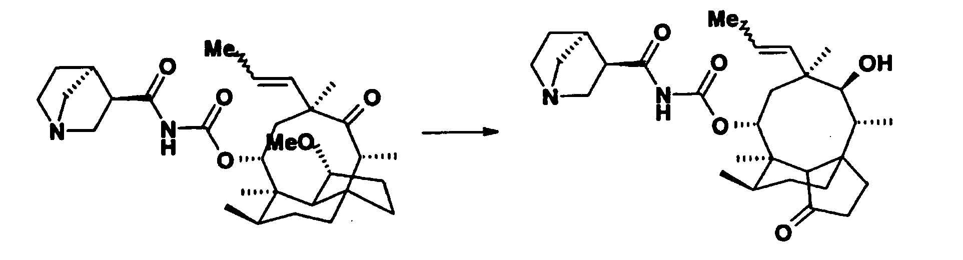

According to Step II of Example 1, 724 mg (1.41 mmol) of the
compound of Step III was used in the reaction. The resulting residue was purified by silica gel column chromatography (NH, ethyl acetate, and then ethyl acetate:methanol = 10:1) to afford 575 mg of the title compound as a colorless powder (82% yield).
MS (FAB) (m/z): 501 (MH⁺).
HRMS (FAB) (m/z): Calcd. for C₂₉H₄₅N₂O₅(MH⁺) : 501.3328. Found, 501.3314.

### Step V

14-{(3R,4S)-1-azabicyclo[2.2.1]heptane-3-carbonyl}carbamoyl-12 -desethenyl-12-[2-propene-3-yl]mutilin hydrochloride

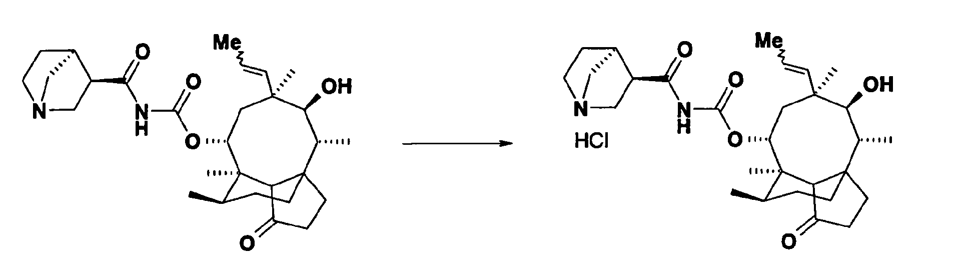

According to Step V of Example 2, 200 mg (0.40 mmol) of the compound of Step IV was used in the reaction to afford 187 mg of the title compound (87% yield).
MS (FAB) (m/z): 501 (MH⁺ for free form).
HRMS (FAB) (m/z) : Calcd. for C₂₉H₄₅N₂O₅(MH⁺ for free form) : 501.3328. Found, 501.3324.

### (Example 16)

### Step I

14-{(3R,4S)-1-azabicyclo[2.2.1]heptane-3-carbonyl}carbamoyl-3-deoxo-11-deoxy-12-desethenyl-3-methoxy-N-methyl-11-oxy-12-[2-p ropene-3-yl]-4 epimutilin

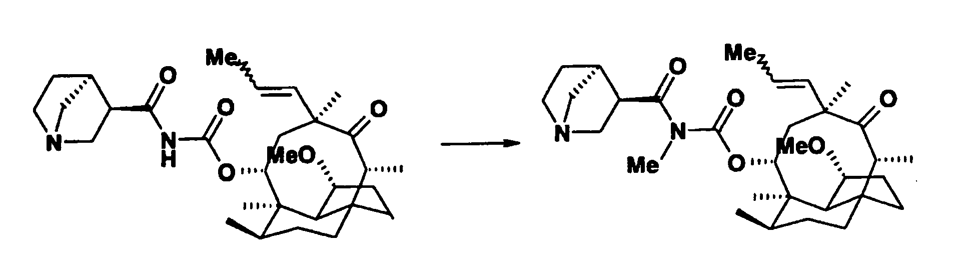

According to Step II of Example 14, 100 mg (0.19 mmol) of the
compound of Step III of Example 18, 11. 6 mg (0.29 mmol) sodium hydride (60% oil) and 18.1 µL (0.29 mmol) methyl iodide were used in the reaction. The resulting residue was purified by silica gel column chromatography (NH, ethyl acetate) to afford 38.4 mg of the title compound as a pale yellow oil (37% yield).
MS (FAB) (m/z): 529 (MH⁺) .
HRMS (FAB) (m/z): Calcd. for C₃₁H₄₉N₂O₅ (MH⁺) : 529.3641. Found, 529.3624.

### Step II

14-{(3R,4S)-1-azabicyclo[2.2.1]heptane-3-carbonyl}carbamoyl-12 -desethenyl-N-methyl-12-[2-propene-3-yl]mutilin

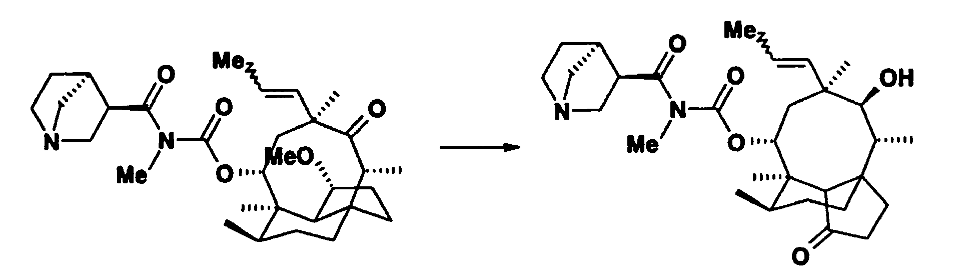

According to Step II of Example 1, 31. 2 mg (59.0 µmol) of the
compound of Step I was used in the reaction. The resulting residue was purified by silica gel column chromatography (NH, ethyl acetate) to afford 12.2 mg of the title compound as a yellow oil (39% yield). MS (FAB) (m/z): 510 (MH⁺-5).
Rf = 0.16 (ethyl acetate:methanol = 1:1)

### (Example 17)

### Step I

(3R)-3-deoxo-11-deoxy-12-desethenyl-3-methoxy-14-O-methoxymeth yl-11-oxo-12-propyl-4-epimutilin

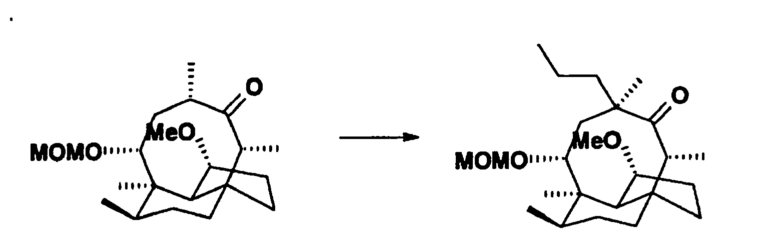

According to Step I of Example 2, 2.00 g (5.67 mmol) of the compound of Reference Example 3, 0.66 mL (6.81 mmol) 1-iodopropane and 13.6 mL (6.81 mmol) potassium bis(trimethylsilyl)amide (0.5 mol/L toluene solution) were used in the reaction. The resulting residue was purified by silica gel column chromatography (hexane:ethyl acetate = 4:1) to afford 2.19 g of the title compound as a brown oil (98% yield). MS (FAB) (m/z): 395 (MH⁺).
HRMS (FAB) (m/z) : Calcd. for C₂₄H₄₃O₄(MH⁺) : 395. 3161. Found, 395.3164.

### Step II

(3R)-3-deoxo-11-deoxy-12-desethenyl-3-methoxy-11-oxo-12-propyl -4-epimutilin

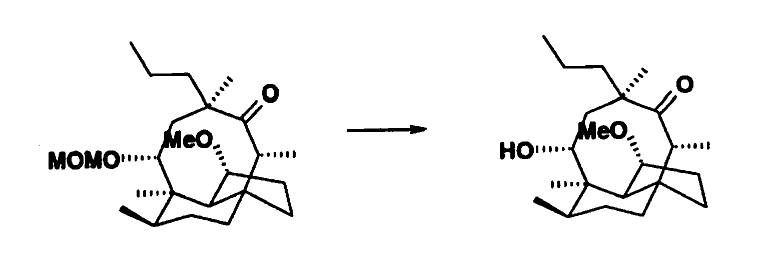

According to Reference Example 4, 2.19 g (5.55 mmol) of the
compound of Step I and 1.06 g (5.55 mmol) p-toluenesulfonic acid were used in the reaction. The resulting residue was purified by silica gel column chromatography (hexane:ethyl acetate = 4:1) to afford 1.36 g of the title compound as a yellow powder (70% yield). MS (FAB) (m/z): 351 (MH⁺) .
HRMS (FAB) (m/z) : Calcd. for C₂₂H₃₉O₃(MH⁺) : 351.2899. Found, 351. 2913.

### Step III

(3R)-14-{(3R,4S)-1-azabicyclo[2.2.1]heptane-3-carbonyl}carbamo yl-3-deoxo-11-deoxy-12-desethenyl-3-methoxy-11-oxo-12-propyl-4 -epimutilin

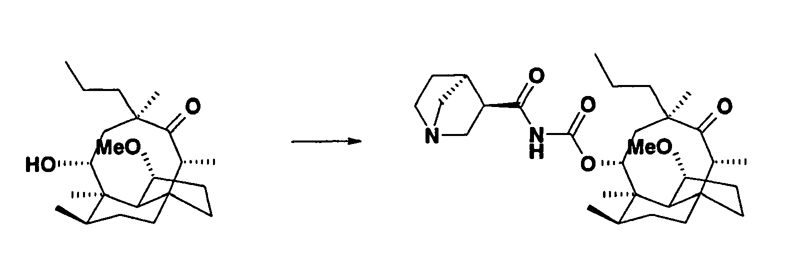

According to Step I of Example 1, the following compounds were used in the reaction: 500 mg (1.43 mmol) of the compound of Step II, an acid chloride prepared from 382 mg (2.15 mmol) carboxylic acid, 537 mg (3.58 mmol) silver cyanide and 0.30 mL (2.15 mmol) triethylamine. The resulting residue was purified by silica gel column chromatography (NH, ethyl acetate, and then ethyl acetate:methanol = 20:1) to afford 403 mg of the title compound as a colorless powder (55% yield).
MS (FAB) (m/z): 517 (MH⁺).
HRMS (FAB) (m/z): Calcd. for C₃₀H₄₉N₂O₅ (MH⁺) : 517. 3641. Found, 517.3601.

### Step IV

14-{(3R,4S)-1-azabicyclo[2.2.1]heptane-3-carbonyl}carbamoyl-12 -desethenyl-12-propylmutilin

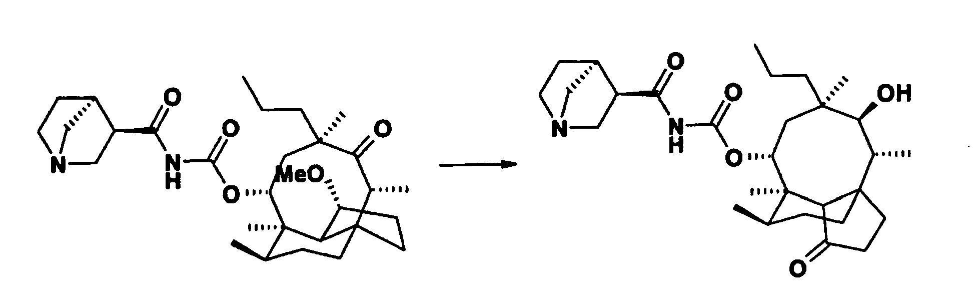

According to Step II of Example 1, 367 mg (0.71 mmol) of the
compound of Step III was used in the reaction. The resulting residue was purified by silica gel column chromatography (NH, ethyl acetate, and then ethyl acetate:methanol = 20:1) to afford 312 mg of the title compound as a colorless powder (87% yield).
MS (FAB) (m/z): 503 (MH⁺).
HRMS (FAB) (m/z): Calcd. for C₂₉H₄₇N₂O₅(MH⁺): 503.3485. Found, 503.3481.

### Step V

14-{(3R,4S)-1-azabicyclo[2.2.1]heptane-3-carbonyl}carbamoyl-12 -desethenyl-12-propylmutilin hydrochloride

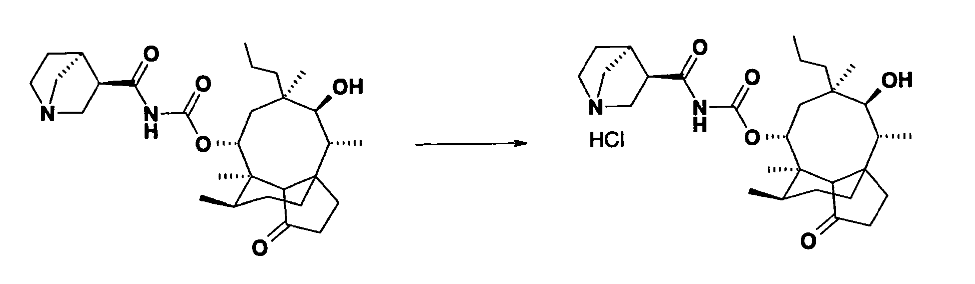

According to Step V of Example 2, 120 mg (0.24 mmol) of the
compound of Step IV was used in the reaction to afford 117 mg of the title compound (90% yield).
MS (FAB) (m/z): 503 (MH⁺ for free form).
HRMS (FAB) (m/z): Calcd. for C₂₉H₄₇N₂O₅(MH⁺ for free form) : 503.3485. Found, 503.3481.

### (Example 18)

### Step I

(3R)-12-butyl-3-deoxo-11-deoxy-12-desethenyl-3-methoxy-14-O-me thoxymethyl-11-oxo-4-epimutilin

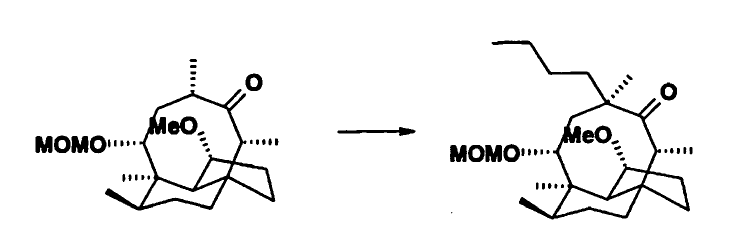

According to Step I of Example 2, 2.00 g (5.67 mmol) of the compound of Reference Example 3, 0.77 mL (6.81 mmol) 1-iodobutane and 13.6 mL (6.81 mmol) potassium bis(trimethylsilyl)amide (0.5 mol/L toluene solution) were used in the reaction. The resulting residue was purified by silica gel column chromatography (hexane:ethyl acetate =4:1) to afford 1.96 g of the title compound as a brown oil (85% yield). MS (FAB) (m/z): 409 (MH⁺) .
HRMS (FAB) (m/z) : Calcd. for C₂₅H₄₅O₄ (MH⁺) : 409.3318. Found, 409.3304.

### Step II

(3R)-12-butyl-3-deoxo-11-deoxy-12-desethenyl-3-methoxy-11-oxo-4-epimutilin

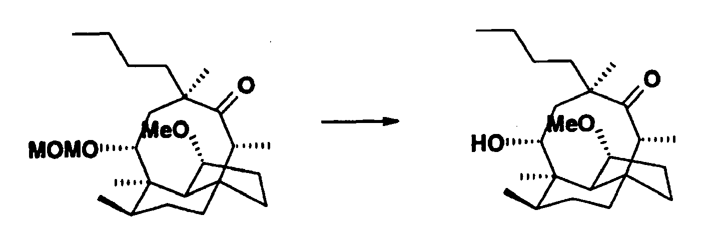

According to Reference Example 4, 1.96 g (4.80 mmol) of the
compound of Step I and 0.91 g (4.80 mmol) p-toluenesulfonic acid were used in the reaction. The resulting residue was purified by silica gel column chromatography (hexane:ethyl acetate = 4:1) to afford 1.19 g of the title compound as a brown oil (68% yield). MS (FAB) (m/z): 365 (MH⁺) .
HRMS (FAB) (m/z) : Calcd. for C₂₃H₄₁O₃(MH⁺): 365. 3056. Found, 365.3070.

### Step III

(3R)-12-butyl-14-{(3R,4S)-1-azabicyclo[2.2.1]heptane-3-carbony 1}carbamoyl-3-deoxo-11-deoxy-12-desethenyl-3-methoxy-11-oxo-4-epimutilin

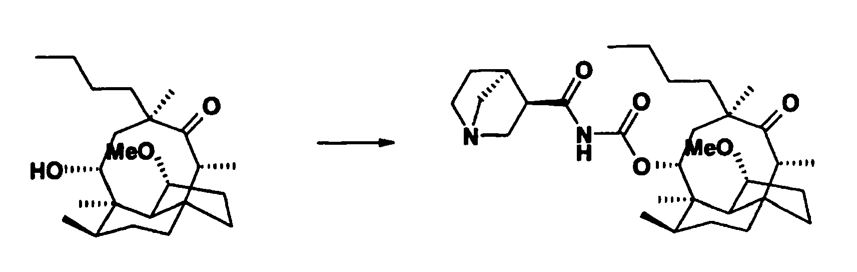

According to Step I of Example 1, the following compounds were used in the reaction: 500 mg (1.37 mmol) of the compound of Step II, an acid chloride prepared from 366 mg (2.06 mmol) carboxylic acid, 514 mg (3.43 mmol) silver cyanide and 0.29 mL (2.06 mmol) triethylamine. The resulting residue was purified by silica gel column chromatography (NH, ethyl acetate, and then ethyl acetate:methanol = 20:1) to afford 216 mg of the title compound as a colorless powder (23% yield).
MS (FAB) (m/z): 531 (MH⁺) .
HRMS (FAB) (m/z): Calcd. for C₃₁H₅₁N₂O₅(MH⁺) : 531.3798. Found, 531.3765.

### Step IV

12-butyl-14-{(3R,4S)-1-azabicyclo[2.2.1]heptane-3-carbonyl}car bamoyl-12-desethenylmutilin

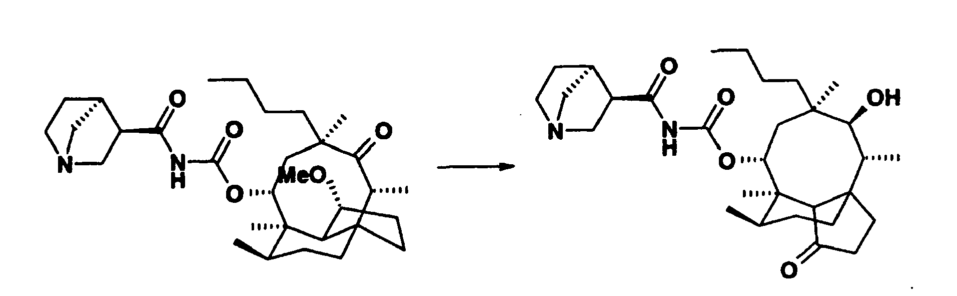

According to Step II of Example 1, 199 mg (0.29 mmol) of the compound of Step III was used in the reaction. The resulting residue was purified by silica gel column chromatography (NH, ethyl acetate, and then ethyl acetate:methanol = 10:1) to afford 80.2 mg of the title compound as a colorless powder (53% yield).
MS (FAB) (m/z): 517 (MH⁺).
HRMS (FAB) (m/z): Calcd. for C₃₀H₄₉N₂O₅(MH⁺): 517.3641. Found, 517.3608.

### (Example 19)

### Step I

(3R)-12-(1-chloropropane-3-yl)-3-deoxo-11-deoxy-12-desethenyl-3-methoxy-14-O-methoxymethyl-11-oxo-4-epimutilin

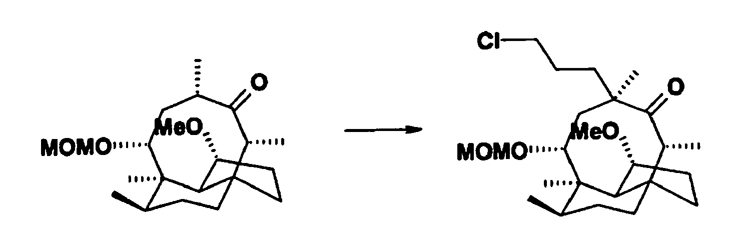

According to Step I of Example 2, 3.00 g (8.51 mmol) of the compound of Reference Example 3, 1.01 mL (10.2 mmol) 1-bromo-3-chloropropane and 20.4 mL (10.2 mmol) potassium bis(trimethylsilyl)amide (0.5 mol/L toluene solution) were used in the reaction. The resulting residue was purified by silica gel column chromatography (hexane:ethyl acetate = 4:1) to afford 2.68 g of the title compound as a colorless oil (73% yield).
MS (FAB) (m/z) : 367 (MH⁺-HOCH₂OCH₃).
HRMS (FAB) (m/z) : Calcd. for C₂₂H₃₆ClO₂(MH⁺-HOCH₂OCH₃) : 367.2404. Found, 367.2440.

### Step II

(3R)-12-(1-chloropropane-3-yl)-3-deoxo-11-deoxy-12-desethenyl-3-methoxy-11-oxo-4-epimutilin

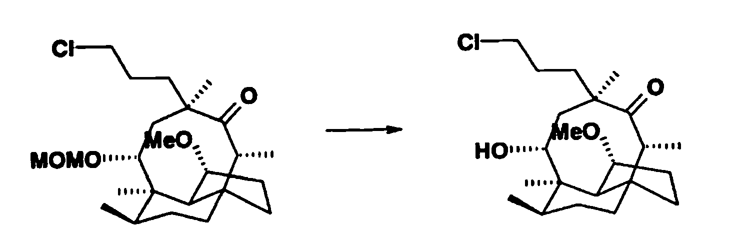

According to Reference Example 4, 2.68 g (6.25 mmol) of the compound of Step I and 1.19 g (6.25 mmol) p-toluenesulfonic acid were used in the reaction. The resulting residue was purified by silica gel column chromatography (hexane:ethyl acetate = 4:1, followed by hexane:ethyl acetate = 2:1) to afford 1.19 g of the title compound as a colorless powder (79% yield).
MS (FAB) (m/z): 367 (MH⁺) .
HRMS (FAB) (m/z): Calcd. for C₂₂H₃₆ClO₃ (MH⁺) : 367.2404. Found, 367.2397.

### Step III

(3R)-14-{(3R,4S)-1-azabicyclo[2.2.1]heptane-3-carbonyl}carbamo yl-12-(1-chloropropane-3-yl)-3-deoxo-11-deoxy-12-desethenyl-3-methoxy-11-oxo-4-epimutilin

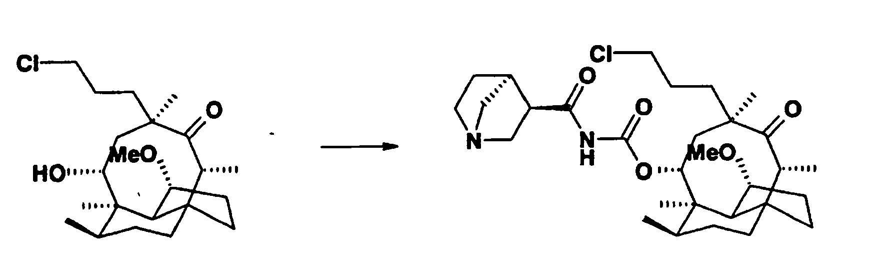

According to Step I of Example 1, the following compounds were used in the reaction: 600 mg (1.56 mmol) of the compound of Step II, an acid chloride prepared from 416 mg (2.34 mmol) carboxylic acid, 581 mg (3.90 mmol) silver cyanide and 0.33 mL (2.34 mmol) triethylamine. The resulting residue was purified by silica gel column chromatography (NH, ethyl acetate, and then ethyl acetate:methanol = 20:1) to afford 76.6 mg of the title compound as a colorless powder (9% yield). MS (FAB) (m/z): 551 (MH⁺).
HRMS (FAB) (m/z) : Calcd. for C₃₀H₄₈ClN₂O₅(MH⁺) : 551.3252. Found, 551.3203.

### Step IV

14-{(3R,4S)-1-azabicyclo[2.2.1]heptane-3-carbonyl}carbamoyl-12 -(1-chloropropane-3-yl)-12-desethenylmutilin

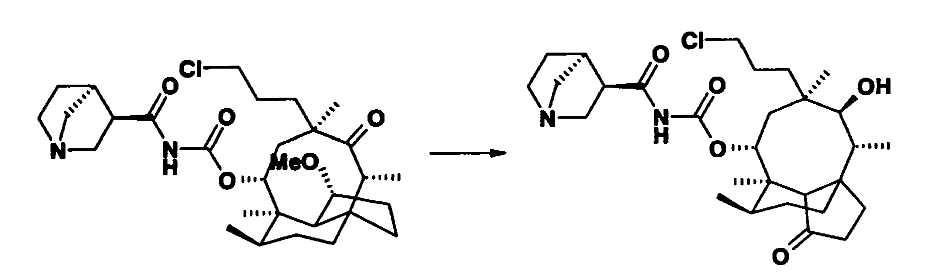

According to Step II of Example 1, 76.6 mg (0.14 mmol) of the
compound of Step III was used in the reaction. The resulting residue was purified by silica gel column chromatography (NH, ethyl acetate, and then ethyl acetate:methanol = 10:1) to afford 43.5 mg of the title compound as a colorless powder (58% yield).
MS (FAB) (m/z) : 537 (MH⁺) .
HRMS (FAB) (m/z): Calcd. for C₂₉H₄₆ClN₂O₅ (MH⁺) : 537.3095. Found, 537.3120.

### (Example 20)

### Step I

(3R)-12-[(E)-2-butene-4-yl]-3-deoxo-11-deoxy-12-desethenyl-3-m ethoxy-14-O-methoxymethyl-11-oxo-4-epimutilin

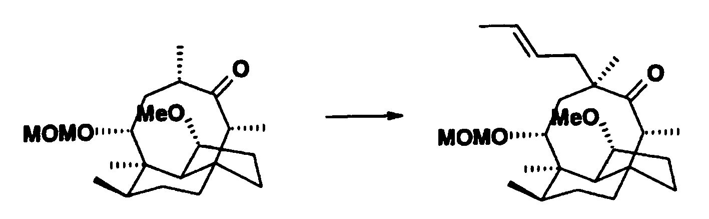

According to Step I of Example 2, 3.00 g (8.51 mmol) of the reference Example 3, 1.00 mL (10.2 mmol) crotyl chloride and 20. 4mL (10.2 mmol) potassium bis(trimethylsilyl)amide (0.5 mol/L toluene solution) were used in the reaction. The resulting residue was purified by silica gel column chromatography (hexane:ethyl acetate = 4:1) to afford 2.96 g of the title compound as a colorless powder (86% yield).
MS (FAB) (m/z) : 345 (MH⁺-HOCH₂OCH₃)
HRMS (FAB) (m/z) : Calcd. for C₂₃H₃₇O₂(MH⁺-HOCH₂OCH₃) : 345.2794. Found, 345.2814.

### Step II

(3R)-12-[(E)-2-butene-4-yl]-3-deoxo-11-deoxy-12-desethenyl-3-m ethoxy-11-oxo-4-epimutilin

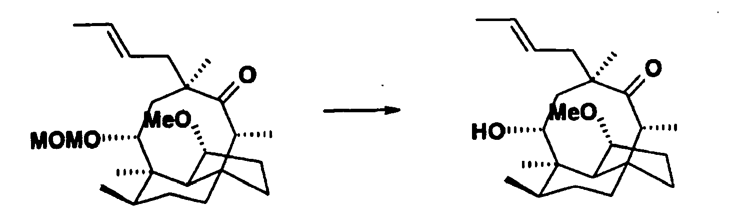

According to Reference Example 4, 2.96 g (7.28 mmol) of the
compound of Step I and 1.38 g (7.28 mmol) p-toluenesulfonic acid were used in the reaction. The resulting residue was purified by silica gel column chromatography (hexane:ethyl acetate = 4:1) to afford 2.28 g of the title compound as a colorless powder (81% yield). MS (FAB) (m/z): 345 (MH⁺-H₂O).
HRMS (FAB (m/z): Calcd. for C₂₃H₃₇O₂(MH⁺-H₂O): 345.2794. Found, 345.2814.

### Step III

(3R)-14-{(3R,4S)-1-azabicyclo[2.2.1]heptane-3-carbonyl}carbamo yl-12-[(E)-2-butene-4-yl]-3-deoxo-11-deoxy-12-desethenyl-3-met hoxy-11-oxo-4-epimutilin

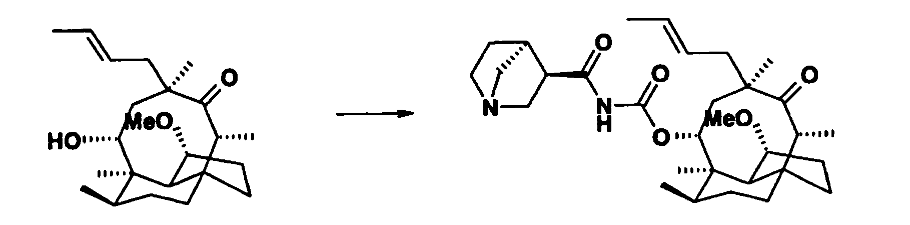

According to Step I of Example 1, the following compounds were used in the reaction: 500 mg (1.38 mmol) of the compound of Step II, an acid chloride prepared from 368 mg (2.07 mmol) carboxylic acid, 517 mg (3.45 mmol) silver cyanide and 0.29 mL (2.07 mmol) triethylamine. The resulting residue was purified by silica gel column chromatography (ethyl acetate, followed by ethyl acetate:methanol = 20:1) to afford 150 mg of the title compound as a colorless powder (21% yield).
MS (FAB) (m/z): 529 (MH⁺).
HRMS (FAB) (m/z): Calcd. for C₃₁H₄₉N₂O₅ (MH⁺) : 529.3641. Found, 529.3658.

### Step IV

14-{(3R,4S)-1-azabicyclo[2.2.1]heptane-3-carbonyl}carbamoyl-12 -[(E)-2-butene-4-yl]-12-desethenylmutilin

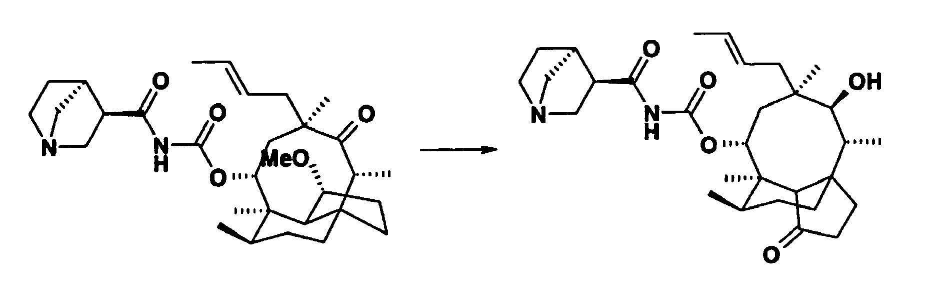

According to Step II of Example 1, 136 mg (0.26 mmol) of the compound of Step III was used in the reaction. The resulting residue was purified by silica gel column chromatography (NH, ethyl acetate, and then ethyl acetate:methanol = 10:1) to afford 122 mg of the title compound as a colorless powder (91% yield).
MS (FAB) (m/z): 515 (MH⁺) .
HRMS (FAB) (m/z): Calcd. for C₃₀H₄N₂O₅ (MH⁺) : 515.3485. Found, 515.3511.

### (Example 21)

### Step I

(3R)-3-deoxo-11-deoxy-12-desethenyl-3-methoxy-14-O-methoxymeth yl-12-[(2-methyl)propane-3-yl]-11-oxo-4-epimutilin

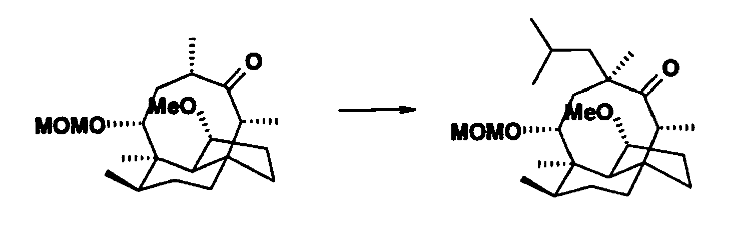

According to Step I of Example 2, 1.00 g (2.84 mmol) of the compound of Reference Example 3, 0.37 mL (3.40 mmol) 1-bromo-2-methylpropane and 6.81 mL (3.40 mmol) potassium bis(trimethylsilyl)amide (0.5 mol/L toluene solution) were used in the reaction. The resulting residue was purified by silica gel column chromatography (hexane:ethyl acetate = 4:1) to afford 926 mg of the title compound as a colorless oil (80% yield).
MS (FAB) (m/z): 409 (MH⁺) .
HRMS (FAB) (m/z) : Calcd. for C₂₅H₄₅O₄(MH⁺) : 409.3274. Found, 409.3313.

### Step II

(3R)-3-deoxo-11-deoxy-12-desethenyl-3-methoxy-12-[(2-methyl)pr opane-3-yl]-11-oxo-4-epimutilin

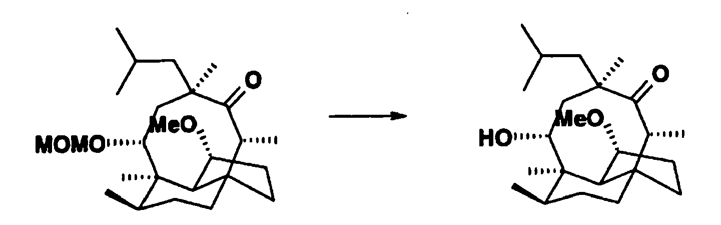

According to Reference Example 4, 926 mg (2.27 mmol) of the
compound of Step I and 0.43 g (2.27 mmol) p-toluenesulfonic acid were used in the reaction. The resulting residue was purified by silica gel column chromatography (hexane:ethyl acetate = 4:1) to afford 636 mg of the title compound as a colorless powder (77% yield). MS (FAB) (m/z): 365 (MH⁺) .
HRMS (FAB) (m/z) : Calcd. for C₂₃H₄₁O₃ (MH⁺) : 365. 3056. Found, 365.3065.

### Step III

(3R)-14-{(3R,4S)-1-azabicyclo[2.2.1]heptane-3-carbonyl}carbamo yl-3-deoxo-11-deoxy-12-desethenyl-3-methoxy-12-[(2-methyl)prop ane-3-yl]-11-oxo-4-epimutilin

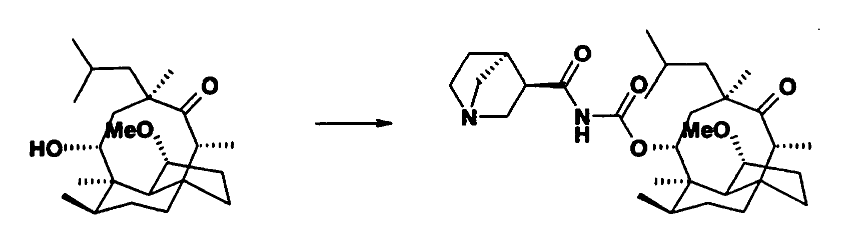

According to Step I of Example 1, the following compounds were used in the reaction: 300 mg (0.82 mmol) of the compound of Step II, an acid chloride prepared from 218 mg (1.23 mmol) carboxylic acid, 307 mg (2.05 mmol) silver cyanide and 0.17 mL (1.23 mmol) triethylamine. The resulting residue was purified by silica gel column chromatography (NH, ethyl acetate, and then ethyl acetate:methanol = 20:1) to afford 143 mg of the title compound as a colorless powder (33% yield).
MS (FAB) (m/z): 531 (MH⁺).
HRMS (FAB) (m/z): Calcd. for C₃₁H₅₁N₂O₅(MH⁺) : 531.3798. Found, 531.3802.

### Step IV

14-{(3R,4S)-1-azabicyclo[2.2.1]heptane-3-carbonyl}carbamoyl-12 -desethenyl-12-[(2-methyl)propane-3-yl]mutilin

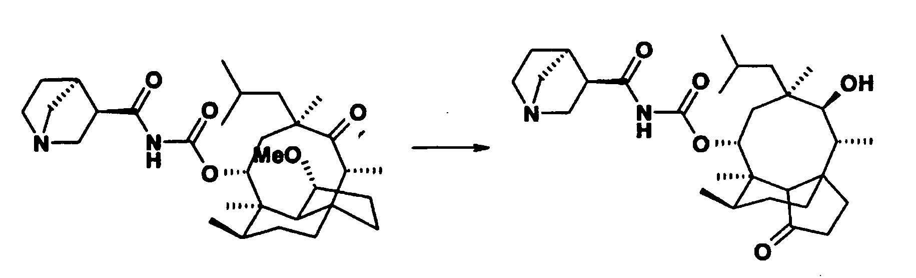

According to Step II of Example 1, 121 mg (0.23 mmol) of the compound of Step III was used in the reaction. The resulting residue was purified by silica gel column chromatography (NH, ethyl acetate:methanol = 20:1) to afford 97.3 mg of the title compound as a colorless powder (82% yield).
MS (FAB) (m/z): 517 (MH⁺) .
HRMS (FAB) (m/z): Calcd. for C₃₀H₄₉N₂O₅(MH⁺): 517.3641. Found, 517.3660.

### (Example 22)

### Step I

(3R)-12-cyclohexyl-3-deoxo-11-deoxy-12-desethenyl-3-methoxy-14 -O-methoxymethyl-11-oxo-4-epimutilin

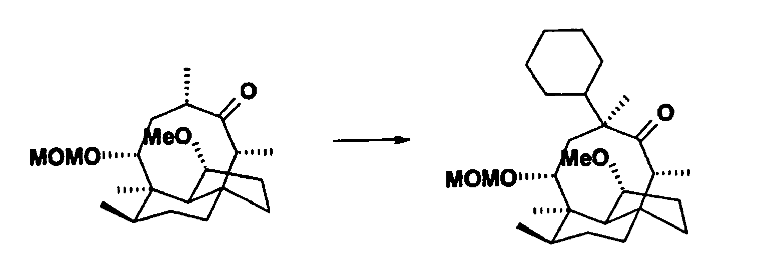

According to Step I of Example 2, 1.00 g (2.84 mmol) of the compound of Reference Example 3, 0.52 mL (4.26 mmol) cyclohexyl bromide and 6. 81 mL (3. 40 mmol) potassium bis (trimethylsilyl) amide (0.5 mol/L toluene solution) were used in the reaction. The resulting residue was purified by silica gel column chromatography (hexane:ethyl acetate = 4:1) to afford 436 mg of the title compound as a colorless oil (35% yield).
MS (FAB) (m/z): 435 (MH⁺) .
HRMS (FAB) (m/z) : Calcd. for C₂₇H₄₇O₄ (MH⁺) : 435.3474. Found, 435.3469.

### Step II

(3R)-12-cyclohexyl-3-deoxo-11-deoxy-12-desethenyl-3-methoxy-11 -oxo-4-epimutilin

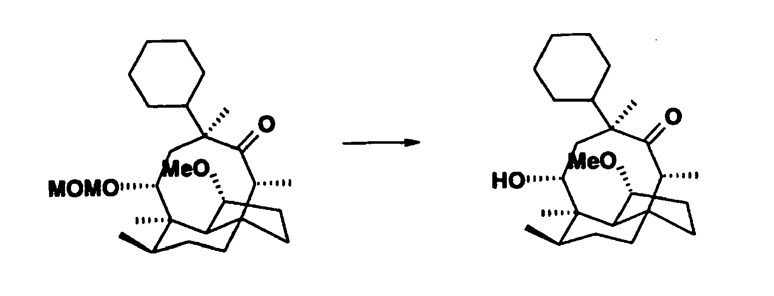

According to Reference Example 4, 436 mg (1.00 mmol) of the compound of Step I and 191 mg (1.00 mmol) p-toluenesulfonic acid were used in the reaction. The resulting residue was purified by silica gel column chromatography (hexane:ethyl acetate = 4:1) to afford 266 mg of the title compound as a colorless powder (68% yield). MS (FAB) (m/z): 391 (MH⁺) .
HRMS (FAB) (m/z) : Calcd. for C₂₅H₄₃O₃ (MH⁺) : 391.3212. Found, 391.3214.

### Step III

(3R)-14-{(3R,4S)-1-azabicyclo[2.2.1]heptane-3-carbonyl}carbamo yl-12-cyclohexyl-3-deoxo-11-deoxy-12-desethenyl-3-methoxy-11-o xo-4-epimutilin

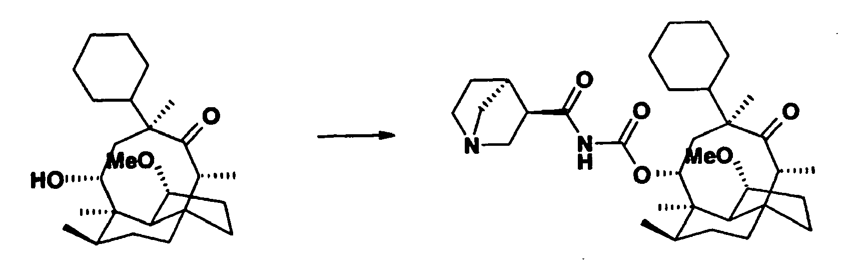

According to Step I of Example 1, the following compounds were used in the reaction: 200 mg (0.51 mmol) of the compound of Step II, an acid chloride prepared from 137 mg (0.77 mmol) carboxylic acid, 192 mg (1.28 mmol) silver cyanide and 0.11 mL (0.77 mmol) triethylamine. The resulting residue was purified by silica gel column chromatography (NH, ethyl acetate, and then ethyl acetate:methanol = 20:1) to afford 162 mg of the title compound as a colorless powder (57% yield).
MS (FAB) (m/z): 557 (MH⁺). HRMS (FAB) (m/z): Calcd. for C₃₃H₅₃N₂O₅(MH⁺) : 557.3954. Found, 557.3974.

### Step IV

14-{(3R,4S)-1-azabicyclo[2.2.1]heptane-3-carbonyl}carbamoyl-12 -cyclohexyl-12-desethenylmutilin

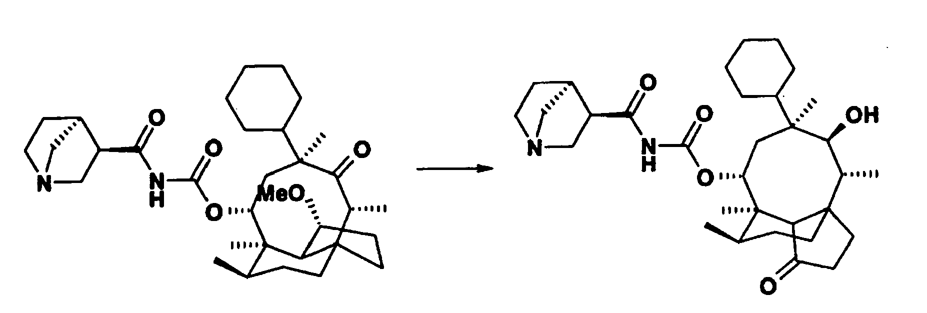

According to Step II of Example 1, 127 mg (0.23 mmol) of the
compound of Step III was used in the reaction. The resulting residue was purified by silica gel column chromatography (NH, ethyl acetate, and then ethyl acetate:methanol = 20:1) to afford 76.7 mg of the title compound as a colorless powder (64% yield).
MS (FAB) (m/z): 543 (MH⁺) .
HRMS (FAB) (m/z): Calcd. for C₃₂H₅₁N₂O₅ (MH⁺) : 543.3798. Found, 543.3844.

### (Example 23)

### Step I

(3R)-3-deoxo-11-deoxy-12-desethenyl-3-methoxy-12-(1-methoxyeth ane-2-yl)-14-O-methoxymethyl-11-oxo-4-epimutilin

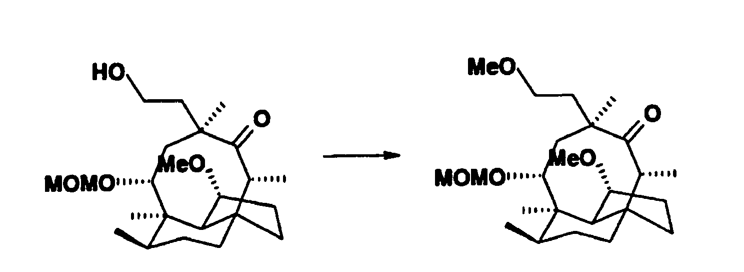

According to Step II of Example 14, the following compounds were used in the reaction: 0.95 g (2.40 mmol) (3R)-3-deoxo-11-deoxy-12-desethenyl-12-(1-hydroxyethane-2-yl)-3-methoxy-14-O-methoxymethyl-11-oxo-4-epimutilin, 0.14 g (3.59 mmol) sodium hydride (60% oil) and 0.45 mL (7.19 mmol) methyl iodide. The resulting residue was purified by silica gel column chromatography (hexane:ethyl acetate = 4:1) to afford 0.98 g of the title compound as a colorless oil (100% yield).
MS (FAB) (m/z) : 349 (MH⁺-HOCH₂OCH₃).
HRMS (FAB) (m/z) : Calcd. for C₂₂H₃₇O₃ (MH⁺- HOCH₂OCH₃) : 349.2743. Found, 349.2737.

### Step II

(3R)-3-deoxo-11-deoxy-12-desethenyl-3-methoxy-12-(1-methoxyeth ane-2-yl)-11-oxo-4-epimutilin

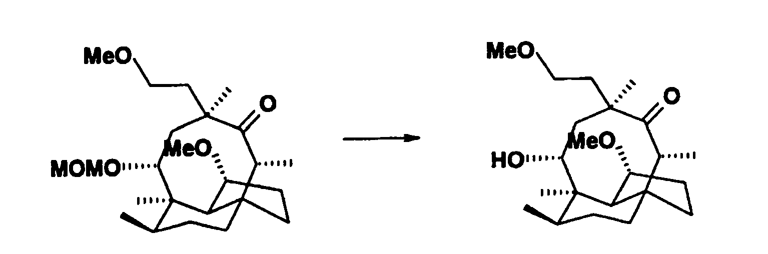

According to Reference Example 4, 0.98 g (2.40 mmol) and 0.46 g (2.40 mmol) p-toluenesulfonic acid were used in the reaction. The resulting residue was purified by silica gel column chromatography (hexane:ethylacetate = 4:1, followed by hexane:ethyl acetate = 1:1) to afford 633 mg of the title compound as a colorless oil (72% yield).
MS (FAB) (m/z): 349 (MH⁺-H₂O).
HRMS (FAB) (m/z) : Calcd. for C₂₂H₃₇O₃(MH⁺-H₂O) : 349.2743. Found, 349.2737.

### Step III

(3R)-14-{(3R,4S)-1-azabicyclo[2.2.1]heptane-3-carbonyl}carbamo yl-3-deoxo-11-deoxy-12-desethenyl-3-methoxy-12-(1-methoxyethan e-2-yl)-11-oxo-4-epimutilin

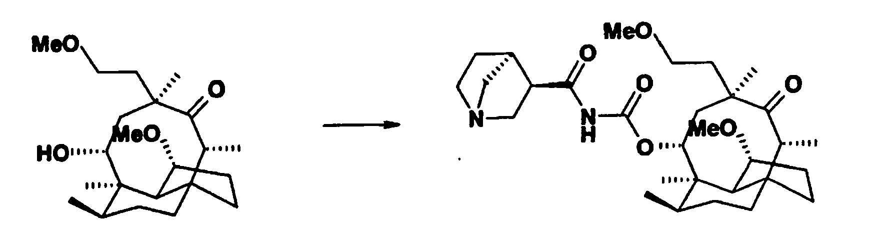

According to Step I of Example 1, the following compounds were used in the reaction: 300 mg (0.82 mmol) of the compound of Step II, an acid chloride prepared from 218 mg (1.23 mmol) carboxylic acid, 307 mg (2.05 mmol) silver cyanide and 0.17 mL (1.23 mmol) triethylamine. The resulting residue was purified by silica gel column chromatography (NH, ethyl acetate, and then ethyl acetate:methanol = 20:1) to afford 166 mg of the title compound as a colorless powder (38% yield).
MS (FAB) (m/z) : 533 (MH⁺) .
HRMS (FAB) (m/z): Calcd. for C₃₀H₄₉N₂O₆ (MH⁺) : 533.3591. Found, 533.3580.

### Step IV

14-{(3R,4S)-1-azabicyclo[2.2.1]heptane-3-carbonyl}carbamoyl-12 -desethenyl-12-(1-methoxyethane-2-yl)mutilin

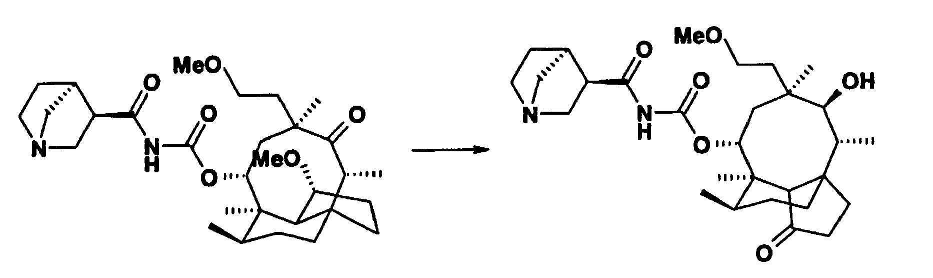

According to Step II of Example 1, 145 mg (0.27 mmol) of the compound of Step III was used in the reaction. The resulting residue was purified by silica gel column chromatography (NH, ethyl acetate:methanol = 30:1) to afford 126 mg of the title compound as a colorless powder (90% yield).
MS (FAB) (m/z): 519 (MH⁺) .
HRMS (FAB) (m/z): Calcd. for C₂₉H₄₇N₂O₆(MH⁺): 519.3434. Found, 519.3458.

### (Example 24)

### Step I

(3R)-12-(1-chloropropene-3-yl)-3-deoxo-11-deoxy-12-desethenyl-3-methoxy-14-O-methoxymethyl-11-oxo-4-epimutilin

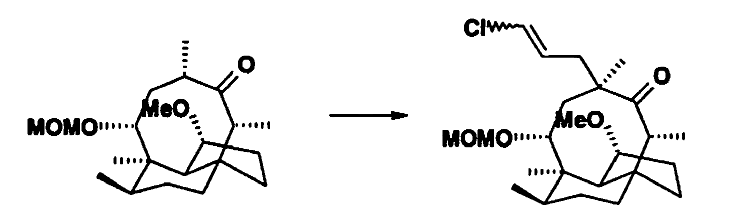

According to Step I of Example 2, 1.00 g (2.84 mmol) of the compound of Reference Example 3, 0.35 mL (3.40 mmol) 1,3-dichloro-1-propene and 6.81 mL (3.40 mmol) potassium bis(trimethylsilyl)amide (0.5 mol/L toluene solution) were used in the reaction. The resulting residue was purified by silica gel column chromatography (hexane:ethyl acetate = 4:1) to afford 1.00 g of the title compound as a yellow oil (83% yield).
MS (FAB) (m/z): 427 (MH⁺) .
HRMS (FAB) (m/z): Calcd. for C₂₄H₄₀ClO₄(MH⁺): 427.2615. Found, 427.2598.

### Step II

(3R)-12-(1-chloropropene-3-yl)-3-deoxo-11-deoxy-12-desethenyl-3-methoxy-11-oxo-4-epimutilin

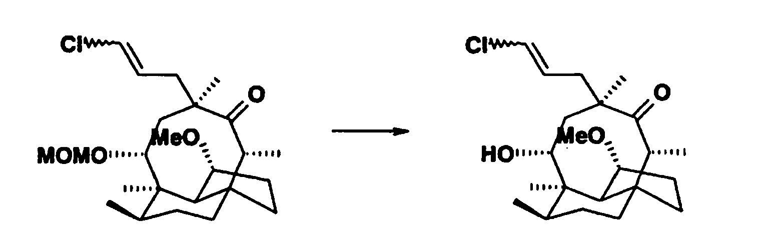

According to Reference Example 4, 1.00 g (2.34 mmol) of the compound of Step I and 0.45 g (2.34 mmol) p-toluenesulfonic acid were used in the reaction. The resulting residue was purified by silica gel column chromatography (hexane:ethyl acetate = 4:1) to afford 593 mg of the title compound as a colorless powder (66% yield). MS (FAB) (m/z): 383 (MH⁺).
HRMS (FAB) (m/z): Calcd. for C₂₂H₃₆ClO₃(MH⁺): 383.2353. Found, 383.2326.

### Step III

(3R)-14-{(3R,4S)-1-azabicyclo[2.2.1]heptane-3-carbonyl}carbamo yl-12-(1-chloropropene-3-yl)-3-deoxo-11-deoxy-12-desethenyl-3-methoxy-11-oxo-4-epimutilin

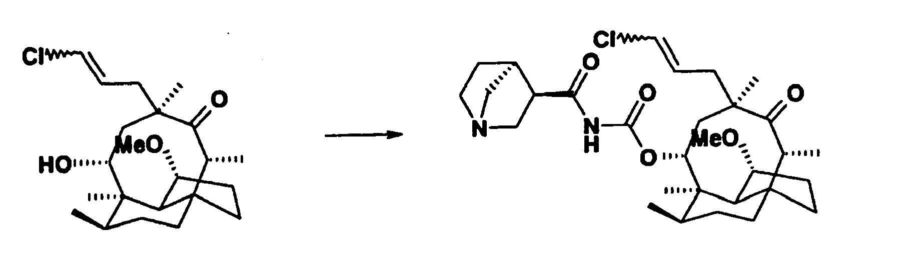

According to Step I of Example 1, the following compounds were used in the reaction: 300 mg (0.78 mmol) of the compound of Step II, an acid chloride prepared from 208 mg (1.17 mmol) carboxylic acid, 292 mg (1.95 mmol) silver cyanide and 0.16 mL (1.17 mmol) triethylamine. The resulting residue was purified by silica gel column chromatography (NH, ethyl acetate, and then ethyl acetate:methanol = 20:1) to afford 184 mg of the title compound as a colorless powder (43% yield).
MS (FAB) (m/z): 549 (MH⁺) . HRMS (FAB) (m/z): Calcd. for C₃₀H₄₆ClN₂O₅(MH⁺) : 549.3095. Found, 549.3054.

### Step IV

14-{(3R,4S)-1-azabicyclo[2.2.1]heptane-3-carbonyl}carbamoyl-12 -(1-chloropropene-3-yl)-12-desethenylmutilin

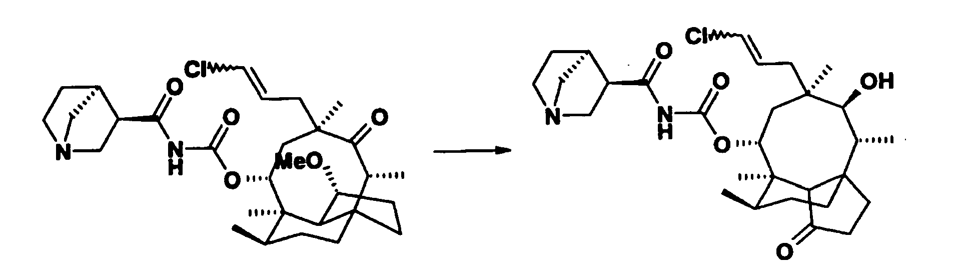

According to Step II of Example 1, 151 mg (0.27 mmol) of the compound of Step III was used in the reaction. The resulting residue was purified by silica gel column chromatography (NH, ethyl acetate:methanol = 30:1) to afford 128 mg of the title compound as a colorless powder (89% yield).
MS (FAB) (m/z): 535 (MH⁺) .
HRMS (FAB) (m/z): Calcd. for C₂₉H₄₄ClN₂O₅ (MH⁺) : 535.2939. Found, 535.2918.

### (Example 25)

### Step I

(3R)-3-deoxo-11-deoxy-12-desethenyl-12-ethoxymethyl-3-methoxy-14-O-methoxymethyl-11-oxo-4-epimutilin

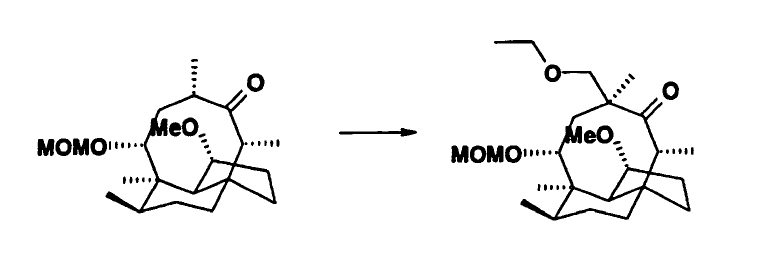

According to Step I of Example 2, 2.00 g (5.67 mmol) of the compound of Reference Example 3, 0.63 mL (6.81 mmol) chloromethyl ethyl ether and 13.6 mL (6.81 mmol) potassium bis (trimethylsilyl) amide (0.5mol/L toluene solution) were used in the reaction. The resulting residue was purified by silica gel column chromatography (hexane:ethyl acetate = 5:1) to afford 2.44 g of the title compound as a colorless oil (96% yield).
MS (FAB) (m/z) : 349 (MH⁺-HOCH₂OCH₃).
HRMS (FAB) (m/z) : Calcd. for C₂₂H₃₇O₃ (MH⁺-HOCH₂OCH₃) : 349.2743. Found, 349.2767.

### Step II

(3R)-3-deoxo-11-deoxy-12-desethenyl-12-ethoxymethyl-3-methoxy-11-oxo-4-epimutilin

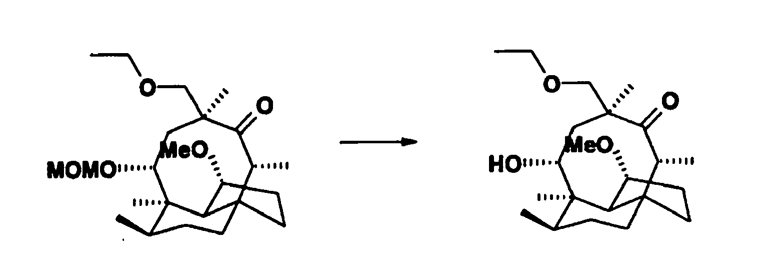

According to Reference Example 4, 2.24 g (5.46 mmol) of the
compound of Step I and 1.04 g (5.46 mmol) p-toluenesulfonic acid were used in the reaction. The resulting residue was purified by silica gel column chromatography (hexane:ethyl acetate = 2:1) to afford 1. 70 g of the title compound as a colorless powder (85% yield) . MS (FAB) (m/z): 349 (MH⁺-H₂O) .
HRMS (FAB) (m/z): Calcd. for C₂₂H₃₇O₃(MH⁺-H₂O) : 349.2743. Found, 349.2747.

### Step III

(3R)-14-{(3R,4S)-1-azabicyclo[2.2.1]heptane-3-carbonyl}carbamo yl-3-deoxo-11-deoxy-12-desethenyl-12-ethoxymethyl-3-methoxy-11 -oxo-4-epimutilin

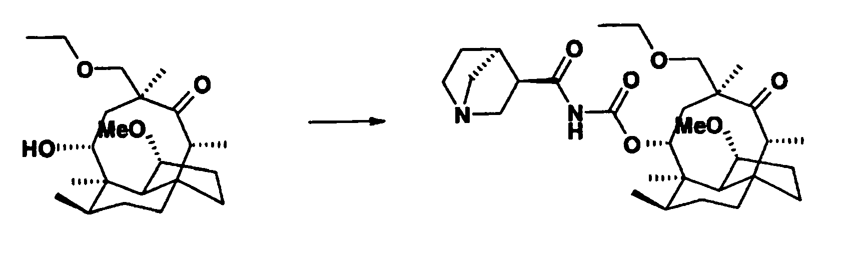

According to Step I of Example 1, the following compounds were used in the reaction: 300 mg (0.82 mmol) of the compound of Step II, an acid chloride prepared from 218 mg (1.23 mmol) carboxylic acid, 307 mg (2.05 mmol) silver cyanide and 0.17 mL (1.23 mmol) triethylamine. The resulting residue was purified by silica gel column chromatography (NH, ethyl acetate, and then ethyl acetate:methanol = 20:1) to afford 256 mg of the title compound as a colorless powder (59% yield).
MS (FAB) (m/z): 533 (MH⁺).
HRMS (FAB) (m/z): Calcd. for C₃₀H₄₉N₂O₆(MH⁺) : 533.3591. Found, 533.3622.

### Step IV

14-{(3R,4S)-1-azabicyclo[2.2.1]heptane-3-carbonyl}carbamoyl-12 -desethenyl-12-ethoxymethylmutilin

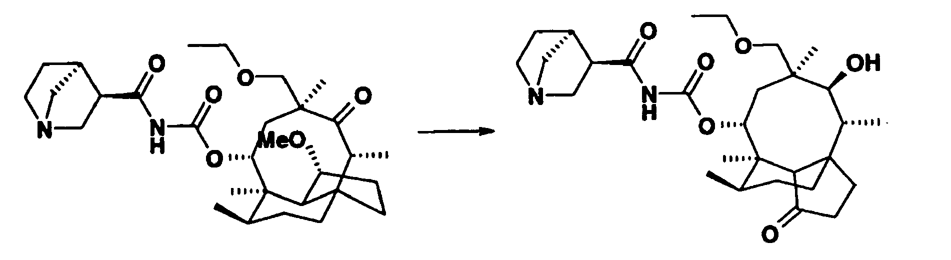

According to Step II of Example 1, 200 mg (0.38 mmol) of the compound of Step III was used in the reaction. The resulting residue was purified by silica gel column chromatography (NH, ethyl acetate:methanol = 30:1) to afford 156 mg of the title compound as a colorless powder (79% yield).
MS (FAB) (m/z): 519 (MH⁺).
HRMS (FAB) (m/z): Calcd. for C₂₉H₄₇N₂O₆(MH⁺) : 519.3434. Found, 519.3475.

### (Example 26)

### Step I

(3R)-3-deoxo-11-deoxy-12-desethenyl-12-(1-fluoropropene-3-yl)-3-methoxy-14-O-methoxymethyl-11-oxo-4-epimutilin

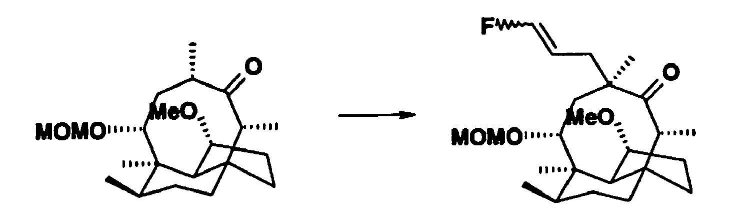

According to Step I of Example 2, the following compounds were used in the reaction: 1.42 g (4.02 mmol) of the compound of Reference Example 3, 912 mg (9.65 mmol) of 3-chloro-1-fluoro-1-propene prepared by a process described in literature (Tetrahedron Lett. 1988, 29, 53-56.) and 9.65 mL (4.83 mmol) potassium bis(trimethylsilyl)amide (0.5 mol/L toluene solution). The resulting residue was purified by silica gel column chromatography (hexane:ethyl acetate = 10:1) to afford 846 mg of the title compound as a colorless oil (51% yield).
MS (FAB) (m/z): 349 (MH⁺-HOCH₂OCH₃).
HRMS (FAB) (m/z) : Calcd. for C₂₂H₃₄FO₂(MH⁺-HOCH₂OCH₃): 349.2543. Found, 349.2574.

### Step II

(3R)-3-deoxo-11-deoxy-12-desethenyl-12-(1-fluoropropene-3-yl)-3-methoxy-11-oxo-4-epimutilin

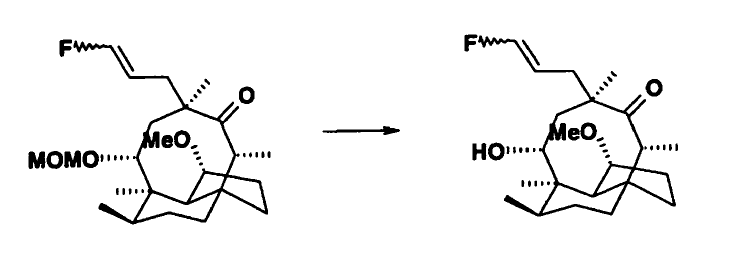

According to Reference Example 4, 846 mg (2.06 mmol) of the compound of Step I and 392 mg (2.06 mmol) p-toluenesulfonic acid were used in the reaction. The resulting residue was purified by silica gel column chromatography (hexane:ethyl acetate = 2:1) to afford 544 mg of the title compound as a colorless powder (72% yield) .
MS (FAB) (m/z): 367 (MH⁺) .
HRMS (FAB) (m/z): Calcd. for C₂₂H₃₆FO₃ (MH⁺) : 367.2648. Found, 367.2664.

### Step III

(3R)-14-{(3R,4S)-1-azabicyclo[2.2.1]heptane-3-carbonyl}carbamo yl-3-deoxo-11-deoxy-12-desethenyl-12-(1-fluoropropene-3-yl)-3-methoxy-11-oxo-4-epimutilin

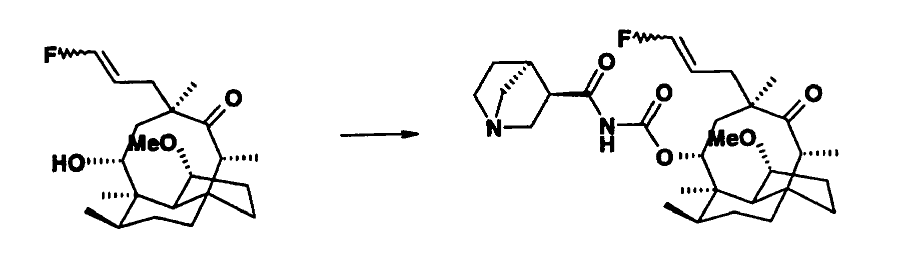

According to Step I of Example 1, the following compounds were used in the reaction: 300 mg (0.82 mmol) of the compound of Step II, an acid chloride prepared from 218 mg (1.23 mmol) carboxylic acid, 307 mg (2.05 mmol) silver cyanide and 0.17 mL (1.23 mmol) triethylamine. The resulting residue was purified by silica gel column chromatography (NH, ethyl acetate, and then ethyl acetate:methanol = 25:1) to afford 183mg of the title compound as a colorless powder (42% yield).
MS (FAB) (m/z) : 533 (MH⁺) .
HRMS (FAB) (m/z): Calcd. for C₃₀H₄₆FN₂O₅ (MH⁺): 533.3391. Found, 533.3375.

### Step IV

14-{(3R,4S)-1-azabicyclo[2.2.1]heptane-3-carbonyl}carbamoyl-12 -desethenyl-12-(1-fluoropropene-3-yl)mutilin

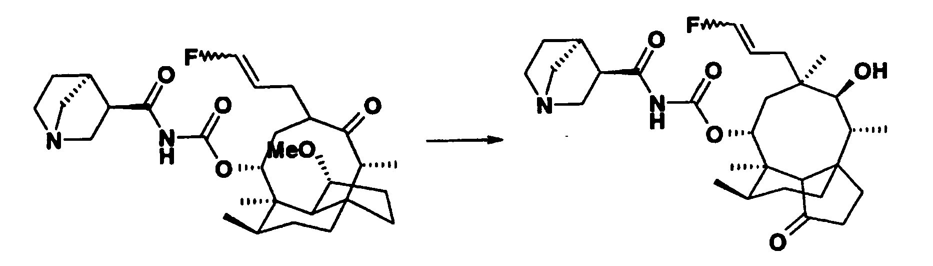

According Step II of Example 1, 170 mg (0.32 mmol) of the compound of Step III was used in the reaction. The resulting residue was purified by silica gel column chromatography (NH, ethyl acetate:methanol = 20:1) to afford 110 mg of the title compound as a colorless powder (66% yield).
MS (FAB) (m/z): 519 (MH⁺) .
HRMS (FAB) (m/z): Calcd. for C₂₉H₄₄FN₂O₅(MH⁺): 519.3234. Found, 519.3212.

### (Example 27)

### Step I

(3R)-3-deoxo-11-deoxy-12-desethenyl-12-(1-fluoropropane-3-yl)-3-methoxy-14-O-methoxymethyl-11-oxo-4-epimutilin

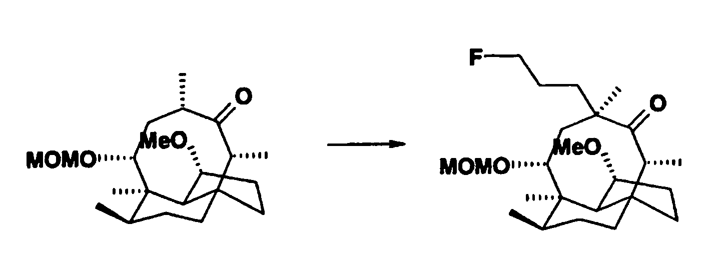

According to Step I of Example 2, the following compounds were used in the reaction: 2.00 g (5. 67 mmol) of the compound of Reference Example 3,1.84g (6.81mmol) 3-bromo-1-propanol-O-benzoate prepared from 3-bromo-1-propanol and benzoyl chloride, and 13.6 mL (6.81 mmol) potassium bis(trimethylsilyl)amide (0.5 mol/L toluene solution) . The resulting residue was purified by silica gel column chromatography (hexane:ethyl acetate = 4:1) to give 2.92 g of (3R)-12-[(1-benzoyloxy)propane-3-yl]-3-deoxo-11-deoxy-12-deset henyl-3-methoxy-14-O-methoxymethyl-11-oxo-4-epimutilin as a colorless oil (100% yield).
MS (FAB) (m/z) : 453 (MH⁺-HOCH₂OCH₃).
HRMS (FAB) (m/z) : Calcd. for C₂₉H₄₁O₄ (MH⁺- HOCH₂OCH₃) : 453.3005. Found, 453.2982.

According to Reference Example 3, 2.00 g (3.89 mmol) of this product was reacted with 1.07 g (7.77 mmol) potassium carbonate. The resulting residue was purified by silica gel column chromatography(hexane:ethylacetate=1:1,followedbyhexane:ethyl acetate = 1:4) to give 1.20 g of (3R)-3-deoxo-11-deoxy-12-desethenyl-12-(1-hydroxypropane-3-yl) -3-methoxy-14-O-methoxymethyl-11-oxo-4-epimutilin as a colorless oil (75% yield).
MS (FAB) (m/z) : 349 (MH⁺-HOCH₂OCH₃).
HRMS (FAB) (m/z) : Calcd. for C₂₂H₃₇O₃ (MH⁺-HOCH₂OCH₃) : 349.2743. Found, 349.2773.

According to Step I of Example 13, 1.20 g (2.92 mmol) of this compound was reacted with 1.21 mL (4.38 mmol) perfluorooctanesulfonyl fluoride. The resulting residue was purified by silica gel column chromatography (hexane: ethyl acetate = 10:1, followed by hexane:ethyl acetate = 4:1) to afford 142 mg of the title compound as a colorless oil (12% yield).
MS (EI) (m/z): 412 (M⁺).
HRMS (EI) (m/z) : Calcd. for C₂₄H₄₁FO₄(M⁺) : 412.2989. Found, 412.2991.

### Step II

(3R)-3-deoxo-11-deoxy-12-desethenyl-12-(1-fluoropropane-3-yl)-3-methoxy-11-oxo-4-epimutilin

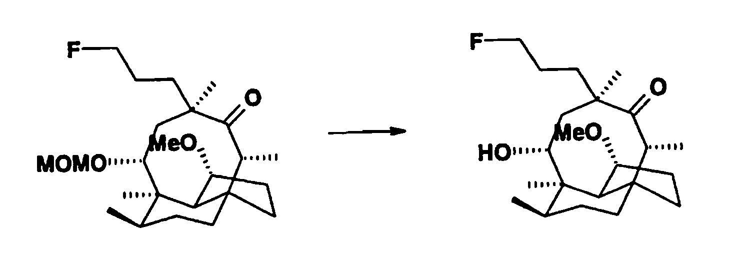

According to Reference Example 4, 142 mg (0.34 mmol) of the
compound of Step I and 65.5 mg (0.34 mmol) p-toluenesulfonic acid were used in the reaction. The resulting residue was purified by silica gel column chromatography (hexane:ethyl acetate = 2:1) to afford 41.8 mg of the title compound as a yellow oil (33% yield). MS (EI) (m/z): 368 (M⁺).
HRMS (EI) (m/z) : Calcd. for C₂₂H₃₇FO₃(M⁺): 368.2727. Found, 368.2727.

### Step III

(3R)-14-{(3R,4S)-1-azabicyclo[2.2.1]heptane-3-carbonyl}carbamo yl-3-deoxo-11-deoxy-12-desethenyl-12-(1-fluoropropane-3-yl)-3-methoxy-11-oxo-4-epimutilin

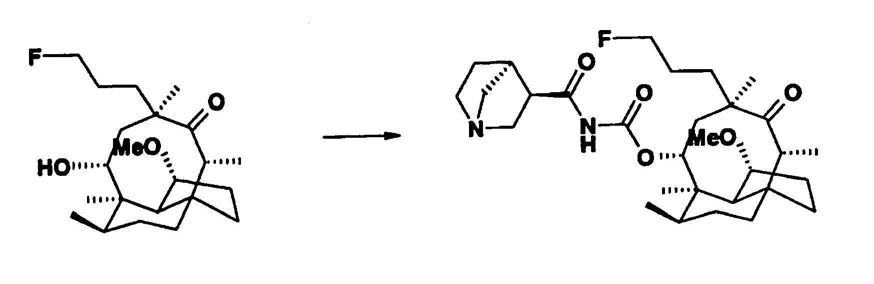

According to Step I of Example 1, the following compounds were used in the reaction: 41.8 mg (0.11 mmol) of the compound of Step II, an acid chloride prepared from 30.2 mg (0.17 mmol) carboxylic acid, 42.0 mg (0.28 mmol) silver cyanide and 23.7 µL (0.17mmol) triethylamine. The resulting residue was purified by silica gel column chromatography (NH, ethyl acetate, and then ethyl acetate:methanol = 20:1) to afford 20.7 mg of the title compound as a colorless powder (35% yield).
MS (FAB) (m/z): 535 (MH⁺) .
HRMS (FAB) (m/z): Calcd. for C₃₀H₄₈FN₂O₅(MH⁺): 535.3547. Found, 535.3570.

### Step IV

14-{(3R,4S)-1-azabicyclo[2.2.1]heptane-3-carbonyl}carbamoyl-12 -desethenyl-12-(1-fluoropropane-3-yl)mutilin

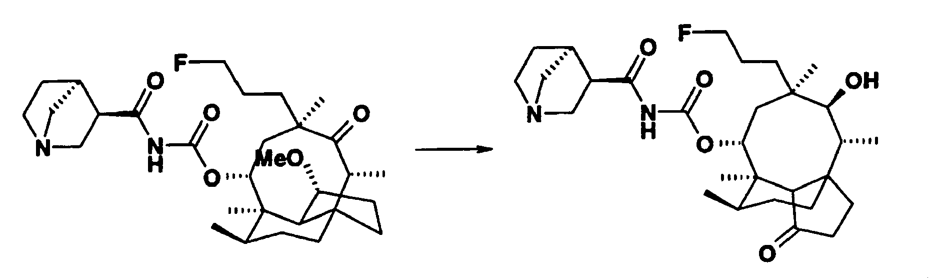

According to Step II of Example 1, 20.7 mg (38.7 µmol) of the
compound of Step III is used in the reaction. The resulting residue was purified by silica gel column chromatography (NH, ethyl acetate, and then ethyl acetate:methanol = 20:1) to afford 19.3 mg of the title compound as a colorless powder (96% yield).
MS (FAB) (m/z) : 521 (MH⁺).
HRMS (FAB) (m/z): Calcd. for C₂₉H₄₆FN₂O₅(MH⁺) : 521. 3391. Found, 521.3381.

### (Example 28)

### Step I

(3R)-3-deoxo-11-deoxy-12-desethenyl-12-[1-fluoro-1-(phenylsulf inyl)ethane-2-yl]-3-methoxy-14-O-methoxymethyl-11-oxo-4-epimut ilin

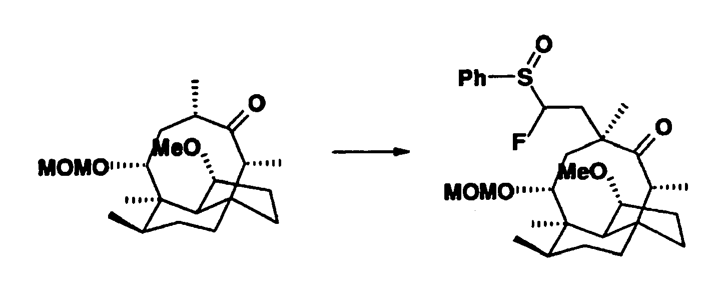

According to Step I of Example 2, the following compounds were used in the reaction: 1. 95 g (5.53 mmol) of the compound of Reference Example 3, 1.13 g (6.64 mmol) 1-fluoro-1-(phenylsulfinyl)ethene prepared by a process described in literature (J. Fluoro. Chem. 2002, 99.) and 13.3 mL (6.64 mmol) potassium bis(trimethylsilyl)amide (0.5 mol/L toluene solution). The resulting residue was purified by silica gel column chromatography (hexane:ethyl acetate = 2:1) to afford 2.37 g of the title compound as a yellow oil (82% yield).
MS (FAB) (m/z) : 461 (MH⁺-HOCH₂OCH₃).
HRMS (FAB) (m/z): Calcd. for C₂₇H_{3S}FO₃S (MH⁺-HOCH₂OCH₃) : 461.2526. Found, 461.2568.

### Step II

(3R)-3-deoxo-11-deoxy-12-desethenyl-12-(1-fluoroethene-2-yl)-3 -methoxy-14-O-methoxymethyl-11-oxo-4-epimutilin

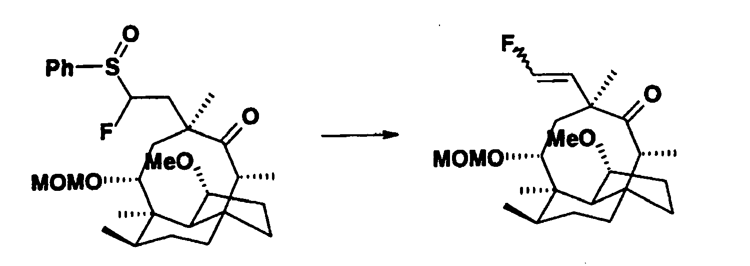

A solution of 2.37 g (4.53 mmol) of the compound of Step I in toluene (30 mL) was stirred for about 3 hours while heated to 150°C. After cooling, the solvent was evaporated under reduced pressure and the resulting residue was purified by column chromatography (hexane:ethyl acetate = 10:1) to afford 1.13 g of a mixture of E- and Z-forms of the title compound as a colorless oil (63% yield).
MS (FAB) (m/z): 397 (MH⁺).
HRMS (FAB) (m/z): Calcd. for C₂₃H₃₈FO₄ (MH⁺) : 397.2754. Found, 397.2726.

### Step III

(3R)-3-deoxo-11-deoxy-12-desethenyl-12-[(E)-1-fluoroethene-2-y 1]-3-methoxy-11-oxo-4-epimutilin and (3R)-3-deoxo-11-deoxy-12-desethenyl-12-[(Z)-1-fluoroethene-2-y 1]-3-methoxy-11-oxo-4-epimutilin

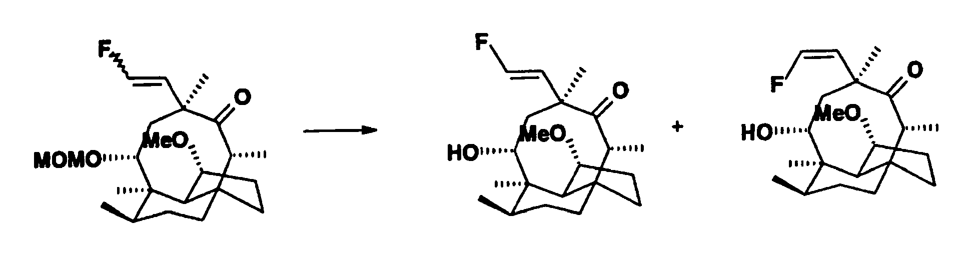

According to Reference Example 4, 1.13 g (2.85 mmol) of the compound of Step II and 542 mg (2.85 mmol) p-toluenesulfonic acid were used in the reaction. The resulting residue was purified by silica gel column chromatography (hexane:ethyl acetate = 2:1) to afford 612 mg of the former title compound as a colorless powder (61% yield) and 162 mg of the latter title compound as a colorless powder (16% yield).
E-form:
MS (FAB) (m/z): 335 (MH⁺-H₂O).
HRMS (FAB) (m/z): Calcd. for C₂₁H₃₂FO₂(MH⁺-H₂O) : 335.2386. Found, 335.2349.
Z-form:
MS (FAB) (m/z): 335 (MH⁺-H₂O).
HRMS (FAB) (m/z): Calcd. for C₂₁H₃₂FO₂(MH⁺-H₂O): 335.2386. Found, 335.2374.

### Step IV

(3R)-14-{(3R,4S)-1-azabicyclo[2.2.1]heptane-3-carbonyl}carbamo yl-3-deoxo-11-deoxy-12-desethenyl-12-[(E)-1-fluoroethene-2-yl] -3-methoxy-11-oxo-4-epimutilin

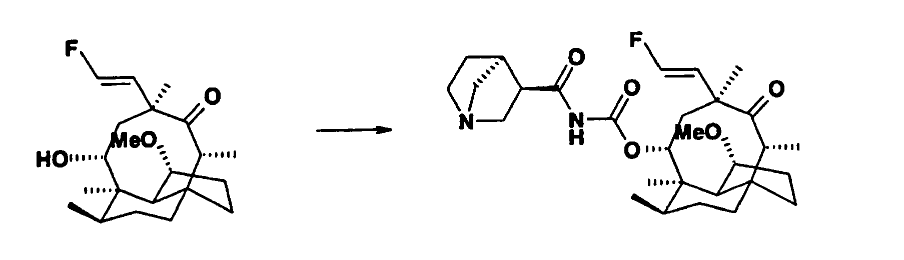

According to Step I of Example 1, the following compounds were used in the reaction: 500 mg (1.42 mmol) of the (E) -isomer, an acid chloride prepared from 378 mg (2.13 mmol) carboxylic acid, 532 mg (3.55 mmol) silver cyanide and 0.30 mL (2.13 mmol) triethylamine. The resulting residue was purified by silica gel column chromatography (NH, ethyl acetate, and then ethyl acetate:methanol = 20:1) to afford 276 mg of the title compound as a colorless powder (37% yield).
MS (FAB) (m/z): 519 (MH⁺).
HRMS (FAB) (m/z) : Calcd. for C₂₉H₄₄FN₂O₅ (MH⁺) : 519. 3234. Found, 519.3226.

### Step V

14-{(3R,4S)-1-azabicyclo[2.2.1]heptane-3-carbonyl}carbamoyl-12 -desethenyl-12-[(E)-1-fluoroethene-2-yl]mutilin

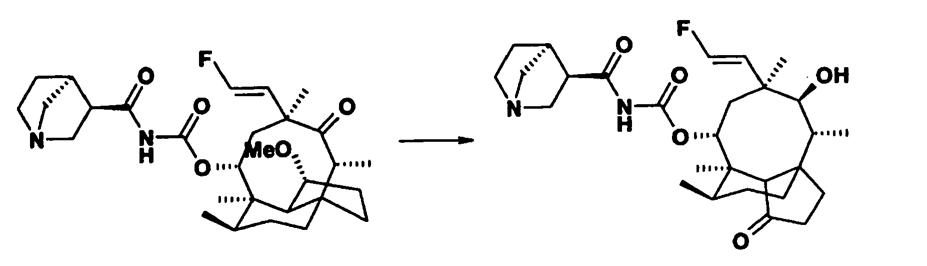

According to Step II of Example 1, 252 mg (0.49 mmol) of the compound of Step IV was used in the reaction. The resulting residue was purified by silica gel column chromatography (NH, ethyl acetate:methanol = 30:1) to afford 214 mg of the title compound as a colorless powder (87% yield).
MS (FAB) (m/z): 505 (MH⁺) .
HRMS (FAB) (m/z) : Calcd. for C₂₈H₄₂FN₂O₅ (MH⁺) : 505.3078. Found, 505.3101.

### (Example 29)

### Step I

(3R)-14-{(3R,4S)-1-azabicyclo[2.2.1]heptane-3-carbonyl}carbamo yl-3-deoxo-11-deoxy-12-desethenyl-12-[(Z)-1-fluoroethene-2-yl] -3-methoxy-11-oxo-4-epimutilin

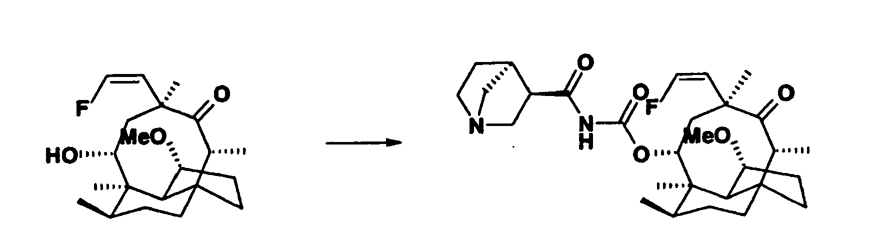

According to Step I of Example 1, the following compounds were used in the reaction: 150 mg (0.43 mmol) of the (Z)-isomer obtained in Step III of Example 35, an acid chloride prepared from 115 mg (0.65 mmol) carboxylic acid, 162 mg (1.08 mmol) silver cyanide and 91.0 µL (0.65 mmol) triethylamine. The resulting residue was purified by silica gel column chromatography (NH, ethyl acetate, and then ethyl acetate:methanol = 20:1) to afford 78.8 mg of the title compound as a colorless powder (35% yield).
MS (FAB) (m/z): 519.5 (MH⁺).
HRMS (FAB) (m/z) : Calcd. for C₂₉H₄₄FN₂O₅(MH⁺): 519.3234. Found, 519.3203.

### Step II

14-{(3R,4S)-1-azabicyclo[2.2.1]heptane-3-carbonyl}carbamoyl-12 -desethenyl-12-[(Z)-1-fluoroethene-2-yl]mutilin

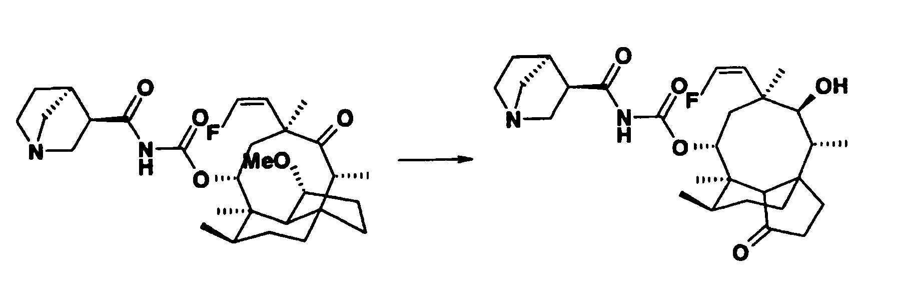

According to Step II of Example 1, 69.9 mg (0.13 mmol) of the compound of Step I was used in the reaction. The resulting residue was purified by silica gel column chromatography (NH, ethyl acetate:methanol = 30:1) to afford 56.6 mg of the title compound as a colorless powder (86% yield).
MS (FAB) (m/z): 505 (MH⁺) .
HRMS (FAB) (m/z) : Calcd. for C₂₈H₄₂FN₂O₅(MH⁺): 505.3078. Found, 505.3095.

### (Example 30)

### Step I

(3R)-12-cyclopentyl-3-deoxo-11-deoxy-12-desethenyl-3-methoxy-1 4-O-methoxymethyl-11-oxo-4-epimutilin

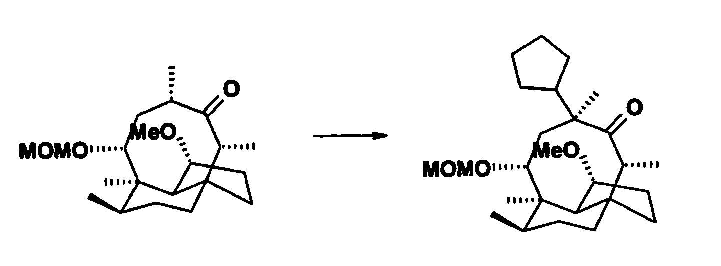

According to Step I of Example 2, 2.00 g (5.67 mmol) of the compound of Reference Example 3, 0.73 mL (6.81 mmol) cyclopentyl bromide and 13. 6 mL (6. 81 mmol) potassium bis (trimethylsilyl) amide (0. 5mol/L toluene solution) were used in the reaction. The resulting residue was purified by silica gel column chromatography (hexane:ethyl acetate = 8:1) to afford 767 mg of the title compound as a colorless oil (32% yield).
MS (EI) (m/z): 420 (M⁺).
HRMS (EI) (m/z): Calcd. for C₂₆H₄₄O₄ (M⁺) : 420.3240. Found, 420.3233.

### Step II

(3R)-12-cyclopentyl-3-deoxo-11-deoxy-12-desethenyl-3-methoxy-1 1-oxo-4-epimutilin

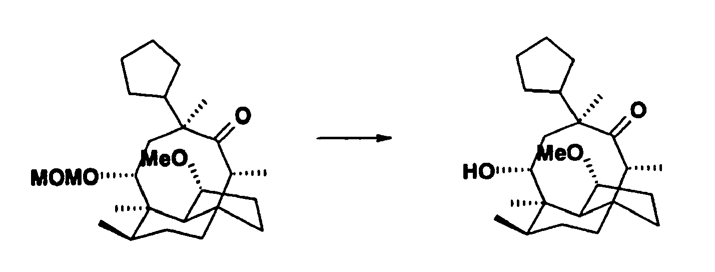

According to Reference Example 4, 767 mg (1.82 mmol) of the compound of Step I and 347 mg (1.82 mmol) p-toluenesulfonic acid were used in the reaction. The resulting residue was filtered by hexane-ethyl acetate to afford 554 mg of the title compound as a colorless powder (81% yield).
MS (FAB) (m/z): 377 (MH⁺).
HRMS (FAB) (m/z) : Calcd. for C₂₄H₄₁O₃ (MH⁺) : 377. 3056. Found, 377. 3063.

### Step III

(3R)-14-{(3R,4S)-1-azabicyclo[2.2.1]heptane-3-carbonyl}carbamo yl-12-cyclopentyl-3-deoxo-11-deoxy-12-desethenyl-3-methoxy-11-oxo-4-epimutilin

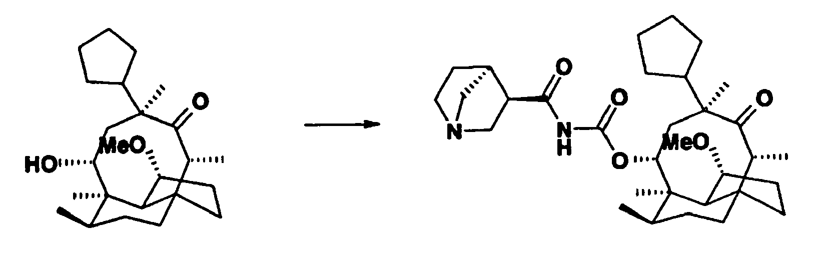

According to Step I of Example 1, the following compounds were used in the reaction: 300 mg (0.80 mmol) of the compound of Step II, an acid chloride prepared from 213 mg (1.20 mmol) carboxylic acid, 300 mg (2.00 mmol) silver cyanide and 0.17 mL (1.20 mmol) triethylamine. The resulting residue was purified by silica gel column chromatography (NH, ethyl acetate, and then ethyl acetate:methanol = 20:1) to afford 131 mg of the title compound as a colorless powder (30% yield).
MS (FAB) (m/z) : 543 (MH⁺) . HRMS (FAB) (m/z): Calcd. for C₃₂H₅₁N₂O₅(MH⁺) : 543.3798. Found, 543.3815.

### Step IV

14-{(3R,4S)-1-azabicyclo[2.2.1]heptane-3-carbonyl}carbamoyl-12 -cyclopentyl-12-desethenylmutilin

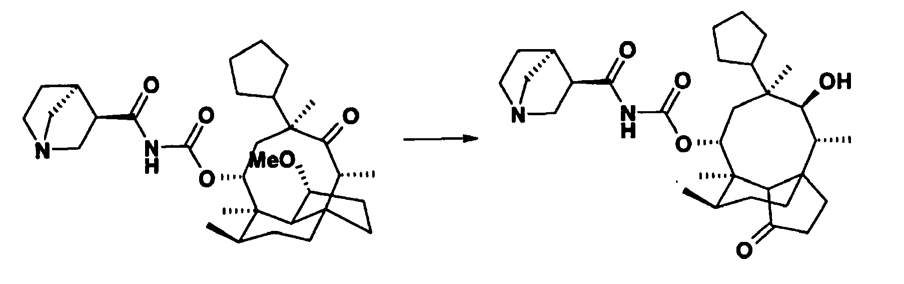

According to Step II of Example 1, 117 mg (0.22 mmol) of the compound of Step III was used in the reaction. The resulting residue was purified by silica gel column chromatography (NH, ethyl acetate:methanol = 30:1) to afford 60.4 mg of the title compound as a colorless powder (52% yield).
MS (FAB) (m/z): 529 (MH⁺).
HRMS (FAB) (m/z): Calcd. for C₃₁H₄₉N₂O₅(MH⁺) : 529.3641. Found, 529.3680.

### (Example 31)

### Step I

(3R)-12-(2-cyclohexene-1-yl)-3-deoxo-11-deoxy-12-desethenyl-3-methoxy-14-O-methoxymethyl-11-oxo-4-epimutilin

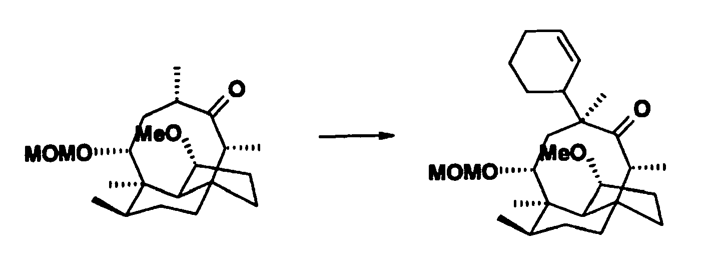

According to Step I of Example 2, 2.00 g (5.67 mmol) of the compound of Reference Example 3, 0.79 mL (6.81 mmol) 1-bromo-2-cyclohexene and 13.6 mL (6.81 mmol) potassium bis(trimethylsilyl)amide (0.5 mol/L toluene solution) were used in the reaction. The resulting residue was purified by silica gel column chromatography (hexane: ethyl acetate = 8:1) to afford 2.14 g of the title compound as a colorless oil (87% yield).
MS (EI) (m/z): 432 (M⁺) .
HRMS (EI) (m/z) : Calcd. for C₂₇H₄₄O₄ (MH⁺) : 432.3240. Found, 432.3206.

### Step II

(3R)-12-(2-cyclohexene-1-yl)-3-deoxo-11-deoxy-12-desethenyl-3-methoxy-11-oxo-4-epimutilin

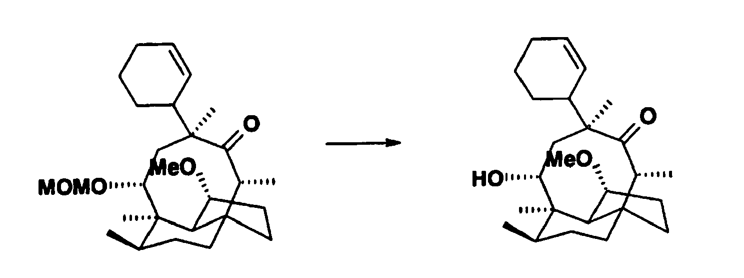

According to Reference Example 4, 2.14 g (4.95 mmol) of the
compound of Step I and 941 mg (4.95 mmol) p-toluenesulfonic acid were used in the reaction. The resulting residue was filtered by hexane-ethyl acetate to afford 1.05 g of the title compound as a colorless powder (55% yield).
MS (FAB) (m/z) : 389 (MH⁺).
HRMS (FAB) (m/z) : Calcd. for C₂₅H₄₁O₃(MH⁺) : 389.3056. Found, 389.3067.

### Step III

(3R)-14-{(3R,4S)-1-azabicyclo[2.2.1]heptane-3-carbonyl}carbamo yl-12-(2-cyclohexene-1-yl)-3-deoxo-11-deoxy-12-desethenyl-3-me thoxy-11-oxo-4-epimutilin

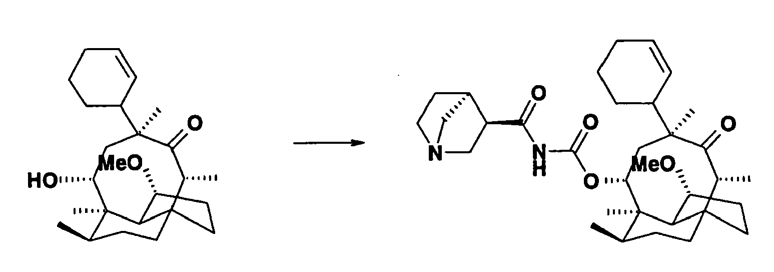

According to Step I of Example 1, the following compounds were
used in the reaction: 300 mg (0.77 mmol) of the compound of Step II, an acid chloride prepared from 206 mg (1.16 mmol) carboxylic acid, 289 mg (1.93 mmol) silver cyanide and 0.16 mL (1.16 mmol) triethylamine were used in the reaction. The resulting residue was purified by silica gel column chromatography (NH, ethyl acetate, and then ethyl acetate:methanol = 20:1) to afford 134 mg of the title compound as a colorless powder (32% yield).
MS (FAB) (m/z): 555 (MH⁺).
HRMS (FAB) (m/z): Calcd. for C₃₃H₅₁N₂O₅(MH⁺) : 555.3798. Found, 555.3823.

### Step IV

14-{(3R,4S)-1-azabicyclo[2.2.1]heptane-3-carbonyl}carbamoyl-12 -(2-cyclohexene-1-yl)-12-desethenylmutilin

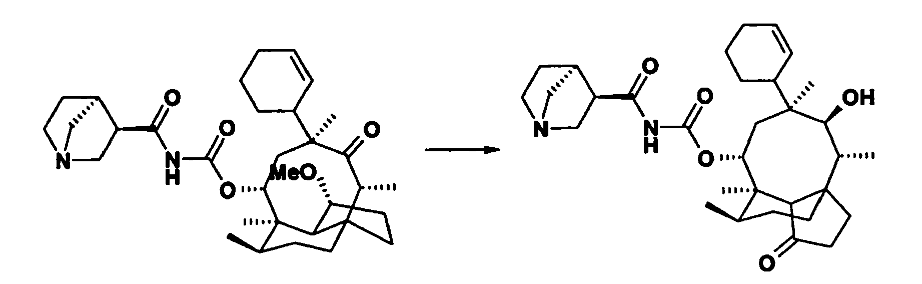

According to Step II of Example 1, 126 mg (0.23 mmol) of the compound of Step III was used in the reaction. The resulting residue was purified by silica gel column chromatography (NH, ethyl acetate:methanol = 30:1) to afford 72.8 mg of the title compound as a colorless powder (59% yield).
MS (FAB) (m/z): 541 (MH⁺).
HRMS (FAB) (m/z): Calcd. for C₃₂H₄₉N₂O₅(MH⁺) : 541.3641. Found, 541.3660.

### (Example 32)

### Step I

(3R)-3-deoxo-11-deoxy-12-desethenyl-3-methoxy-14-O-methoxymeth yl-12-(3-methyl-1-propene-3-yl)-11-oxo-4-epimutilin

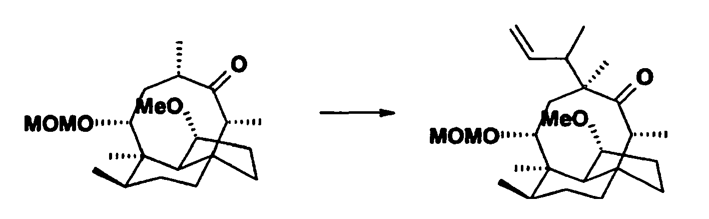

According to Step I of Example 2, 1.50 g (4.26 mmol) of the compound of Reference Example 3, 0.51 mL (5.11 mmol) 2-chloro-3-butene and 10.2 mL (5.11 mmol) potassium bis(trimethylsilyl)amide (0.5 mol/L toluene solution) were used in the reaction. The resulting residue was purified by silica gel column chromatography (hexane:ethyl acetate = 8:1) to afford 1.23 g of the title compound as a colorless oil (71% yield).
MS (FAB) (m/z): 407 (MH⁺) .
HRMS (FAB) (m/z) : Calcd. for C₂₅H₄₃O₄(MH⁺) : 407.3161. Found, 407.3160.

### Step II

(3R)-3-deoxo-11-deoxy-12-desethenyl-3-methoxy-12-(3-methyl-1-p ropene-3-yl)-11-oxo-4-epimutilin

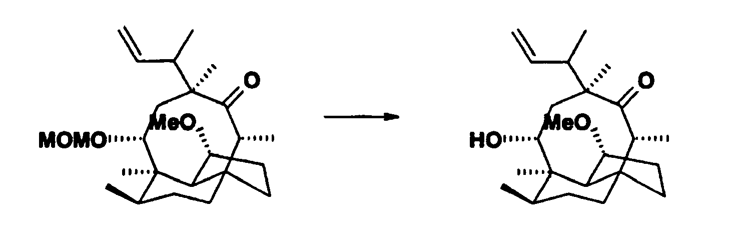

According to Reference Example 4, 1.23 g (3.03 mmol) of the
compound of Step I and 575 mg (3.03 mmol) p-toluenesulfonic acid were used in the reaction. The resulting residue was purified by silica gel column chromatography (hexane:ethyl acetate = 4:1) to afford 822 mg of the title compound (1:1 mixture of diastereomers) as a colorless powder (81% yield).
MS (FAB) (m/z): 363 (MH⁺).
HRMS (FAB) (m/z) : Calcd. for C₂₃H₃₉O₃ (MH⁺) : 363.2899. Found, 363.2876.

### Step III

(3R)-14-{(3R,4S)-1-azabicyclo[2.2.1]heptane-3-carbonyl}carbamo yl-3-deoxo-11-deoxy-12-desethenyl-3-methoxy-12-(3-methyl-1-pro pene-3-yl)-11-oxo-4-epimutilin

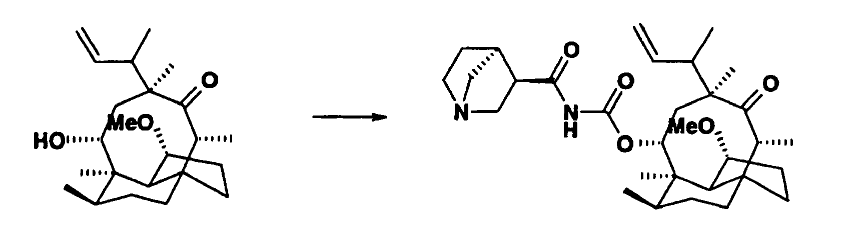

According to Step I of Example 1, the following compounds were used in the reaction: 300 mg (0.83 mmol) of the compound of Step II, an acid chloride prepared from 222 mg (1.25 mmol) carboxylic acid, 312 mg (2.08 mmol) silver cyanide and 0.17 mL (1.25 mmol) triethylamine. The resulting residue was purified by silica gel column chromatography (NH, ethyl acetate, and then ethyl acetate:methanol = 20:1) to afford 154 mg of the title compound as a colorless powder (32% yield).
MS (FAB) (m/z): 529 (MH⁺).
HRMS (FAB) (m/z): Calcd. for C₃₁H₄₉N₂O₅(MH⁺) : 529.3641. Found, 529.3628.

### Step IV

14-{(3R,4S)-1-azabicyclo[2.2.1]heptane-3-carbonyl}carbamoyl-12 -desethenyl-12-(3-methyl-1-propene-3-yl)mutilin

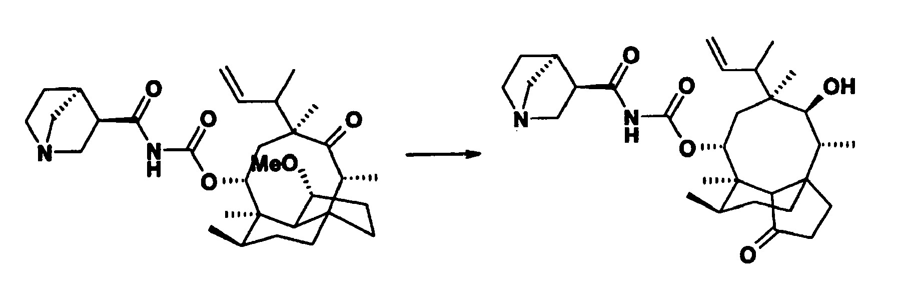

According to Step II of Example 1, 138 mg (0.26 mmol) of the compound of Step III was used in the reaction. The resulting residue was purified by silica gel column chromatography (NH, ethyl acetate:methanol = 30:1) to afford 103 mg of the title compound as a colorless powder (77% yield).
MS (FAB) (m/z): 515 (MH⁺).
HRMS (FAB) (m/z): Calcd. for C₃₀H₄₇N₂O₅(MH⁺) : 515.3485. Found, 515.3513.

### (Example 33)

### Step I

(3R)-3-deoxo-11-deoxy-12-desethenyl-3-methoxy-14-O-methoxymeth yl-12-(3-methyl-1-propyne-3-yl)-11-oxo-4-epimutilin

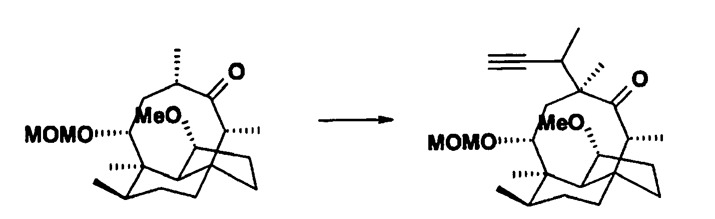

According to Step I of Example 2, 1.00 g (2.84 mmol) of the compound of Reference Example 3, 0.31 mL (3.40 mmol) 3-chloro-1-butene and 6.81 mL (3.40 mmol) potassium bis(trimethylsilyl)amide (0.5 mol/L toluene solution) were used in the reaction. The resulting residue was purified by silica gel column chromatography (hexane:ethyl acetate = 10:1) to afford 538 mg of the title compound as a yellow oil (47% yield).
MS (EI) (m/z): 404 (M⁺).
HRMS (EI) (m/z): Calcd. for C₂₅H₄₀O₄(M⁺) : 404.2927. Found, 404.2925.

### Step II

(3R)-3-deoxo-11-deoxy-12-desethenyl-3-methoxy-12-(3-methyl-1-p ropyne-3-yl)-11-oxo-4-epimutilin

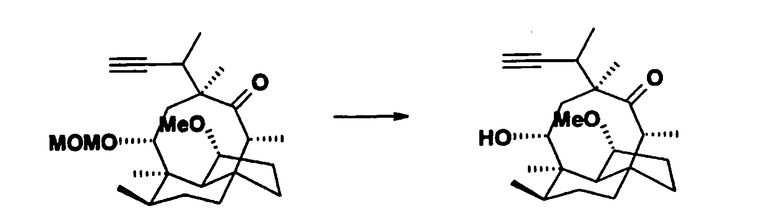

According to Reference Example 4, 538 mg (1.33 mmol) of the compound of Step I and 253 mg (1.33 mmol) p-toluenesulfonic acid were used in the reaction. The resulting residue was purified by silica gel column chromatography (hexane:ethyl acetate = 4:1) to give 126 mg of one type of the title compound with relatively low polarity (26% yield) and 107 mg of another type of the title compound with relatively high polarity (81% yield), each as a colorless powder. Low polarity title compound:
MS (FAB) (m/z) : 361 (MH⁺) .
HRMS (FAB) (m/z) : Calcd. for C₂₃H₃₇O₃ (MH⁺) : 361.2743. Found, 361.2760. High polarity title compound:
MS (FAB) (m/z): 361 (MH⁺).
HRMS (FAB (m/z) : Calcd. for C₂₃H₃₇O₃(MH⁺): 361.2743. Found, 361.2720.

### Step III

(3R)-14-{(3R,4S)-1-azabicyclo[2.2.1]heptane-3-carbonyl}carbamo yl-3-deoxo-11-deoxy-12-desethenyl-3-methoxy-12-(3-methyl-1-pro pyne-3-yl)-11-oxo-4-epimutilin

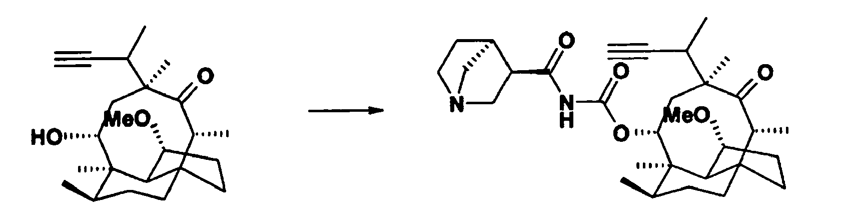

According to Step I of Example 1, the following compounds were used in the reaction: 126 mg (0. 35 mmol) of the low polarity compound obtained in Step II, an acid chloride prepared from 94.1 mg (0.53 mmol) carboxylic acid, 132 mg (0.88 mmol) silver cyanide and 73.9 µL (0.53 mmol) triethylamine. The resulting residue was purified by silica gel column chromatography (NH, ethyl acetate, and then ethyl acetate:methanol = 20: 1) to afford 44.2 mg of the title compound as a colorless powder (24% yield).
MS (FAB) (m/z): 527 (MH⁺) .
HRMS (FAB) (m/z): Calcd. for C₃₁H₄₇N₂O₅ (MH⁺) : 527.3485. Found, 527.3470.

### Step IV

14-{(3R,4S)-1-azabicyclo[2.2.1]heptane-3-carbonyl}carbamoyl-12 -desethenyl-12-(3-methyl-1-propyne-3-yl)mutilin

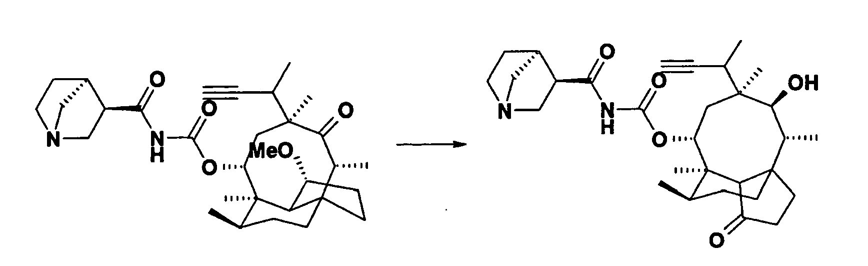

According to Step II of Example 1, 40.1 mg (76.1 µmol) of the compound of Step III was used in the reaction. The resulting residue was purified by silica gel column chromatography (NH, ethyl acetate:methanol = 30:1) to afford 22.6 mg of the title compound as a colorless powder (58% yield).
MS (FAB) (m/z): 513 (MH⁺).
HRMS (FAB) (m/z): Calcd. for C₃₀H₄₅N₂O₅ (MH⁺) : 513.3328. Found, 513.3318.

### (Example 34)

### Step I

(3R)-14-{(3R,4S)-1-azabicyclo[2.2.1]heptane-3-carbonyl}carbamo yl-3-deoxo-11-deoxy-12-desethenyl-3-methoxy-12-(3-methyl-1-pro pyne-3-yl)-11-oxo-4-epimutilin

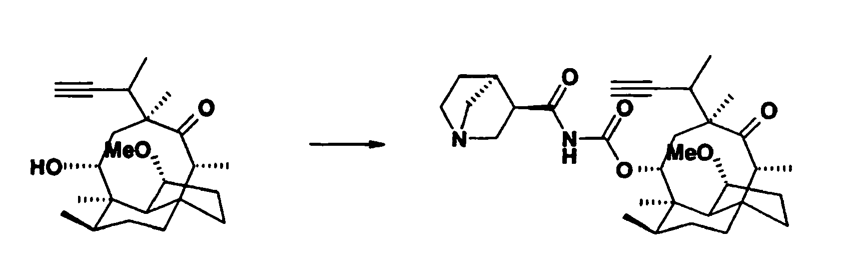

According to Step I of Example 1, the following compounds were
used in the reaction: 107 mg (0. 30 mmol) of the high polarity compound obtained in Step II of Example 33, an acid chloride prepared from 79.9mg (0.45 mmol) carboxylic acid, 112 mg (0.75 mmol) silver cyanide and 62.7 µL (0.45 mmol) triethylamine. The resulting residue was purified by silica gel column chromatography (NH, ethyl acetate, and then ethyl acetate:methanol = 20:1) to afford 31.7 mg of the title compound as a colorless powder (20% yield).
MS (FAB) (m/z): 527 (MH⁺).
HRMS (FAB) (m/z): Calcd. for C₃₁H₄₇N₂O₅(MH⁺) : 527.3485. Found, 527.3497.

### Step II

14-{(3R,4S)-1-azabicyclo[2.2.1]heptane-3-carbonyl}carbamoyl-12 -desethenyl-12-(3-methyl-1-propyne-3-yl)mutilin

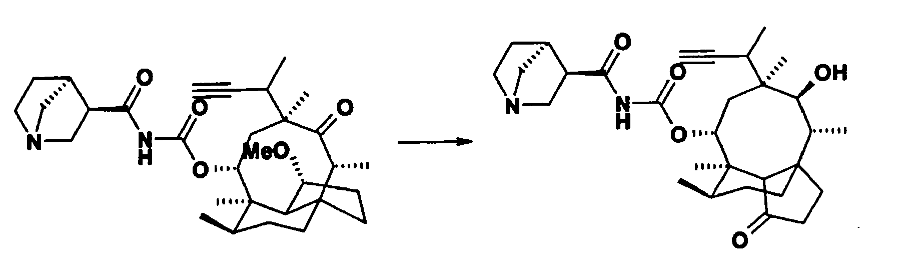

According to Step II of Example 1, 27. 7 mg (52.6 µmol) of the compound of Step I was used in the reaction. The resulting residue was purified by silica gel column chromatography (NH, ethyl acetate:methanol = 20:1) to afford 16.4 mg of the title compound as a colorless powder (61% yield).
MS (FAB) (m/z): 513 (MH⁺) .
HRMS (FAB) (m/z) : Calcd. for C₃₀H₄₅N₂O₅ (MH⁺) : 513.3328. Found, 513.3358.

### (Example 35)

### Step I

(3R)-12-cyclopropylmethyl-3-deoxo-11-deoxy-12-desethenyl-3-met hoxy-14-O-methoxymethyl-11-oxo-4-epimutilin

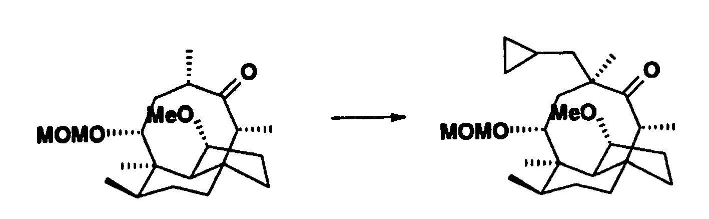

According to Step I of Example 2, 2.00 g (5.67 mmol) of the compound of Reference Example 3, 0.66 mL (6.80 mmol) cyclopropylmethyl bromide and 13.6 mL (6.80 mmol) potassium bis(trimethylsilyl)amide (0.5 mol/L toluene solution) were used in the reaction. The resulting residue was purified by silica gel column chromatography (hexane:ethyl acetate = 8:1) to afford 2.17 g of the title compound as a colorless oil (94% yield).
MS (FAB) (m/z) : 407 (MH⁺).
HRMS (FAB) (m/z): Calcd. for C₂₅H₄₃O₄ (MH⁺) : 407. 3161. Found, 407. 3114.

### Step II

(3R)-12-cyclopropylmethyl-3-deoxo-11-deoxy-12-desethenyl-3-met hoxy-11-oxo-4-epimutilin

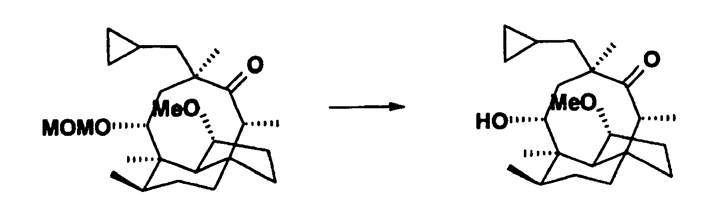

According to Reference Example 4, 2.00 g (4.92 mmol) of the compound of Step I and 936 mg (4.92 mmol) p-toluenesulfonic acid were used in the reaction. The resulting residue was purified by silica gel column chromatography (hexane:ethyl acetate = 8:1) to afford 1. 55 g of the title compound as a colorless powder (87% yield). MS (FAB) (m/z): 345 (MH⁺-H₂O).
HRMS (FAB) (m/z): Calcd. for C₂₃H₃₇O₂(MH⁺-H₂O): 345.2794. Found, 345.2794.

### Step III

(3R)-14-{(3R,4S)-1-azabicyclo[2.2.1]heptane-3-carbonyl}carbamo yl-12-cyclopropylmethyl-3-deoxo-11-deoxy-12-desethenyl-3-metho xy-11-oxo-4-epimutilin

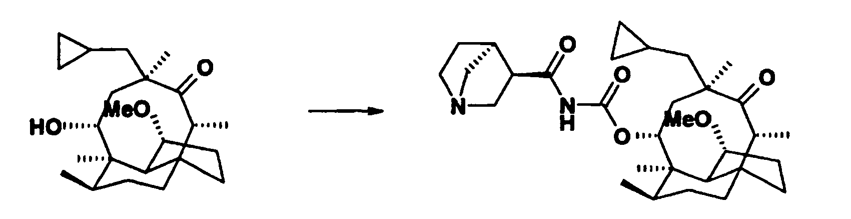

According to Step I of Example 1, the following compounds were used in the reaction: 300 mg (0.83 mmol) of the compound of Step II, an acid chloride prepared from 222 mg (1.25 mmol) carboxylic acid, 312 mg (2.08 mmol) silver cyanide and 0.17 mL (1.25 mmol) triethylamine. The resulting residue was purified by silica gel column chromatography (NH, ethyl acetate, and then ethyl acetate:methanol = 15:1) to afford 153 mg of the title compound as a colorless powder (35% yield).
MS (FAB) (m/z): 529 (MH⁺).
HRMS (FAB) (m/z): Calcd. for C₃₁H₄₉N₂O₅ (MH⁺) : 529.3641. Found, 529.3605.

### (Example 36)

### Step I

(3R)-3-deoxo-11-deoxy-12-desethenyl-12-formylmethyl-3-methoxy-14-0-methoxymethyl-11-oxo-4-epimutilin

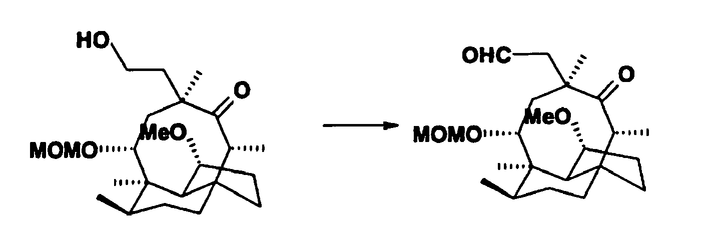

0.22 g (0.63 mmol) tetrapropylammonium perruthenate (VII), 2.22 g (18.9 mmol) 4-methylmorpholine-N-oxide and 6.30 g molecular sieves 4A were added to a solution of 5.00 g (12.6 mmol) (3R)-3-deoxo-11-deoxy-12-desethenyl-12-(1-hydroxyethane-2-yl)-3-methoxy-14-O-methoxymethyl-11-oxo-4-epimutilin in methylene chloride (25 mL). The mixture was stirred at room temperature for 1.5 hours. Subsequently, the reaction mixture was filtered through Celite and the residue was washed with ethyl acetate. The filtrate was evaporated under reduced pressure. The residue was then diluted with ethyl acetate (150 mL) and was washed sequentially with a 10% aqueous Na₂S₂O₃ solution and saturated brine. The organic layer was dried over anhydrous magnesium sulfate, filtered and concentrated. The resulting residue was purified by column chromatography and silica gel column chromatography (hexane:ethyl acetate = 4:1) to afford 3.20 g of the title compound as a colorless powder (64% yield). MS (FAB) (m/z): 395 (MH⁺).
HRMS (FAB) (m/z) : Calcd. for C₂₃H₃₉O₅(MH⁺) : 395.2797. Found, 395.2797.

### Step II

(3R)-3-deoxo-11-deoxy-12-desethenyl-3-methoxy-14-O-methoxymeth yl-11-oxo-12-(1,1,1-trifluoro-2-propene-3-yl)-4-epimutilin

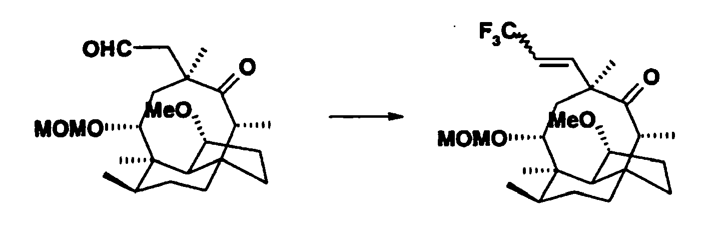

According to a process described in literature (J. Org. Chem. 2002, 67, 7162-7164.), 0.45 mL (3.04 mmol) trifluoromethyltrimethylsilane and a catalytic amount of TBAF (1 mol/L tetrahydrofuran solution) were sequentially added to a solution of 1.00 g (2.53 mmol) of the compound of Step I in tetrahydrofuran (20 mL), at 0°C in an argon atmosphere. The reaction mixture was allowed to warm to room temperature and the reaction was allowed to proceed for 36 hours. Subsequently, a saturated aqueous ammonium chloride solution and 7.60 mL (7.60 mmol) TBAF (1 mol/L tetrahydrofuran solution) were sequentially added and the mixture was stirred for 1 hour. The reaction mixture was then poured into diluted aqueous citric acid. The solvent was evaporated under reduced pressure and the residue was extracted with ethyl acetate (20 mL x 3) . The organic layers were combined, washed with saturated brine (20 mL) and dried over anhydrous magnesium sulfate. The dried product was filtered and the solvent was removed. The resulting residue was purified by column chromatography (hexane:ethyl acetate = 2:1) to give 1.07 g of a yellow oil (91% yield).
MS (FAB) (m/z): 465 (MH⁺).
HRMS (FAB) (m/z): Calcd. for C₂₄H₄₀F₃O₅(MH⁺): 465.2828. Found, 465.2852.

1.07g (2. 30 mmol) of this compound was dissolved in methylene chloride (30mL). To this solution, 0.96mL (6.91 mmol) triethylamine and 0.27 mL (3.45 mmol) methanesulfonylchloride were sequentially added dropwise at 0°C in an argon atmosphere. The mixture was allowed to warm to room temperature and the reaction was allowed to proceed for 60 hours. The reaction mixture was then poured into diluted aqueous citric acid. The solvent was evaporated under reduced pressure and the residue was extracted with ethyl acetate (10 mL x 3) . The organic layers were combined, washed with saturated brine (10mL) and dried over anhydrous magnesium sulfate. Thedriedproduct was filtered and the solvent was removed. The resulting residue was purified by column chromatography (hexane:ethyl acetate = 4:1) to give 1.18 g of a colorless oil (94% yield).
MS (FAB) (m/z) : 543 (MH⁺) .
HRMS (FAB) (m/z): Calcd. for C₂₅H₄₂F₃O₇S (MH⁺) : 543.2603. Found, 543.2644.

To 1.18 g (2.17 mmol) of this compound, 1.03 mL (6.91 mmol) 1,8-diazabicyclo[5.4.0]unde-7-cene was added at room temperature and the reaction was allowed to proceed for 10 hours at 150°C.
Subsequently, the reaction mixture was poured into diluted aqueous citric acid and extracted with ethyl acetate (20 mL x 3). The organic layers were combined, washed with saturated brine (20 mL) and dried over anhydrous magnesium sulfate. The dried product was filtered and the solvent was removed. The resulting residue was purified by column chromatography (hexane:ethyl acetate = 4:1) to give 211 mg of a colorless oil (22% yield).
MS (FAB) (m/z): 447 (MH⁺) .
HRMS (FAB) (m/z): Calcd. for C₂₉H₃₈F₃O₄ (MH⁺) : 447.2722. Found, 447.2687.

### Step III

(3R)-3-deoxo-11-deoxy-12-desethenyl-3-methoxy-11-oxo-12-(1,1,1 -trifluoro-2-propene-3-yl)-4-epimutilin

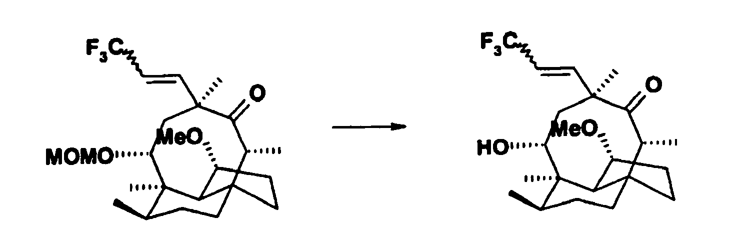

According to Reference Example 4, 211 mg (0.47 mmol) of the
compound of Step II and 89.9 mg (0.47 mmol) p-toluenesulfonic acid were used in the reaction. The resulting residue was purified by silica gel column chromatography (hexane:ethyl acetate = 4:1) to afford 101 mg of the title compound as a colorless powder (53% yield). MS (FAB) (m/z) : 385 (MH⁺-H₂O).
HRMS (FAB) (m/z): Calcd. for C₂₂H₃₂F₃O₃(MH⁺-H₂O) : 385.2354. Found, 385.2390.

### Step IV

(3R)-14-{(3R,4S)-1-azabicyclo[2.2.1]heptane-3-carbonyl}carbamo yl-3-deoxo-11-deoxy-12-desethenyl-3-methoxy-11-oxo-12-(1,1,1-t rifluoro-2-propene-3-yl)-4-epimutilin

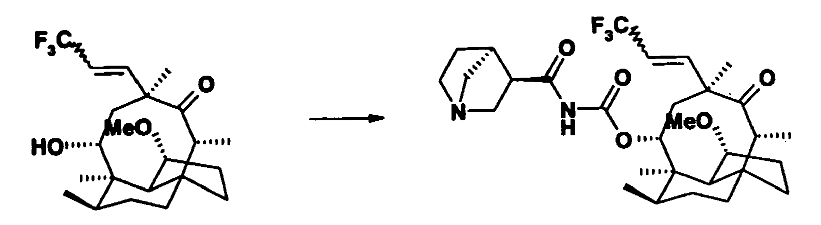

According to Step I of Example 1, the following compounds were used in the reaction: 101 mg (0.25 mmol) of the compound of Step III, an acid chloride prepared from 67.5 mg (0.38 mmol) carboxylic acid, 94.4 mg (0.63 mmol) silver cyanide and 53.0 µL (0.38 mmol) triethylamine. The resulting residue was purified by silica gel column chromatography (NH, ethyl acetate, and then ethyl acetate:methanol = 15:1) to afford 55.5 mg of the title compound as a colorless powder (39% yield).
MS (FAB) (m/z): 569 (MH⁺) .
HRMS (FAB) (m/z) : Calcd. for C₃₀H₄₄F₃N₂O₅ (MH⁺) : 569.3202. Found, 569.3225.

### Step V

14-{(3R,4S)-1-azabicyclo[2.2.1]heptane-3-carbonyl}carbamoyl-12 -desethenyl-12-(1,1,1-trifluoro-2-propene-3-yl)mutilin

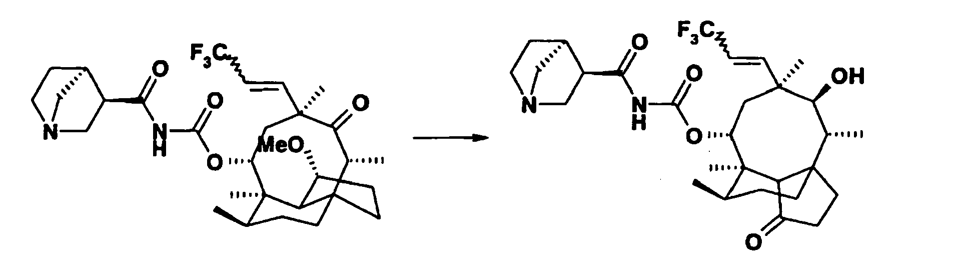

According to Step II of Example 1, 47.4 mg (83.4 µmol) of the compound of Step IV was used in the reaction. The resulting residue was purified by silica gel column chromatography (NH, ethyl acetate:methanol = 30:1) to afford 37.1 mg of the title compound as a colorless powder (80% yield).
MS (FAB) (m/z): 555 (MH⁺).
HRMS (FAB) (m/z): Calcd. for C₂₉H₄₂F₃N₂O₅ (MH⁺) : 555.3046. Found, 555.3033.

### (Example 37)

### Step I

(3R)-3-deoxo-11-deoxy-12-desethenyl-12-(1-formylethane-2-yl)-3 -methoxy-14-O-methoxymethyl-11-oxo-4-epimutilin

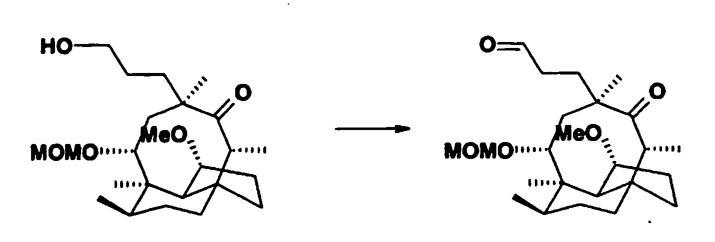

According to Step I of Example 36, the following compounds were used in the reaction: 4.13 g (10.1 mmol) (3R)-3-deoxo-11-deoxy-12-desethenyl-12-(1-hydroxypropane-3-yl) -3-methoxy-14-O-methoxymethyl-11-oxo-4-epimutilin obtained in Step I of Example 27, 179 mg (0.51 mmol) tetrapropylammonium perruthenate (VII), 1.78 g (15.2 mmol) 4-methylmorpholine-N-oxide and 5.05 g molecular sieves 4A. The resulting residue was purified by silica gel column chromatography (hexane:ethyl acetate = 3:1) to afford 2. 63 g of the title compound as a colorless oil (64% yield).
MS (EI) (m/z): 408 (M⁺).
HRMS (EI) (m/z) : Calcd. for C₂₄H₄₀O₅(M⁺) : 408.2876. Found, 408.2901.

### Step II

(3R)-12-(1-butyne-4-yl)-3-deoxo-11-deoxy-12-desethenyl-3-metho xy-14-O-methoxymethyl-11-oxo-4-epimutilin

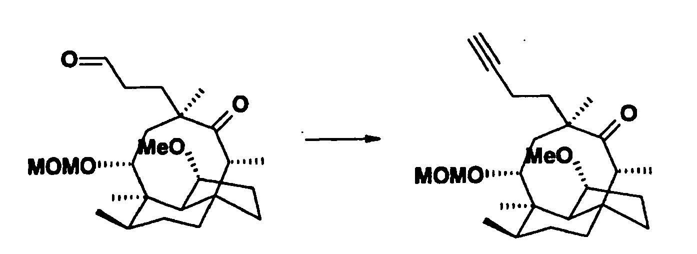

According to a process described in literature (Synthesis 2004, 59-62.), 2.50 g (6.12 mmol) of the compound of Step I, 1.90 g (76% purity, 7.34 mmol) p-toluenesulfonic acid azide, 2.54 g (18.4 mmol) potassium carbonate and 1.01 mL (7.34 mmol) dimethyl 2-oxopropylphosphonate were used in the reaction. The resulting residue was purified by silica gel column chromatography (hexane:ethyl acetate = 8:1) to afford 1.05 g of the title compound as a pale yellow oil (42% yield).
MS (FAB) (m/z): 343 (MH⁺-HOCH₂OCH₃) .
HRMS (FAB) (m/z) : Calcd. for C₂₃H₃₅O₂ (MH⁺-HOCH₂OCH₃) : 343.2637. Found, 343.2668.

### Step III

(3R)-12-(1-butyne-4-yl)-3-deoxo-11-deoxy-12-desethenyl-3-metho xy-11-oxo-4-epimutilin

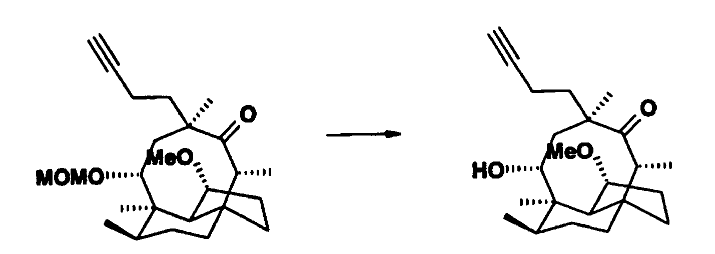

According to Reference Example 4, 950 mg (2.35 mmol) of the
compound of Step II and 447 mg (2.35 mmol) p-toluenesulfonic acid were used in the reaction. The resulting residue was purified by silica gel column chromatography (hexane:ethyl acetate = 6:1) to afford 571 mg of the title compound as a colorless oil (67% yield) . MS (FAB) (m/z): 361 (MH⁺) .
HRMS (FAB) (m/z) : Calcd. for C₂₃H₃₇O₃ (MH⁺) : 361.2743. Found, 361.2743.

### Step IV

(3R)-14-{(3R,4S)-1-azabicyclo[2.2.1]heptane-3-carbonyl}carbamo yl-12-(1-butyne-4-yl)-3-deoxo-11-deoxy-12-desethenyl-3-methoxy -11-oxo-4-epimutilin

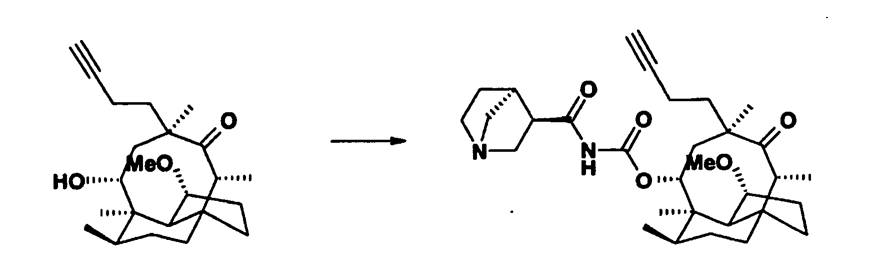

According to Step I of Example 1, the following compounds were used in the reaction: 300 mg (0.83 mmol) of the compound of Step III, an acid chloride prepared from 222 mg (1.25 mmol) carboxylic acid, 312 mg (2.08 mmol) silver cyanide and 0.17 mL (1.25 mmol) triethylamine. The resulting residue was purified by silica gel column chromatography (NH, ethyl acetate, and then ethyl acetate:methanol = 15:1) to afford 60.8 mg of the title compound as a colorless powder (14% yield).
MS (FAB) (m/z): 527 (MH⁺).
HRMS (FAB) (m/z): Calcd. for C₃₁H₄₇N₂O₅(MH⁺): 527.3485. Found, 527.3498.

### Step V

14-{(3R,4S)-1-azabicyclo[2.2.1]heptane-3-carbonyl}carbamoyl-12 -(1-butyne-4-yl)-12-desethenylmutilin

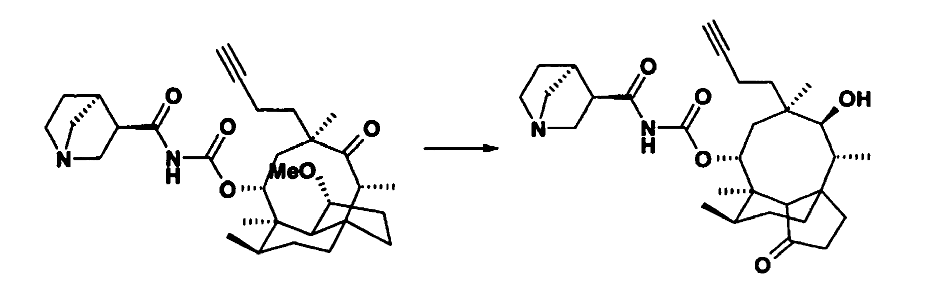

According to Step II of Example 1, 51.3 mg (97.4 µmol) of the compound of Step IV was used in the reaction. The resulting residue was purified by silica gel column chromatography (NH, ethyl acetate:methanol = 30:1) to afford 36.9 mg of the title compound as a colorless powder (74% yield).
MS (FAB) (m/z): 513 (MH⁺) .
HRMS (FAB) (m/z): Calcd. for C₃₀H₄₅N₂O₅ (MH⁺) : 513.3328. Found, 513.3287.

### (Example 38)

12-desethenyl-12-formyl-11-O-methoxymethylmutilin

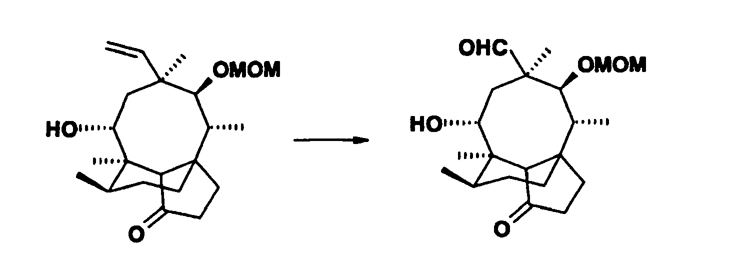

A catalytic amount of osmium tetroxide (5% t-butanol solution) and an aqueous solution (5 mL) of 352 mg (1. 65 mmol) sodium periodate were added to a solution of 300 mg (0.82 mmol) of the compound of Reference Example 12 in tetrahydrofuran (5 mL). The mixture was stirred at roomtemperature for 24 hours. Subsequently, the reaction mixture was filtered through Celite and the residue was washed with ethyl acetate. The filtrate was evaporated under reduced pressure, followed by addition of diluted aqueous citric acid and extraction with ethyl acetate (10 mL x 3). The organic layers were combined, washed with saturated brine (5 mL) and dried over anhydrous magnesium sulfate. The dried product was filtered and the solvent was removed. The resulting residue was purified by silica gel column chromatography (hexane:ethylacetate = 4:1, followed by hexane : ethyl acetate = 1:4) to afford 169 mg of the title compound as a colorless powder (56% yield).
MS (CI) (m/z): 367 (MH⁺).
HRMS (CI) (m/z) : Calcd. for C₂₁H₃₅O₅(MH⁺) : 367.2484. Found, 367.2500.

### (Example 39)

14-acetoxy-12-desethenyl-12-formyl-11-O-methoxymethylmutilin

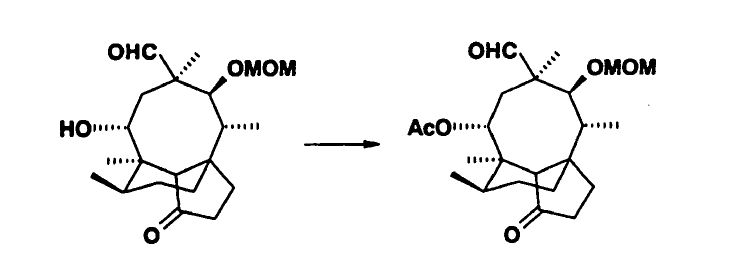

3.86mL (41.0 mmol) of acetic anhydride, 6. 62 mL (41.0 mmol)
pyridine and 4 60 mg (4.09 mmol) 4- (dimethylamino) pyridine were added to 1.50 g (4.09 mmol) of the compound of Example 38. The reaction mixture was stirred at room temperature for 72 hours, followed by addition of diluted aqueous citric acid and extraction with ethyl acetate (30 mL x 3). The organic layers were combined, washed with saturated brine (30 mL) and dried over anhydrous magnesium sulfate. The dried product was filtered and the solvent was removed. The resulting residue was purified by silica gel column chromatography (hexane:ethyl acetate = 2:1) to afford 939 mg of the title compound as a yellow powder (56% yield).
MS (FAB) (m/z): 409 (MH⁺) .
Rf = 0.20 (hexane:ethyl acetate = 2:1)

### (Example 40)

### Step I

12-desethenyl-12-methoxycarbonyl-11-O-methoxymethylmutilin

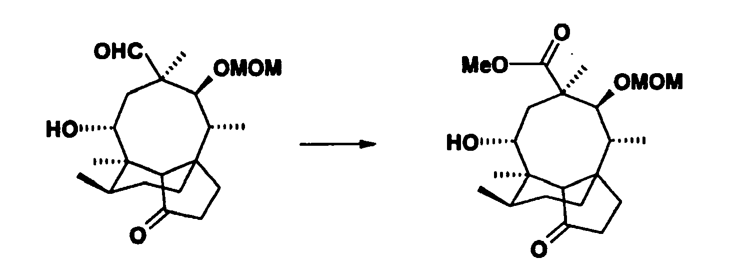

1.01 g (11.2 mmol) of sodium chlorate and 1.75 g sodium dihydrogen phosphate were added to a solution of 1. 37 g (3.74 mmol) of the compound of Example 38 dissolved in a mixture of tetrahydrofuran and water (15 mL). The reaction mixture was stirred at room temperature for 9 hours. Subsequently, a diluted aqueous sodium hydroxide solution was added and the mixture was extracted with ethyl acetate (10 mL x 3). Diluted aqueous citric acid was added to the aqueous layer and the layer was extracted with ethyl acetate (10 mL x 3). The acidic extracts were combined and the organic layer was washed with saturated brine (10 mL) and dried over anhydrous magnesium sulfate. Filtration of the dried product followed by removal of the solvent resulted in 1.16 g of a crude carboxylic acid derivative as a colorless powder (81% yield).
MS (CI) (m/z): 383 (MH⁺) .
HRMS (CI) (m/z): Calcd. for C₂₁H₃₅O₆(MH⁺): 383.2434. Found, 383.2414.

100 mg (0.26 mmol) of the carboxylic acid derivative was dissolved in acetonitrile (2 mL). To this solution, 49.5 µL (0.52 mmol) dimethyl sulfate and 145 mg (1.05 mmol) potassium carbonate were added and the mixture was refluxed for 7 hours. Subsequently, the reaction mixture was filtered through Celite and the residue was washed with ethyl acetate. The filtrate was evaporated under reduced pressure. Water was then added to the resulting residue and the mixture was extracted with ethyl acetate (3 mL x 3). The organic layers were combined, washed with saturated brine (3 mL) and dried over anhydrous magnesium sulfate. The dried product was filtered and the solvent was removed. The resulting residue was purified by silica gel column chromatography (hexane: ethyl acetate = 1:1) to afford 89.3 mg of the title compound as a colorless oil (86% yield).
MS (FAB) (m/z): 397 (MH⁺).
HRMS (FAB) (m/z) : Calcd. for C₂₂H₃₇O₆ (MH⁺) : 397.2590. Found, 397.2598.

### Step II

(3R)-14-{(3R,4S)-1-azabicyclo[2.2.1]heptane-3-carbonyl}carbamo yl-12-desethenyl-12-methoxycarbonyl-11-O-methoxymethylmutilin

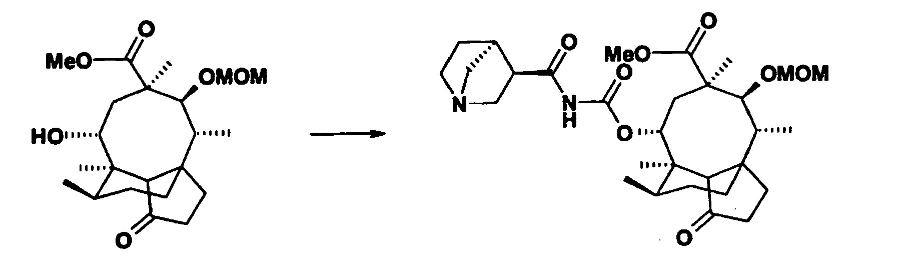

According to Step I of Example 1, the following compounds were used in the reaction: 100 mg (0.25 mmol) of the compound of Step I, an acid chloride prepared from 67.5 mg (0.38 mmol) carboxylic acid, 94.4 mg (0.63 mmol) silver cyanide and 53.0 µL (0.38 mmol) triethylamine. The resulting residue was purified by silica gel column chromatography (NH, ethyl acetate, and then ethyl acetate:methanol = 20:1) to afford 106 mg of the title compound as a colorless powder (75% yield).
MS (FAB) (m/z): 563 (MH⁺).
HRMS (FAB) (m/z) : Calcd. for C₃₀H₄₇N₂O₈(MH⁺): 563.3332. Found, 563.3336.

### Step III

4-{(3R,4S)-1-azabicyclo[2.2.1]heptane-3-carbonyl}carbamoyl-12-desethenyl-12-methoxycarbonylmutilin

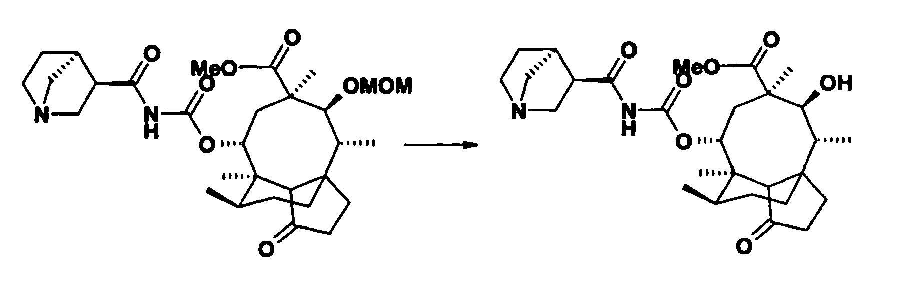

According to Step II of Example 1, 151 mg (0.27 mmol) of the
compound of Step II was used in the reaction. The resulting residue was purified by silica gel column chromatography (NH, ethyl acetate, and then ethyl acetate:methanol = 20:1) to afford 105 mg of the title compound as a colorless powder (75% yield).
MS (FAB) (m/z): 519 (MH⁺) .
HRMS (FAB) (m/z): Calcd. for C₂₈H₄₃N₂O₇(MH⁺) : 519.3070. Found, 519.3085.

### (Example 41)

### Step I

12-desethenyl-12-[1-(E)-(ethoxycarbonyl)ethene-2-yl]-11-0-meth oxymethylmutilin

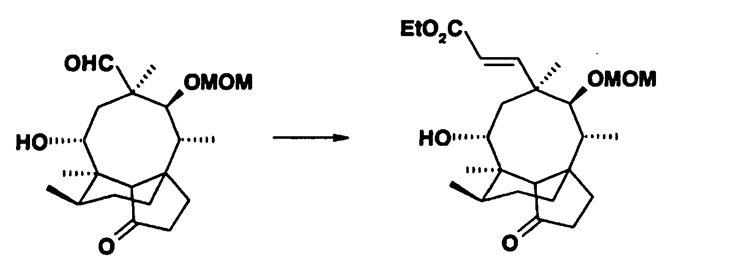

76.9 mg (1.92 mmol) of sodium hydride was added to a solution of 288 mg (1.28 mmol) triethylphosphonoacetate in tetrahydrofuran (10 mL) at room temperature. The mixture was stirred at room temperature for 0.5 hours. While the mixture was kept at the same temperature, a solution of 470 mg (1.28 mmol) of the compound of Example 38 in tetrahydrofuran (10 mL) was added in an argon atmosphere and the mixture was stirred for 1 hour. Subsequently, diluted aqueous citric acid was added and the solvent was evaporated under reduced pressure. The residue was extracted with ethyl acetate (10 mL x 3) . The organic layers were combined, washed with saturated brine (5 mL) and dried over anhydrous magnesium sulfate. The dried product was filtered and the solvent was removed. The resulting residue was purified by silica gel column chromatography (hexane:ethyl acetate = 2:1) to afford 351 mg of the title compound as a colorless powder (59% yield). MS (FAB) (m/z): 437 (MH⁺).
HRMS (FAB) (m/z) : Calcd. for C₂₅H₄₁O₆ (MH⁺) : 437.2903. Found, 437.2903.

### Step II

14-{(3R,4S)-1-azabicyclo[2.2.1]heptane-3-carbonyl}carbamoyl-12 -desethenyl-12-[1-(E)-(ethoxycarbonyl)ethene-2-yl]-11-O-methox ymethylmutilin

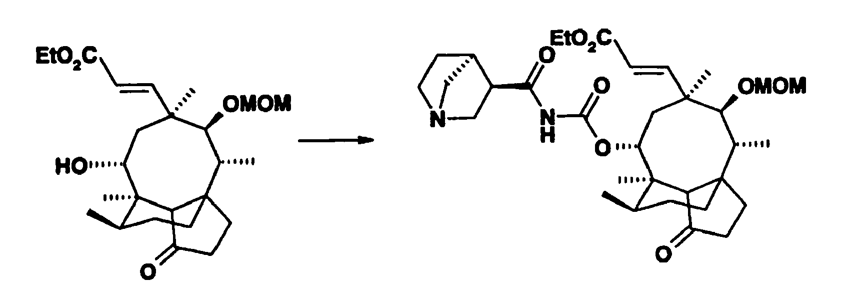

According to Step I of Example 1, the following compounds were used in the reaction: 200 mg (0.46 mmol) of the compound of Step I, an acid chloride prepared from 123 mg (0.69 mmol) carboxylic acid, 172 mg (1.15 mmol) silver cyanide and 96.0 uL (0.69 mmol) triethylamine. The resulting residue was purified by silica gel column chromatography (NH, ethyl acetate, and then ethyl acetate:methanol = 20:1) to afford 91.6 mg of the title compound as a colorless powder (33% yield).
MS (CI) (m/z) : 603 (MH⁺).
HRMS (CI) (m/z) : Calcd. for C₃₃H₅₁N₂O₈(MH⁺) : 603. 3645. Found, 603. 3643.

### Step III

14-{(3R,4S)-1-azabicyclo [2.2.1] heptane-3-carbonyl}carbamoyl-12-desethenyl-12-[1-(E)-(ethoxycarbonyl)ethene-2-yl]mutilin hydrochloride

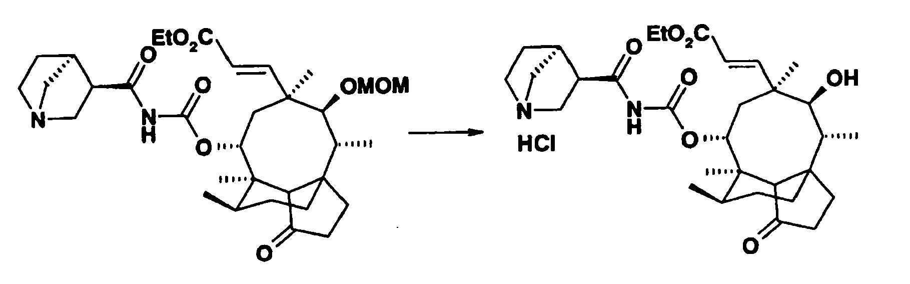

According to Step II of Example 1, 91.6 mg (0.15 mmol) of the compound of Step II was used in the reaction. The resulting residue was purified by silica gel column chromatography (NH, ethyl acetate, and then ethyl acetate:methanol = 20:1) to afford 63.9 mg of 14-{(3R,4S)-1-azabicyclo[2.2.1]heptane-3-carbonyl}carbamoyl-12 -desethenyl-12-[1-(E)-(ethoxycarbonyl)ethene-2-yl]mutilin as a colorless powder (76% yield). To this compound, 4 mol/L hydrogen chloride-dioxane was added. The resulting crystals were collected by filtration and washed with diethyl ether to afford 40.0 mg of the title compound (61% yield).
MS (FAB) (m/z) : 559 (MH⁺ for free form).
HRMS (FAB) (m/z): Calcd. for C₃₁H₄₇N₂O₇ (MH⁺ for free form) : 559.3383. Found, 559.3361.

### (Example 42)

### Step I

12-desethenyl-12-(1-chloroethene-2-yl)-11-O-methoxymethylmutil in

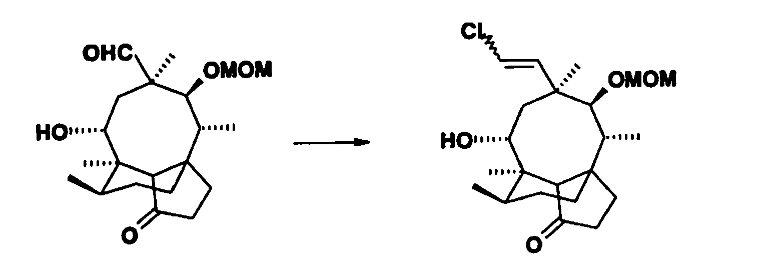

2.84g (8-19 mmol) chloromethyl triphenylphosphonium chloride was suspended in tetrahydrofuran (30 mL). While this suspension was kept chilled on ice in an argon atmosphere, 8.19 mL (8.19 mmol) sodium bis (trimethylsilyl) amide (1mol/L tetrahydrofuran solution) was added dropwise. The mixture was stirred for 0.5 hours at the same temperature and a solution of 1.00 g (2.73 mmol) of the compound of Example 38 in tetrahydrofuran (10 mL) was subsequently added dropwise. The reaction mixture was then stirred for about 1 hour as it was allowed to warm to room temperature. The mixture was poured into diluted aqueous citric acid and was evaporated under reduced pressure. Water was then added to the residue and this mixture was extracted with ethyl acetate (20 mL x 3) . The organic layers were combined, washed with saturated brine (20mL) and dried over anhydrous magnesium sulfate. The dried product was filtered and the solvent was removed. The resulting residue was purified by silica gel column chromatography(hexane:ethylacetate=4:1, followed by hexane:ethyl acetate = 1:1) to afford 1.03 g of the title compound as a colorless powder (95% yield). MS (CI) (m/z) : 399 (MH⁺) .
HRMS (CI) (m/z): Calcd. for C₂₂H₃₆ClO₄(MH⁺) : 399.2302. Found, 399.2329.

### Step II

14-{(3R,4S)-1-azabicyclo[2.2.1]heptane-3-carbonyl}carbamoyl-12 -desethenyl-12-(1-chloroethene-2-yl)-11-O-methoxymethylmutilin

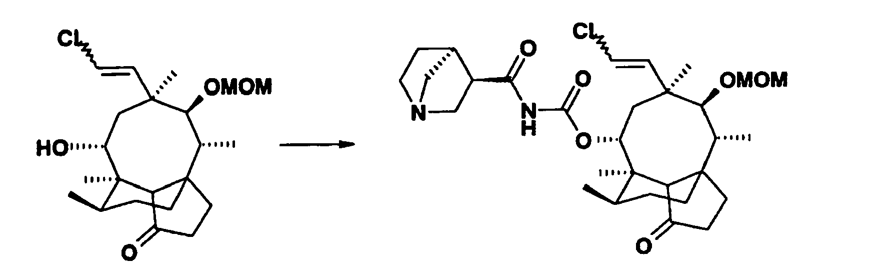

According to Step I of Example 1, the following compounds were used in the reaction: 200 mg (0.50 mmol) of the compound of Step I, an acid chloride prepared from 133 mg (0.75 mmol) carboxylic acid, 187 mg (1.25 mmol) silver cyanide and 0.10 mL (0.75 mmol) triethylamine. The resulting residue was purified by silica gel column chromatography (NH, ethyl acetate, and then ethyl acetate:methanol = 20:1) to afford 31.9 mg of the title compound as a colorless powder (11% yield).
MS (FAB) (m/z): 565 (MH⁺) .
HRMS (FAB) (m/z) : Calcd. for C₃₀H₄₆ClN₂O₆ (MH⁺) : 565.3044. Found, 565.3058.

### Step III

14-{(3R,4S)-1-azabicyclo[2.2.1]heptane-3-carbonyl}carbamoyl-12 -desethenyl-12-(1-chloroethene-2-yl)mutilin

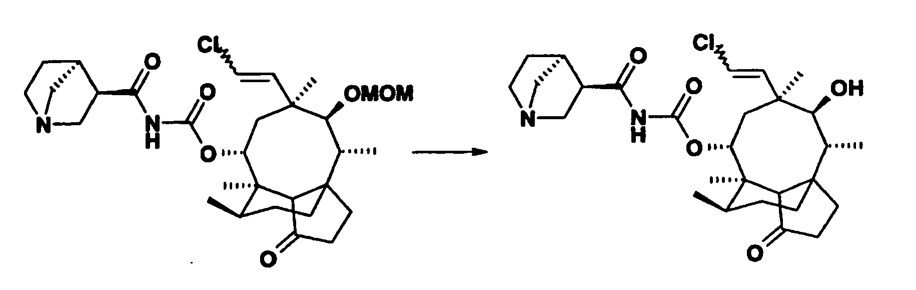

According to Step II of Example 1, 23.9 mg (42.3 µmol) of the compound of Step II was used in the reaction. The resulting residue was purified by silica gel column chromatography (NH, ethyl acetate:methanol = 30:1) to afford 17.6 mg of the title compound as a colorless powder (80% yield).
MS (FAB) (m/z): 521 (MH⁺) .
HRMS (FAB) (m/z): Calcd. for C₂₈H₄₂ClN₂O₅(MH⁺) : 521.2782. Found, 521.2792.

### Step IV

14-{(3R,4S)-1-azabicyclo[2.2.1]heptane-3-carbonyl}carbamoyl-12 -desethenyl-12-(1-chloroethene-2-yl)mutilin hydrochloride

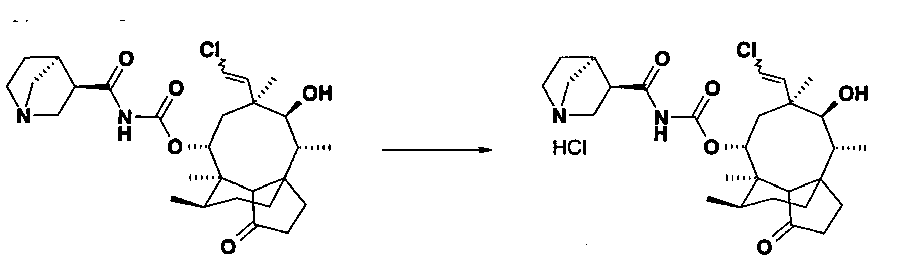

According to Step V of Example 2, 800 mg (1.54 mmol) of the
compound of Step III was used in the reaction to afford 778 mg of the title compound (91% yield).
MS (FAB) (m/z): 521 (MH⁺ for free form).
HRMS (FAB) (m/z) : Calcd. for C₂₈H₄₂ClN₂O₅(MH⁺ for free form) : 521.2782. Found, 521.2762.

### (Example 43)

### Step I

12-desethenyl-12-methoxyiminomethyl-11-O-methoxymethylmutilin

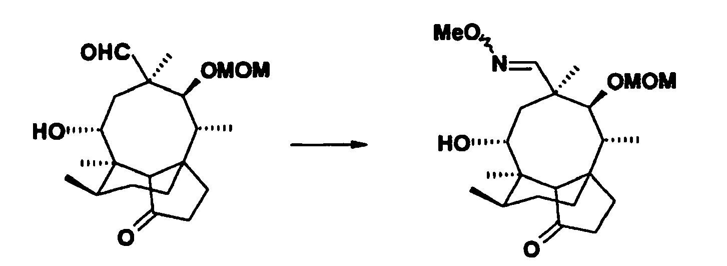

0. 34 g (4.09 mmol) methoxyamine hydrochloride and 0.53 mL (4.09 mmol) triethylamine were added to a solution of 1.00 g (2.73 mmol) of the compound of Example 38 in methanol (20 mL) . The mixture was refluxed for 3 hours. After cooling, the solvent was removed under reduced pressure. Water was then added to the residue and the mixture was extracted with ethyl acetate (20 mL x 3). The organic layers were combined, washed with saturated brine (20 mL) and dried over anhydrous magnesium sulfate. The dried product was filtered and the solvent was removed. Using hexane, the resulting residue was suction-filtered to afford 857 mg of the title compound as a colorless powder (79% yield). MS (CI) (m/z) : 396 (MH⁺) .
HRMS (CI) (m/z) : Calcd. for C₂₂H₃₈NO₅(MH⁺) : 396.2750. Found, 396.2789.

### Step II

14-{(3R,4S)-1-azabicyclo[2.2.1]heptane-3-carbonyl}carbamoyl-12-desethenyl-12-methoxyiminomethyl-11-O-methoxymethylmutilin

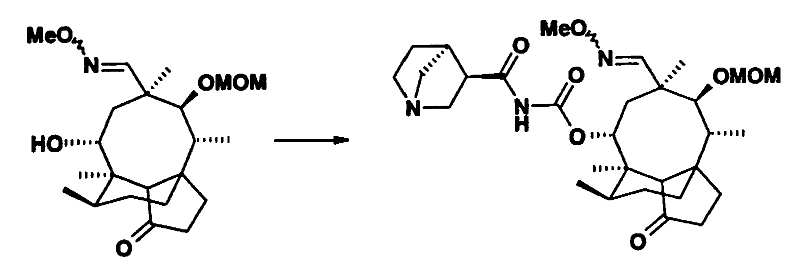

According to Step I of Example 1, the following compounds were used in the reaction: 200 mg (0.51 mmol) of the compound of Step I, an acid chloride prepared from 137 mg (0.77 mmol) carboxylic acid, 192 mg (1.28 mmol) silver cyanide and 0.11 mL (0.77 mmol) triethylamine. The resulting residue was purified by silica gel column chromatography (NH, ethyl acetate, and then ethyl acetate:methanol = 20:1) to afford 34.6 mg of the title compound as a colorless powder (14% yield).
MS (FAB) (m/z): 562 (MH⁺).
HRMS (FAB) (m/z): Calcd. for C₃₀H₄₈N₃O₇(MH⁺) : 562.3492. Found, 562.3506.

### Step III

14-{(3R,4S)-1-azabicyclo[2.2.1]heptane-3-carbonyl}carbamoyl-12 -desethenyl-12-methoxyiminomethylmutilin

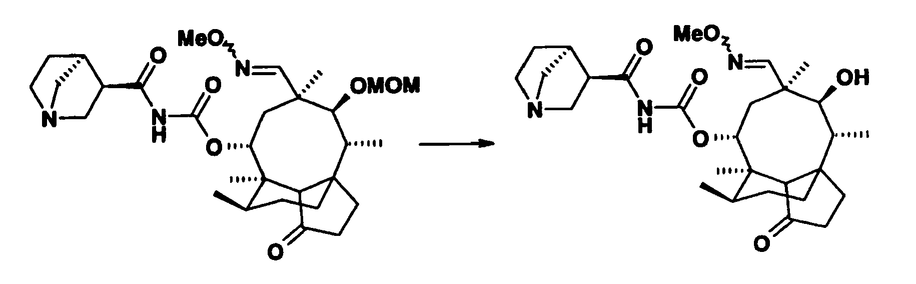

According to Step II of Example 1, 26.6 mg (47.4 µmol) of the compound of Step II was used in the reaction. The resulting residue was purified by silica gel column chromatography (NH, ethyl acetate:methanol = 30:1) to afford 7.90 mg of the title compound as a colorless powder (32% yield).
MS (FAB) (m/z): 518.5 (MH⁺).
HRMS (FAB) (m/z): Calcd. for C₂₈H₄₄N₃O₆(MH⁺): 518.3230. Found, 518.3267.

### (Example 44)

### Step I

12-[(E)-1-butene-1-yl]-12-desethenyl-11-O-methoxymethylmutilin

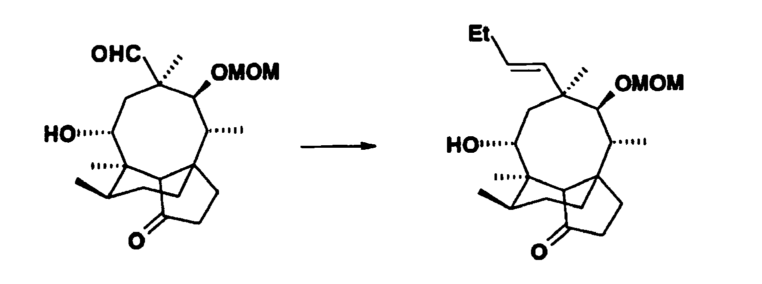

According to Step I of Example 42, 1.00 g (2.73 mmol) of the compound of Example 38, 3.15 g (8.19 mmol) propyltriphenylphosphonium chloride and 8.19 mL (8.19 mmol) sodium bis(trimethylsilyl)amide (1 mol/L tetrahydrofuran solution) were used in the reaction. The resulting residue was purified by silica gel column chromatography (hexane:ethyl acetate = 4:1, followed by hexane:ethyl acetate =1:1) to afford 671 mg of the title compound as a colorless oil (63% yield).
MS (CI) (m/z): 393 (MH⁺) .
HRMS (CI) (m/z) : Calcd. for C₂₄H₄₁O₄(MH⁺) : 393.3005. Found, 303.2984.

### Step II

12-[(E)-1-butene-1-yl]-14-{(3R,4S)-1-azabicyclo[2.2.1]heptane-3-carbonyl}carbamoyl-12-desethenyl-11-O-methoxymethylmutilin

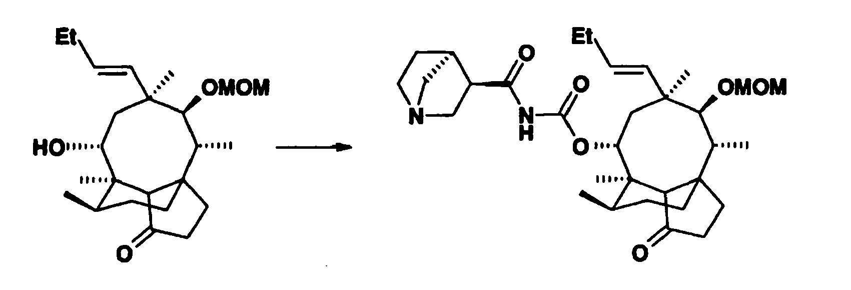

According to Step I of Example 1, the following compounds were used in the reaction: 200 mg (0.51 mmol) of the compound of Step I, an acid chloride prepared from 137 mg (0.77 mmol) carboxylic acid, 192 mg (1.28 mmol) silver cyanide and 0.11 mL (0.77 mmol) triethylamine. The resulting residue was purified by silica gel column chromatography (NH, ethyl acetate, and then ethyl acetate:methanol = 20:1) to afford 40.1 mg of the title compound as a colorless powder (14% yield).
MS (FAB) (m/z): 559.5 (MH⁺).
HRMS (FAB) (m/z): Calcd. for C₃₂H₅₁N₂O₆(MH⁺): 559.3747. Found, 559.3713.

### Step III

12-[(E)-1-butene-1-yl]-14-{(3R,4S)-1-azabicyclo[2.2.1]heptane-3-carbonyl}carbamoyl-12-desethenylmutilin

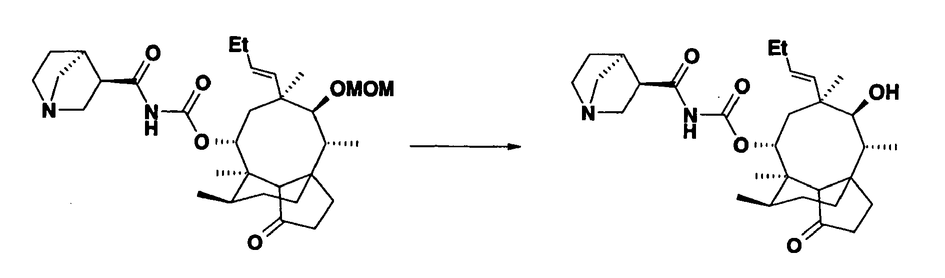

According to Step II of Example 1, 32.5 mg (58.2 µmol) of the compound of Step II was used in the reaction. The resulting residue was purified by silica gel column chromatography (NH, ethyl acetate:methanol = 30:1) to afford 19.2 mg of the title compound as a colorless powder (64% yield).
MS (FAB) (m/z) : 515.5 (MH⁺) .
HRMS (FAB) (m/z) : Calcd. for C₃₀H₄₇N₂O₅(MH⁺): 515.3485. Found, 515.3455.

### Step IV

12-[(E)-1-butene-1-yl]-14-{(3R,4S)-1-azabicyclo[2.2.1]heptane-3-carbonyl}carbamoyl-12-desethenylmutilin hydrochloride

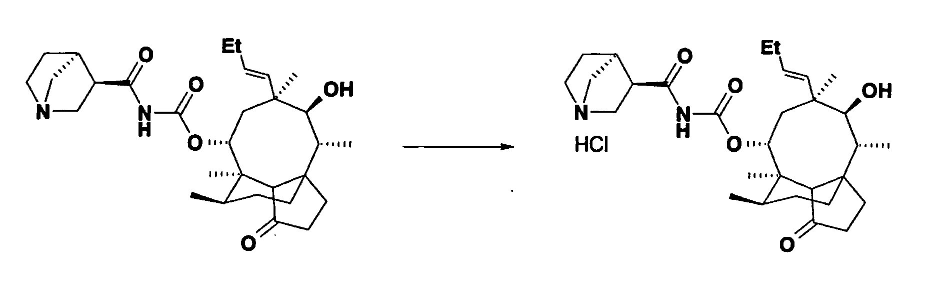

According to Step V of Example 2, 400 mg (0.78 mmol) of the
compound of Step IV was used in the reaction to afford 418 mg of the title compound (97% yield).
MS (FAB) (m/z): 515.6 (MH⁺ for free form).
HRMS (FAB) (m/z) : Calcd. for C₃₀H₄₇N₂O₅(MH⁺ for free form) : 515.3485. Found, 515.3494.

### (Example 45)

### Step I

12-desethenyl-11-O-methoxymethyl-12-[(E)-1-pentene-1-yl]mutili n

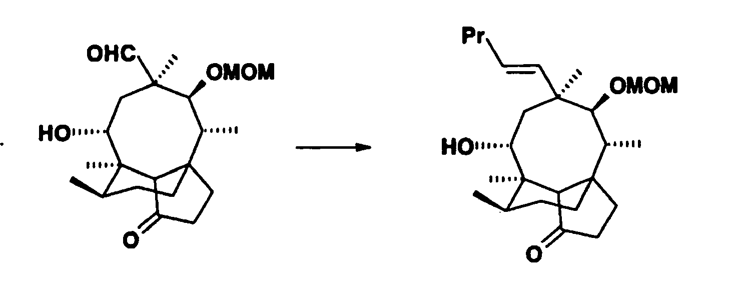

According to Step I of Example 42, 1.00 g (2.73 mmol) of the compound of Example 38, 3.27 g (8.19mmo1) butyltriphenylphosphonium chloride and 8.19 mL (8.19 mmol) sodium bis(trimethylsilyl)amide (1 mol/L tetrahydrofuran solution) were used in the reaction. The resulting residue was purified by silica gel column chromatography (hexane:ethyl acetate = 4:1, followed by hexane:ethyl acetate = 2:1) to afford 408 mg of the title compound as a colorless powder (37% yield). MS (FAB) (m/z): 407 (MH⁺) .
HRMS (FAB) (m/z): Calcd. for C₂₅H₄₃O₄ (MH⁺) : 407.3161. Found, 403. 3178.

### Step II

14-{(3R,4S)-1-azabicyclo[2.2.1]heptane-3-carbonyl}carbamoyl-12 -desethenyl-11-O-methoxymethyl-12-[(E)-1-pentene-1-yl]mutilin

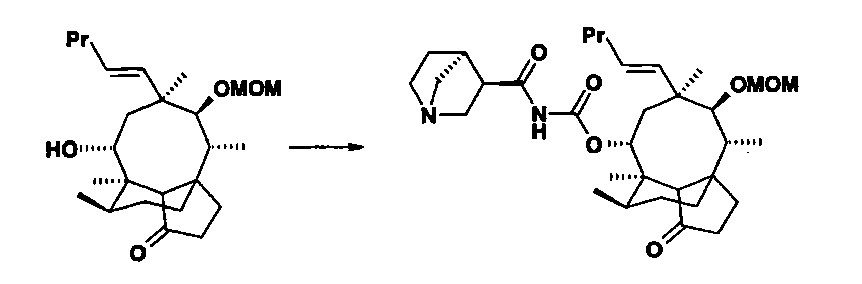

According to Step I of Example 1, the following compounds were used in the reaction: 408 mg (1.00 mmol) of the compound of Step I, an acid chloride prepared from 266 mg (1.50 mmol) carboxylic acid, 375 mg (2.50 mmol) silver cyanide and 0.21 mL (1.50 mmol) triethylamine. The resulting residue was purified by silica gel column chromatography (NH, ethyl acetate, and then ethyl acetate:methanol = 20:1) to afford 72.3 mg of the title compound as a colorless powder (13% yield).
MS (FAB) (m/z) : 573 (MH⁺) .
HRMS (FAB) (m/z): Calcd. for C₃₃H₅₃N₂O₆ (MH⁺): 573.3904. Found, 573.3913.

### Step III

14-{(3R,4S)-1-azabicyclo[2.2.1]heptane-3-carbonyl}carbamoyl-12 -desethenyl-12-[(E)-1-pentene-1-yl]mutilin

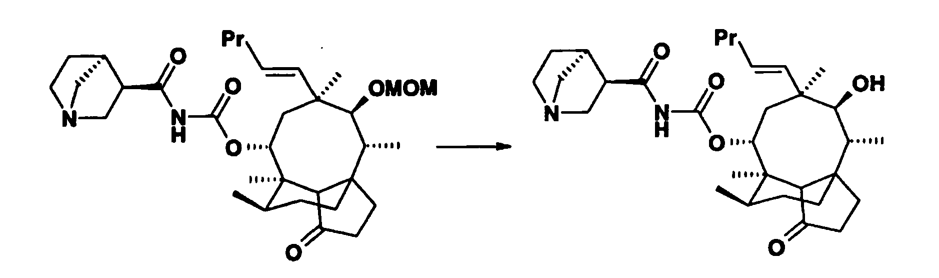

According to Step II of Example 1, 66.4 mg (0.12 mmol) of the compound of Step II was used in the reaction. The resulting residue was purified by silica gel column chromatography (NH, ethyl acetate:methanol = 30:1) to afford 41.8 mg of the title compound as a colorless powder (66% yield).
MS (FAB) (m/z) : 529 (MH⁺) . HRMS (FAB) (m/z): Calcd. for C₃₁H₄₉N₂O₅(MH⁺): 529.3641. Found, 529.3663.

### (Example 46)

### Step I

12-desethenyl-11-O-methoxymethyl-12-(1-phenylethene-2-yl)mutil in

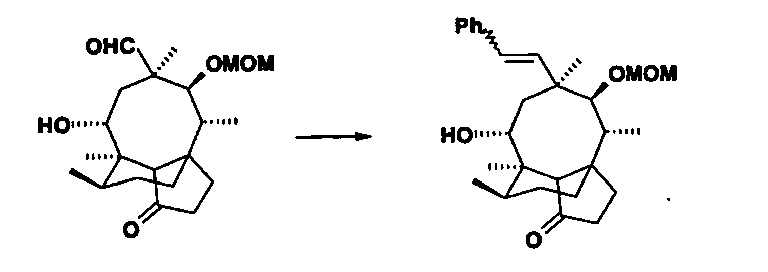

According to Step I of Example 42, 1.00 g (2.73 mmol) of the compound of Example 38, 3.18 g (8.19 mmol) benzyltriphenylphosphonium chloride and 8.19 mL (8.19 mmol) sodium bis(trimethylsilyl)amide (1 mol/L tetrahydrofuran solution) were used in the reaction. The resulting residue was purified by silica gel column chromatography (hexane:ethyl acetate = 4:1, followed by hexane: ethyl acetate = 2: 1) to afford 269 mg of the title compound as a colorless powder (22% yield).
MS (FAB) (m/z) : 441 (MH⁺).
HRMS (FAB) (m/z) : Calcd. for C₂₈H₄₁O₄(MH⁺) : 441.3005. Found, 441.3001.

### Step II

14-{(3R,4S)-1-azabicyclo[2.2.1]heptane-3-carbonyl}carbamoyl-12 -desethenyl-11-0-methoxymethyl-12-(1-phenylethene-2-yl)mutilin

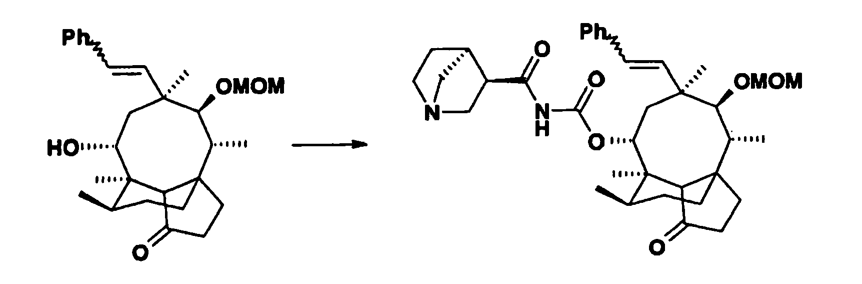

According to Step I of Example 1, the following compounds were used in the reaction: 200 mg (0.45 mmol) of the compound of Step I, an acid chloride prepared from 121 mg (0.68 mmol) carboxylic acid, 169 mg (1.13 mmol) silver cyanide and 94.8 µL (0.68 mmol) triethylamine. The resulting residue was purified by silica gel column chromatography (NH, ethyl acetate, and then ethyl acetate:methanol = 20:1) to afford 60.5 mg of the title compound as a colorless powder (22% yield).
MS (FAB) (m/z): 607 (MH⁺).
HRMS (FAB) (m/z) : Calcd. for C₃₆H₅₁N₂O₆(MH⁺) : 607.3747. Found, 607.3743.

### Step III

14-{(3R,4S)-1-azabicyclo[2.2.1]heptane-3-carbonyl}carbamoyl-12-desethenyl-12-(1-phenylethene-2-yl)mutilin

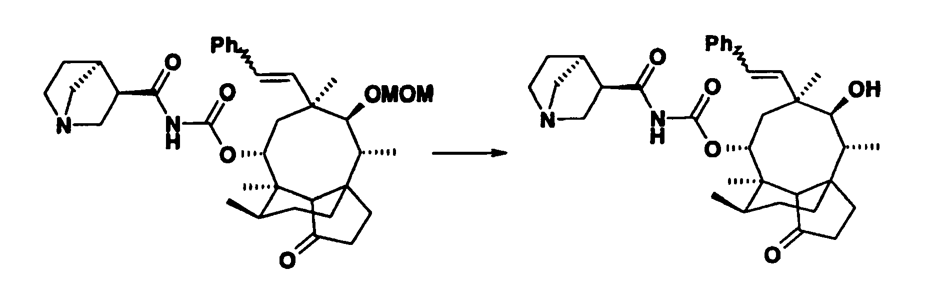

According to Step II of Example 1, 52.0 mg (85.7 mmol) of the compound of Step II was used in the reaction. The resulting residue was purified by silica gel column chromatography (NH, ethyl acetate:methanol = 30:1) to afford 41.5 mg of the title compound as a colorless powder (86% yield).
MS (FAB) (m/z): 563 (MH⁺) .
HRMS (FAB) (m/z): Calcd. for C₃₄H₄₇N₂O₅(MH⁺) : 563.3485. Found, 563.3509.

### (Example 47)

### Step I

12-(buta-1,3-diene-1-yl)-12-desethenyl-11-O-methoxymethylmutil in

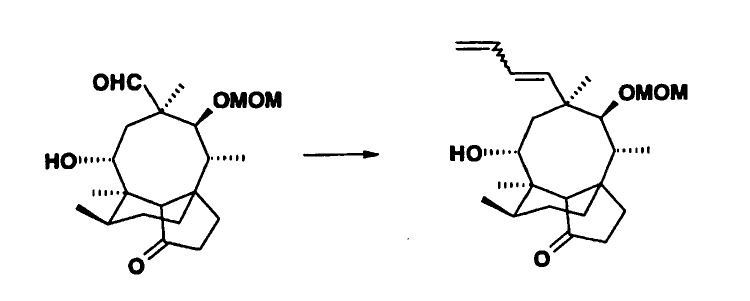

According to Step I of Example 42, 1.00 g (2.73 mmol) of the compound of Example 38, 3.14g (8.19 mmol) allyltriphenylphosphonium bromide and 8.19 mL (8.19 mmol) sodium bis(trimethylsilyl)amide (1 mol/L tetrahydrofuran solution) were used in the reaction. The resulting residue was purified by silica gel column chromatography (hexane:ethyl acetate = 4:1, followed by hexane:ethyl acetate = 2:1) to afford 128 mg of the title compound as a yellow powder (12% yield). MS (FAB) (m/z) : 391 (MH⁺).
HRMS (FAB) (m/z) : Calcd. for C₂₄H₃₉O₄(MH⁺) : 391.2848. Found, 391.2831.

### Step II

14-{(3R,4S)-1-azabicyclo[2.2.1]heptane-3-carbonyl}carbamoyl-12 -(buta-1,3-diene-1-yl)-12-desethenyl-11-O-methoxymethylmutilin

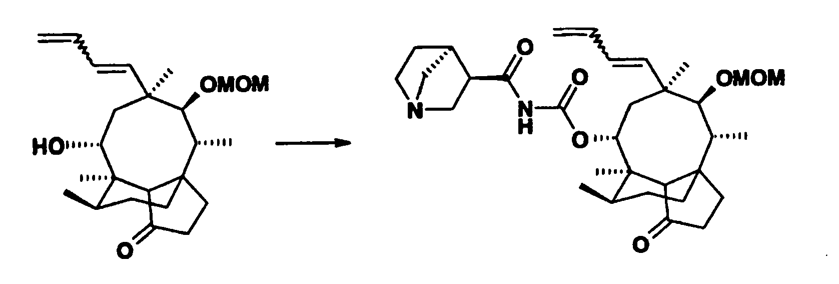

According to Step I of Example 1, the following compounds were used in the reaction: 120 mg (0.31 mmol) of the compound of Step I, an acid chloride prepared from 83.5 mg (0.47 mmol) carboxylic acid, 117 mg (0.78 mmol) silver cyanide and 65.5 µL (0.47 mmol) triethylamine. The resulting residue was purified by silica gel column chromatography (NH, ethyl acetate, and then ethyl acetate:methanol = 15:1) to afford 36.0 mg of the title compound as a colorless powder (21% yield).
MS (FAB) (m/z) : 557 (MH⁺) .
HRMS (FAB) (m/z) : Calcd. for C₃₂H₄₉N₂O₆ (MH⁺) : 557.3591. Found, 557.3560.

### Step III

14-{(3R,4S)-1-azabicyclo[2.2.1]heptane-3-carbonyl}carbamoyl-12 -(buta-1,3-diene-1-yl)-12-desethenylmutilin

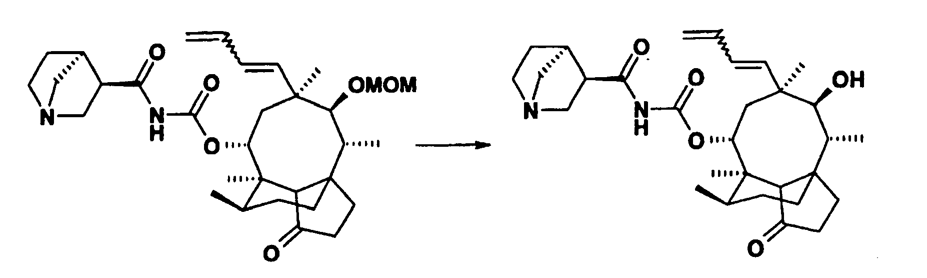

According to Step II of Example 1, 29.7 mg (53.3 µmol) of the compound of Step II was used in the reaction. The resulting residue was purified by silica gel column chromatography (NH, ethyl acetate:methanol = 30:1) to afford 16.5 mg of the title compound as a colorless powder (60% yield).
MS (FAB) (m/z) : 513 (MH⁺) .
HRMS (FAB) (m/z): Calcd. for C₃₀H₄₅N₂O₅ (MH⁺) : 513. 3328. Found, 513.3339.

### (Example 48)

19,20-O,O-isopropylidene-19,20-dihydroxy-12-desethenyl-11-O-me thoxymethylmutilin

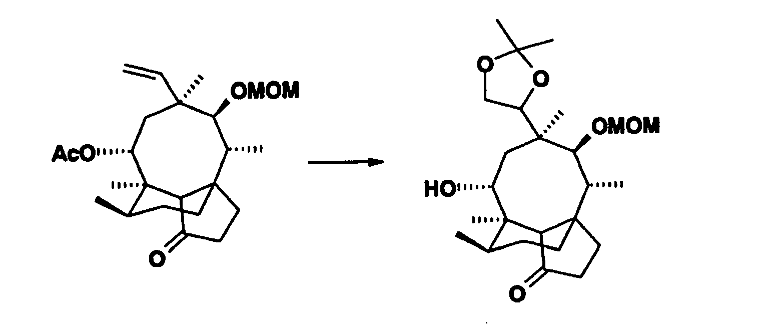

According to Reference Example 2, 3.00 g (7.38 mmol) 14-acetoxy-12-desethenyl-12-formyl-11-O-methoxymethylmutilin obtained in Example 39 was converted to a diol. The crude product was purified by silica gel column chromatography (hexane:ethyl acetate = 2:1, followed by hexane:ethyl acetate = 1:2) to obtain 1.73 g of the diol as a yellow oil (53% yield). The compound was dissolved in methanol (10 mL). To this solution, a diluted aqueous sodium hydroxide solution (10 mL) was added and the mixture was refluxed for 13 hours. After cooling, the solvent was removed under reduced pressure, followed by addition of diluted aqueous citric acid and extraction with ethyl acetate (30 mL x 3). The organic layers were combined, washed with saturated brine (30 mL) and dried over anhydrous magnesium sulfate. Filtration of the dried product followed by removal of the solvent gave a crude triol product. This product was dissolved in acetone (30 mL). To this solution, 1.45 mL (11.8 mmol) acetone dimethyl acetal and a catalytic amount of p-toluenesulfonic acid were added and the mixture was allowed to stand for 12 hours at room temperature. Subsequently, the reaction mixture was evaporated under reduced pressure, followed by addition of diluted aqueous citric acid and extraction with ethyl acetate (30 mL x 3). The organic layers were combined, washed with saturated brine (30 mL) and dried over anhydrous magnesium sulfate. The dried product was filtered and the solvent was removed. The resulting residue was purified by column chromatography (hexane: ethyl acetate = 2:1) to afford 1.22 g of the title compound as a pale yellow powder (71% yield). MS (FAB) (m/z): 439 (MH⁺) .
HRMS (FAB) (m/z) : Calcd. for C₂₅H₄₃O₆(MH⁺): 439. 3060. Found, 439. 3082.

### (Example 49)

12-desethenyl-11-O-methoxymethylmutilin-12-N-methylcarboxamide

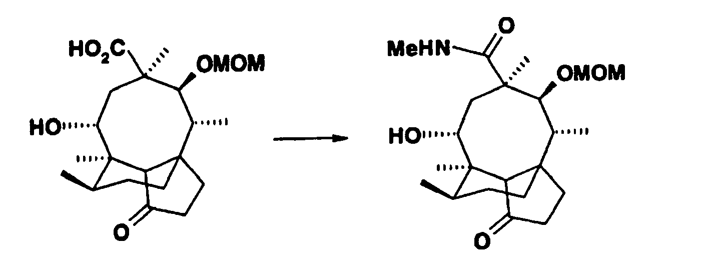

200 mg (0.52 mmol) 12-carboxy-12-desethenyl-11-O-methoxymethylmutilin obtained in Step I of Example 40 was dissolved in methylene chloride (2 mL). To this solution, 100 mg (0.52 mmol) 1-(3-dimethylaminopropyl)-3-ethylcarbodiimide hydrochloride and 0.78 mL (1.57 mmol) methylamine (2 mol/L tetrahydrofuran solution) were added and the mixture was stirred for 12 hours at room temperature. Subsequently, diluted aqueous citric acid was added and the mixture was extracted with ethyl acetate (5 ml x 3). The organic layers were combined, washed with saturated brine (5 mL) and dried over anhydrous magnesium sulfate. The dried product was filtered and the solvent was removed. The resulting residue was purified by column chromatography (NH, hexane:ethyl acetate = 1:1) to afford 46.7 mg of the title compound as a colorless oil (23% yield).
MS (FAB) (m/z) : 396 (MH⁺).
HRMS (FAB) (m/z) : Calcd. for C₂₂H₃₈NO₅(MH⁺): 396.2750. Found, 396.2759.

### (Example 50)

12-desethenyl-11-methoxymethyloxymutilin 12-N,N-dimethylcarboxamide

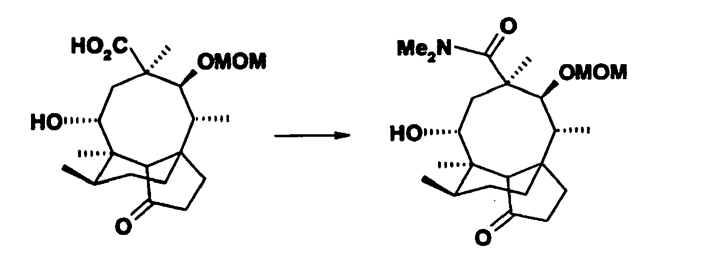

According to Example 49, 200 mg (0.52 mmol) 12-carboxy-12-desethenyl-11-O-methoxymethylmutilin, 100 mg (0.52 mmol) 1-(3-dimethylaminopropyl)-3-ethylcarbodiimide hydrochloride and 0.78 mL (1.57 mmol) dimethylamine (2 mol/L tetrahydrofuran solution) were used in the reaction. The resulting residue was purified by column chromatography (NH, hexane:ethyl acetate = 1 : 1) to afford 46.6 mg of the title compound as a colorless oil (22% yield). MS (FAB) (m/z) : 410 (MH⁺).
HRMS (FAB) (m/z): Calcd. for C₂₃H₄₀NO₅(MH⁺) : 410.2906. Found, 410.2988.

### (Example 51)

### Step I

(3R)-12-[(Z)-2-butene-4-yl]-3-deoxo-11-deoxy-12-desethenyl-3-m ethoxy-11-oxo-4-epimutilin

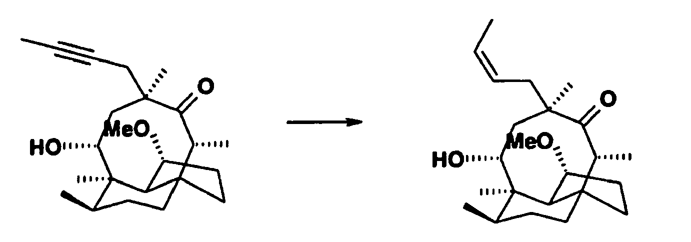

According to Reference Example 4, 100 mg (0.28 mmol) of the compound of Step I of Example 7 was used in the reaction. The resulting residue was purified by silica gel column chromatography (hexane:ethyl acetate =4:1) to afford 103 mg of the title compound as a colorless powder (100% yield).
MS (FAB) (m/z) : 363 (MH⁺)
HRMS (FAB) (m/z) : Calcd. for C₂₃H₃₉O₃(MH⁺) : 363.2899. Found, 363.2864.

### Step II

(3R)-14-{(3R,4S)-1-azabicyclo[2.2.1]heptane-3-carbonyl}carbamo yl-12-[(Z)-2-butene-4-yl]-3-deoxo-11-deoxy-12-desethenyl-3-met hoxy-11-oxo-4-epimutilin

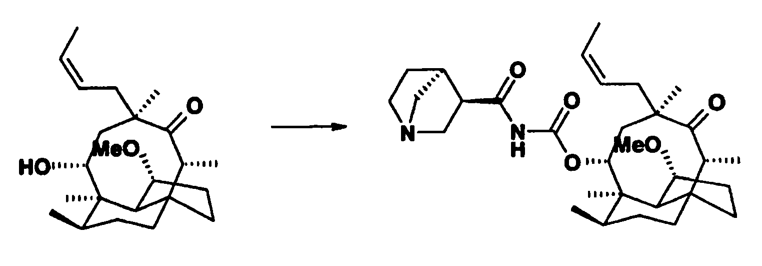

According to Step I of Example 1, the following compounds were used in the reaction: 85.2 mg (0.24 mmol) of the compound of Step I, an acid chloride prepared from 63.9 mg (0.36 mmol) carboxylic acid, 89.9 mg (0.60 mmol) silver cyanide and 50.2 µL (0.36 mmol) triethylamine. The resulting residue was purified by silica gel column chromatography (NH, ethyl acetate, and then ethyl acetate:methanol = 20:1) to afford 55.0 mg of the title compound as a colorless powder (43% yield).
MS (FAB) (m/z): 529 (MH⁺).
HRMS (FAB) (m/z): Calcd. for C₃₁H₄₉N₂O₅ (MH⁺) : 529.3641. Found, 529.3663.

### Step III

14-{(3R,4S)-1-azabicyclo[2.2.1]heptane-3-carbonyl}carbamoyl-12 -[(Z)-2-butene-4-yl]-12-desethenylmutilin

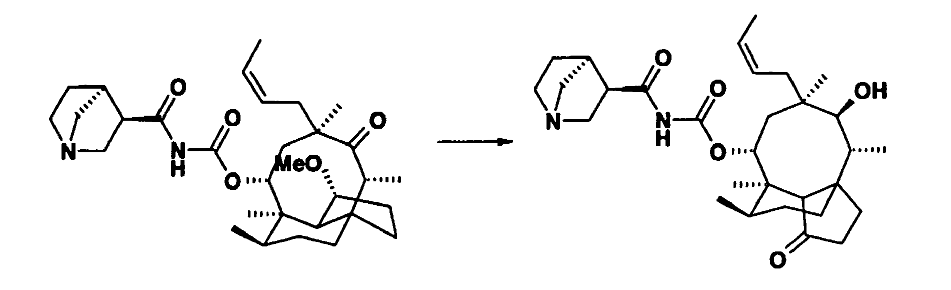

According to Step II of Example 1, 49.0 mg (92.7 µmol) of the compound of Step III was used in the reaction. The resulting residue was purified by silica gel column chromatography (NH, ethyl acetate:methanol = 30:1) to afford 21.8 mg of the title compound as a colorless powder (46% yield).
MS (FAB) (m/z) : 515 (MH⁺) .
HRMS (FAB) (m/z): Calcd. for C₃₀H₄₇N₂O₅(MH⁺): 515.3485. Found, 515.3483.

### (Example 52)

### Step I

14-{(3S,4S)-1-azabicyclo[2.2.1]heptane-3-carbonyl}carbamoyl-12 -desethenyl-12-[2-propene-3-yl]mutilin

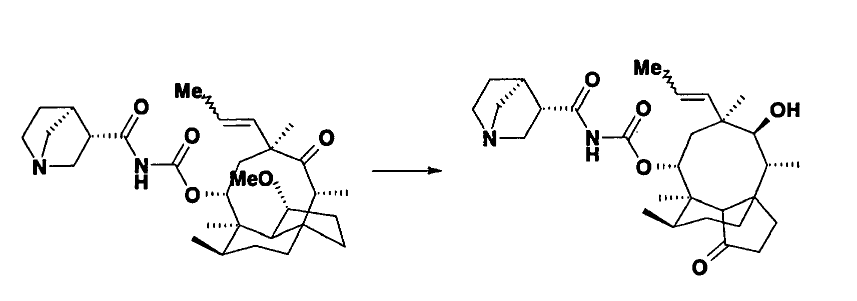

According to Step II of Example 1, 1.50 g (2.91 mmol) 14-{(3S,4S)-1-azabicyclo[2.2.1]heptane-3-carbonyl}carbamoyl-12 -desethenyl-3-methoxy-11-O-methoxymethyl-11-oxo-12-[2-propene-3-yl]-4-epimutilin obtained in Step II of Example 15 was used in the reaction. The resulting residue was purified by silica gel column chromatography (NH, ethyl acetate, and then ethyl acetate:methanol = 10:1) to afford 900 mg of the title compound as a colorless powder (62% yield).
MS (FAB) (m/z) : 501 (MH⁺).
HRMS (FAB) (m/z): Calcd. for C₂₉H₄₅N₂O₅ (MH⁺) : 501.3328. Found, 501.3289.

### Step II

14-{(3S,4S)-1-azabicyclo[2.2.1]heptane-3-carbonyl}carbamoyl-12 -desethenyl-12-[2-propene-3-yl]mutilin hydrochloride

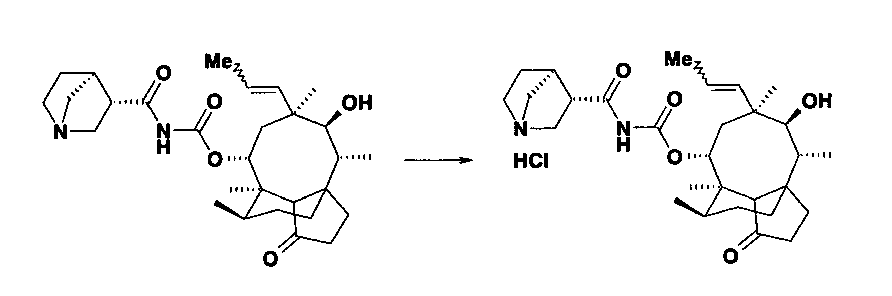

According to Step V of Example 2, 776 mg (1.55 mmol) of the
compound of Step I was used in the reaction to afford 664 mg of the title compound (80% yield).
MS (FAB) (m/z): 501 (MH⁺ for free form).
HRMS (FAB) (m/z) : Calcd. for C₂₉H₄₅N₂O₅ (MH⁺ for free form) : 501.3328. Found, 501.3319.

### (Test Example)

The minimum inhibitory concentration (MIC) was determined for each compound by NCCLS agar dilution technique (Methods for dilution antimicrobial susceptibility tests for bacteria that grow aerobically; approved standard-sixth edition. NCCLS. 2003, M7-A6, Vol. 23 (No.2)). The results are shown in Table below and indicate that the compounds of the present invention have high antimicrobial activity.

### (Table 1)

**Table. In vitro antimicrobial activity**

| | Example 2 | Example 4 | Example 17 | Example 18 | Example 20 | tiamulin |
|---|---|---|---|---|---|---|
| S. aureus 209P | 0.125 | 0.125 | 0.125 | 0.125 | 0.06 | 0.06-0.125 |
| S.pneumoniae IID 553 | 1 | 0.5 | 2 | 2 | 0.5 | 1-2 |

### INDUSTRIAL APPLICABILITY

Novel position 12-substitued mutilin derivatives having high antimicrobial activity, the compounds of the present invention can be used as an effective therapeutic agent for infectious diseases caused by Gram-positive or Gram-negative bacteria, including various drug-resistant bacteria.

## Claims

1. A mutilin derivative or an acid-addition salt thereof, represented by the following chemical formula (1): 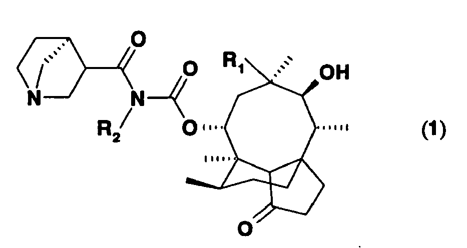 (wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; and R₂ is a hydrogen atom, a lower alkyl group or an aralkyl group whosearomatic ring may be optionally substituted) (R₁ is neither an ethyl group nor a vinyl group).

2. A mutilin derivative or an acid-addition salt thereof, represented by the following chemical formula (1-1): 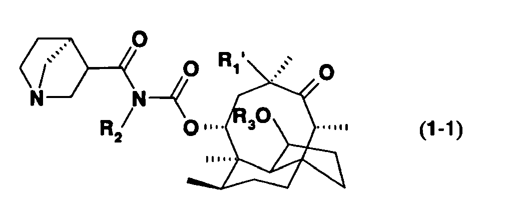 (wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or an aralkyl group whose aromatic ring may be optionally substituted; and R₃ is a protective group for a hydroxyl group) (R₁' is neither an ethyl group nor a vinyl group).

3. A mutilin derivative or an acid-addition salt thereof, represented by the following chemical formula (1-2): 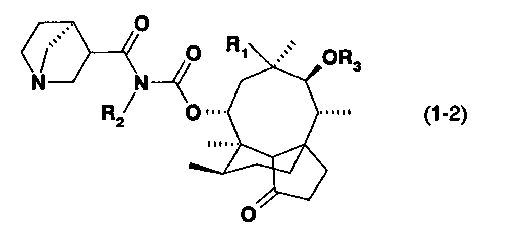 (wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or a substituted or unsubstituted aralkyl group; and R₃ is a protective group for a hydroxyl group) (R₁ is neither an ethyl group nor a vinyl group).

4. An intermediate for the production of the mutilin derivative or an acid-addition salt thereof according to claims 1 to 3, comprising a 4-epimutilin derivative represented by the following general formula (2-1): 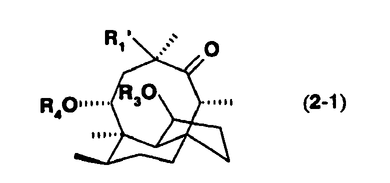 (wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₃ is a protective group for a hydroxyl group; and R₄ is a hydrogen atom or a protective group for a hydroxyl group) (R₁' is neither an ethyl group nor a vinyl group).

5. An intermediate for the production of the mutilin derivative or an acid-addition salt thereof according to claims 1 to 4, comprising a mutilin derivative represented by the following general formula (2-2): 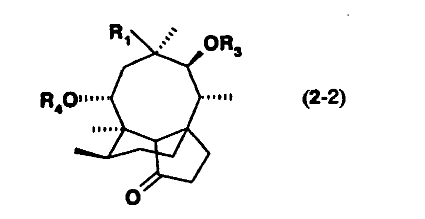 (wherein R₁ is a hydrogen atom, a formyl group, a substituted or unsubstituted lower alkyl group, an aralkyl group whose aromatic ring may be optionally substituted, a heteroaralkyl group whose aromatic ring may be optionally substituted or a lower alkyloxycarbonyl group; and R₃ and R₄ are each independently a hydrogen atom or a protective group for a hydroxyl group) (R₁ is neither an ethyl group nor a vinyl group).

6. A therapeutic agent for infectious diseases comprising as an active ingredient at least one mutilin derivative or an acid-addition salt thereof represented by the following general formula (1): 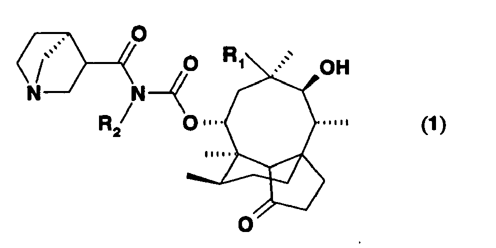 (wherein R₁ is a hydrogen atom, a formyl group, a substituted or unsubstituted lower alkyl group, an aralkyl group whose aromatic ring may be optionally substituted, a heteroaralkyl group whosearomatic ring may be optionally substituted or a lower alkyloxycarbonyl group; and R₂ is a hydrogen atom, a lower alkyl group or an aralkyl group whosearomatic ring may be optionally substituted) (R₁ is neither an ethyl group nor a vinyl group).

7. A therapeutic agent for infectious diseases comprising as an active ingredient at least one mutilin derivative or an acid-addition salt thereof represented by the following general formula (1-1): 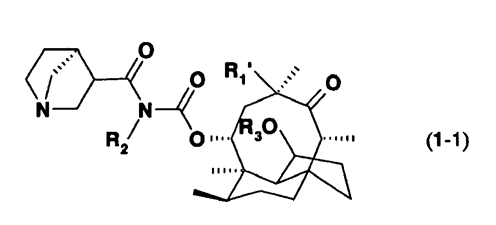 (wherein R₁' is a hydrogen atom, a substituted or unsubstituted lower alkyl group, an aralkyl group whose aromatic ring may be optionally substituted, a heteroaralkyl group whose aromatic ring may be optionally substituted or a lower alkyloxycarbonyl group; R₂ is a hydrogen atom, a lower alkyl group or an aralkyl group whose aromatic ring may be optionally substituted; and R₃ is a protective group for a hydroxyl group) (R₁' is neither an ethyl group nor a vinyl group) .

8. A therapeutic agent for infectious diseases comprising as an active ingredient at least one mutilin derivative or an acid-addition salt thereof represented by the following general formula (1-2): 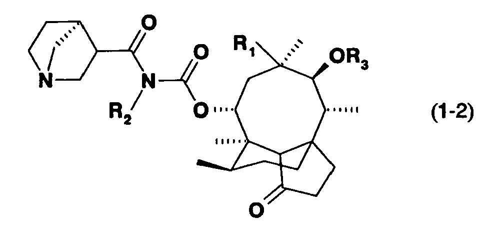 (wherein R₁ is a hydrogen atom, a formyl group, a substituted or unsubstituted lower alkyl group, an aralkyl group whose aromatic ring may be optionally substituted , a heteroaralkyl group whose aromatic ring may be optionally substituted or a lower alkyloxycarbonyl group; R₂ is a hydrogen atom, a lower alkyl group or a substituted or unsubstituted aralkyl group; and R₃ is a protective group for a hydroxyl group) (R₁ is neither an ethyl group nor a vinyl group) .
